# EUROPEAN PATENT SPECIFICATION

(11) **EP 1 244 708 B1**
(45) Date of publication and mention of the grant of the patent: **13.09.2006**
(21) Application number: 00988365.3
(22) Date of filing: 22.12.2000
(51) Int. Cl.: C07K 16/24, C07K 16/28, C07K 14/54, A61K 39/395, C07K 19/00, A61P 17/06, A61P 11/06, C07K 14/715

(54) **METHOD FOR TREATING INFLAMMATION**
VERFAHREN ZUR BEHANDLUNG VON ENTZÜNDUNGEN
METHODE DESTINEE A TRAITER L'INFLAMMATION

(30) Priority: 23.12.1999 US 470898; 22.06.2000 US 213341 P
(43) Date of publication of application: 02.10.2002
(60) Divisional application: 05020542.6
(73) Proprietor: ZymoGenetics, Inc., Seattle, WA 98102 (US)
(72) Inventor: THOMPSON, Penny, Snohomish, WA 98290 (US); FOSTER, Donald, C., Lake Forest Park, WA 98155 (US); XU, Wenfeng, Seattle, WA 98115 (US); MADDEN, Karen, L., Bellevue, WA 98004 (US); KELLY, James, D., Mercer Island, WA 98040 (US); SPRECHER, Cindy, A., Hereford, AZ 85615 (US); BLUMBERG, Hal, Seattle, WA 98103 (US); EAGAN, Maribeth, A., Seattle, WA 98117 (US); JASPERS, Stephen, R., Edmonds, WA 98026 (US); CHANDRASEKHER, Jasmin, Saratoga, CA 95070 (US); NOVAK, Julia, E., Suquamish, WA 98392 (US)
(74) Representative: MacLean, Martin Robert
(86) International application number: PCT/US2000/035305
(87) International publication number: WO 2001/046261

(56) References cited:
- WO-A-00/42189
- WO-A-99/03982
- WO-A-99/27103
- US-A- 5 945 511
- MCKINNON M ET AL: "STRATEGIES FOR THE DISCOVERY OF CYTOKINE RECEPTOR ANTAGONISTS" DRUG NEWS AND PERSPECTIVES,XX,XX, vol. 9, 1996, pages 389-398, XP000882849 ISSN: 0214-0934
- MOHLER K M ET AL: "IMMUNOTHERAPEUTIC POTENTIAL OF SOLUBLE SYTOKINE RECEPTORS IN INFLAMMATORY DISEASE" FASEB JOURNAL,US,FED. OF AMERICAN SOC. FOR EXPERIMENTAL BIOLOGY, BETHESDA, MD, vol. 6, no. 4, 26 February 1992 (1992-02-26), page A1123 XP002015786 ISSN: 0892-6638

## Description

### BACKGROUND OF THE INVENTION

Inflammation normally is a localized, protective response to trauma or microbial invasion that destroys, dilutes, or walls-off the injurious agent and the injured tissue. It is characterized in the acute form by the classic signs of pain, heat, redness, swelling, and loss of function. Microscopically, it involves a complex series of events, including dilation of arterioles, capillaries, and venules, with increased permeability and blood flow, exudation of fluids, including plasma proteins, and leukocyte migration into the area of inflammation.

Diseases characterized by inflammation are significant causes of morbidity and mortality in humans. Commonly, inflammation occurs as a defensive response to invasion of the host by foreign, particularly microbial, material. Responses to mechanical trauma, toxins, and neoplasia also may results in inflammatory reactions. The accumulation and subsequent activation of leukocytes are central events in the pathogenesis of most forms of inflammation. Deficiencies of inflammation compromise the host. Excessive inflammation caused by abnormal recognition of host tissue as foreign or prolongation of the inflammatory process may lead to inflammatory diseases as diverse as diabetes, arteriosclerosis, cataracts, reperfusion injury, and cancer, to post-infectious syndromes such as in infectious meningitis, rheumatic fever, and to rheumatic diseases such as systemic lupus erythematosus and rheumatoid arthritis. The centrality of the inflammatory response in these varied disease processes makes its regulation a major element in the prevention control or cure of human disease.

An important cytokine in the inflammatory process is interleukin-8 (IL-8). IL-8 is a chemokine. It was identified as an agonist for neutrophils on the basis of two effects, chemotaxis and the release of granule enzymes. IL-8 binds to two receptors on neutrophils. IL-8 receptors are also found on monocytes, basophils, and eosinophils. In human fibroblasts, cytomegalovirus has been shown to induce the expression of IL-8 receptors and to replicate more rapidly when cells are exposed to IL-8. IL-8 is a potent chemoattractant for neutrophils; and the early stages of periodontal disease are characterized by the influx of neutrophils. IL-8 is a potent inducer of angiogenesis in several angiogenesis-dependent chronic inflammatory conditions, including rheumatoid arthritis, psoriasis, and idiopathic pulmonary fibrosis. Additionally, IL-8 is an important source of angiogenic activity in human lung cancer. Also, IL-8 expression correlates with experimental metastatic activity of some melanoma cell lines. Thus, an effective method to treat inflammatory diseases would be to administer an agent that would inhibit IL-8.

WO 99/27103 describes Interleukin-20 (IL-20) polypeptides, and corresponding polynucleotides, and uses thereof for detecting and treating immune system - related disorders.

US 5945511 describes class II cytokine receptor polypeptides, and corresponding polypeptides, and uses thereof in promoting proliferentiation and/or differentiation of a range of tissues.

The present invention meets this need in the art by providing use of a soluble receptor that binds to an IL-20 polypeptide, wherein the IL-20 polypeptide comprises an amino acid sequence selected from the group consisting of SEQ ID Nos: 1, 2, 3, 4, 5, 6, 7, 8 and 9, for the manufacture of a medicament for treating an inflammatory disease, wherein said soluble receptor comprises: a first subunit, said first subunit comprising an amino acid sequence selected from SEQ ID Nos: 11, 12, 55, 63 and 65, or a fragment thereof that binds to an IL-20 polypeptide and a second subunit, said second subunit comprising an amino acid sequence selected from SEQ ID Nos: 14, 15, 19, 59, 61, 67, 68 and 69, or a fragment thereof that binds to an IL-20 polypeptide; wherein said inflammatory disease is a skin disease.

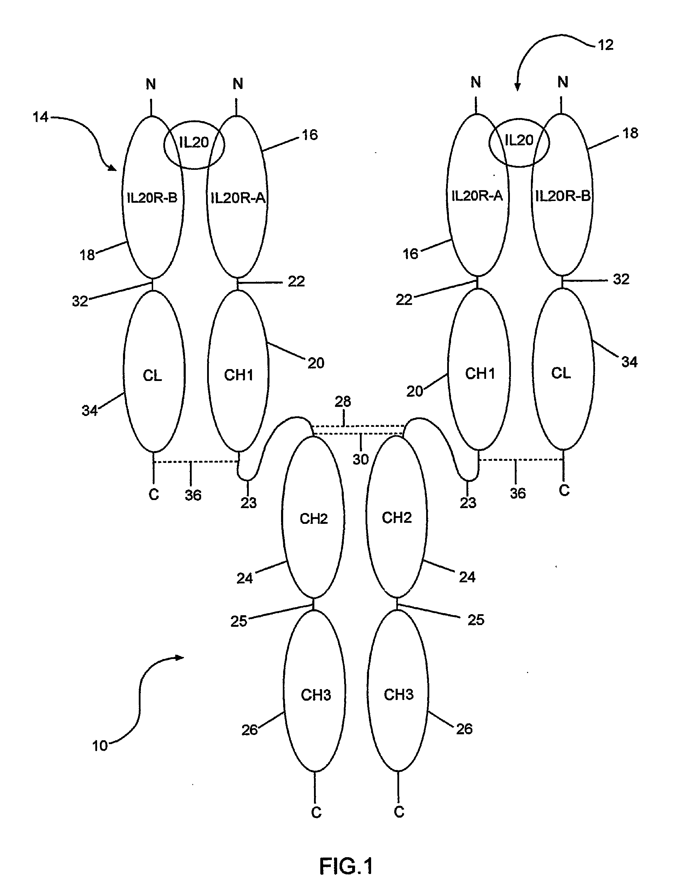

Figures 1-8 are schematic representations of a representative number of embodiments of the IL-20 soluble receptor.

We have shown that IL-20 upregulates IL-8. Inflammatory diseases in which IL-8 plays a significant role, and for which a decrease in IL-8 would be beneficial are, adult respiratory disease (ARD), septic shock, multiple organ failure, inflammatory lung injury such as asthma or bronchitis, bacterial pneumonia, psoriasis and inflammatory bowel disease such as ulcerative colitis and Crohn's disease, eczema, atopic dermatitis and contact dermatitis. Thus, antagonists to IL-20 can be used to treat these diseases.

The teachings of all the references cited herein are incorporated in their entirety by reference.

### Definitions

Prior to setting forth the invention in detail, it may be helpful to the understanding thereof to define the following terms.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

The term "complement/anti-complement pair" denotes non-identical moieties that form a non-covalently associated, stable pair under appropriate conditions. For instance, biotin and avidin (or streptavidin) are prototypical members of a complement/anti-complement pair. Other exemplary complement/anti-complement pairs include receptor/ligand pairs, antibody/antigen (or hapten or epitope) pairs, sense/antisense polynucleotide pairs, and the like. Where subsequent dissociation of the complement/anti-complement pair is desirable, the complement/anti-complement pair preferably has a binding affinity of <10⁹ M⁻¹.

The term "complements of a polynucleotide molecule" is a polynucleotide molecule having a complementary base sequence and reverse orientation as compared to a reference sequence. For example, the sequence 5' ATGCACGGG 3' is complementary to 5' CCCGTGCAT 3'.

The term "contig" denotes a polynucleotide that has a contiguous stretch of identical or complementary sequence to another polynucleotide. Contiguous sequences are said to "overlap" a given stretch of polynucleotide sequence either in their entirety or along a partial stretch of the polynucleotide. For example, representative contigs to the polynucleotide sequence 5'-ATGGCTTAGCTT-3' are 5'-TAGCTTgagtct-3' and 3'-gtcgacTACCGA-5'.

The term "degenerate nucleotide sequence" denotes a sequence of nucleotides that includes one or more degenerate codons (as compared to a reference polynucleotide molecule that encodes a polypeptide). Degenerate codons contain different triplets of nucleotides, but encode the same amino acid residue (i.e., GAU and GAC triplets each encode Asp).

The term "expression vector" is used to denote a DNA molecule, linear or circular, that comprises a segment encoding a polypeptide of interest operably linked to additional segments that provide for its transcription. Such additional segments include promoter and terminator sequences, and may also include one or more origins of replication, one or more selectable markers, an enhancer, a polyadenylation signal, etc. Expression vectors are generally derived from plasmid or viral DNA, or may contain elements of both.

The term "isolated", when applied to a polynucleotide, denotes that the polynucleotide has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art (see for example, Dynan and Tijan, *Nature 316*:774-78 (1985).

An "isolated" polypeptide or protein is a polypeptide or protein that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e. greater than 95% pure, more preferably greater than 99% pure. When used in this context, the term "isolated" does not exclude the presence of the same polypeptide in alternative physical forms, such as dimers or alternatively glycosylated or derivatized forms.

The term "operably linked", when referring to DNA segments, indicates that the segments are arranged so that they function in concert for their intended purposes, e.g., transcription initiates in the promoter and proceeds through the coding segment to the terminator.

A "polynucleotide" is a single- or double-stranded polymer of deoxyribonucleotide or ribonucleotide bases read from the 5' to the 3' end. Polynucleotides include RNA and DNA, and may be isolated from natural sources, synthesized *in vitro,* or prepared from a combination of natural and synthetic molecules. Sizes of polynucleotides are expressed as base pairs (abbreviated "bp"), nucleotides ("nt"), or kilobases ("kb"). Where the context allows, the latter two terms may describe polynucleotides that are single-stranded or double-stranded. When the term is applied to double-stranded molecules it is used to denote overall length and will be understood to be equivalent to the term "base pairs". It will be recognized by those skilled in the art that the two strands of a double-stranded polynucleotide may differ slightly in length and that the ends thereof may be staggered as a result of enzymatic cleavage; thus all nucleotides within a double-stranded polynucleotide molecule may not be paired. Such unpaired ends will in general not exceed 20 nucleotides in length.

A "polypeptide" is a polymer of amino acid residues joined by peptide bonds, whether produced naturally or synthetically. Polypeptides of less than about 10 amino acid residues are commonly referred to as "peptides".

The term "promoter" is used herein for its art-recognized meaning to denote a portion of a gene containing DNA sequences that provide for the binding of RNA polymerase and initiation of transcription. Promoter sequences are commonly, but not always, found in the 5' non-coding regions of genes.

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

The term "receptor" denotes a cell-associated protein that binds to a bioactive molecule (i.e., a ligand) and mediates the effect of the ligand on the cell. Membrane-bound receptors are characterized by a multi-domain structure comprising an extracellular ligand-binding domain and an intracellular effector domain that is typically involved in signal transduction. Binding of ligand to receptor results in a conformational change in the receptor that causes an interaction between the effector domain and other molecule(s) in the cell. This interaction in turn leads to an alteration in the metabolism of the cell. Metabolic events that are linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids. In general, receptors can be membrane bound, cytosolic or nuclear, monomeric (*e.g.*, thyroid stimulating hormone receptor, beta-adrenergic receptor) or multimeric (*e.g.*, PDGF receptor, growth hormone receptor, IL-3 receptor, GM-CSF receptor, G-CSF receptor, erythropoietin receptor and IL-6 receptor).

The term "secretory signal sequence" denotes a DNA sequence that encodes a polypeptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

Molecular weights and lengths of polymers determined by imprecise analytical methods (*e.g.*, gel electrophoresis) will be understood to be approximate values. When such a value is expressed as "about" X or "approximately" X, the stated value of X will be understood to be accurate to ±10%.

### Introduction

The present invention relates to the treatment of inflammation and inflammatory diseases in which IL-20 plays a role either in initiating or spreading or maintenance by administering to the afflicted individual an antagonist to IL-20. That antagonist can be an antibody to IL-20, a soluble receptor that binds to IL-20, an antisense molecule or an antibody that binds to either the IL-20RA subunit or the IL-20RB subunit of the IL-20 receptor.

IL-20 is defined and methods for producing it and antibodies to IL-20 are contained in International Patent Application No. PCT/US98/25228, publication no. WO 99/27103,published November 25, 1998 and U.S. Patent Application No. 09/313,458 filed May 17, 1999. The polynucleotide and polypeptide of human IL-20 are represented by SEQ ID NOs: 1 - 4, and mouse IL-20 by SEQ ID NOs: 5-9.

The receptor to IL-20 has been discovered and is a heterodimer comprised of the polypeptide termed 'IL-20RA' (formally called Zcytor7) and a polypeptide termed 'IL-20RB'. The IL-20RA polypeptide, nucleic acid that encodes it, antibodies to IL-20RA, and methods for producing it are disclosed in U.S. Patent No. 5,945,511 issued August 31, 1999. SEQ ID NOs: 10 -12 are the IL-20RA polynucleotides and polypeptides. The extracellular domain of the human IL-20RA is comprised of a polypeptide selected from the group consisting of SEQ ID NOs: 12, 55, 63 and 65, the full-length receptor subunit being comprised of SEQ ID NO: 11. The extracellular domain of mouse IL-20RA is SEQ ID NO: 38, SEQ ID NO: 37 being the entire mouse IL-20RA.

The extracellular domain of IL-20RB (SEQ ID NOs: 13-14, and a variant SEQ ID NOs: 18 and 19) is comprised of a polypeptide selected from the group consisting of SEQ ID NOs: 15, 59, 61, 67, 68 and 69. Preferably, the extracellular domain of the IL-20RA polypeptide and the extracellular domain of the IL-20RB polypeptide are covalently linked together. In a preferred embodiment one extracellular subunit polypeptide has a constant region of a heavy chain of an immunoglobulin fused to its carboxy terminus and the other extracellular subunit has a constant light chain of an immunoglobulin (Ig) fused to its carboxy terminus such that the two polypeptides come together to form a soluble receptor and a disulfide bond is formed between the heavy and the light Ig chains. In another method, a peptide linker could be fused to the two carboxy-termini of the polypeptides to form a covalently bonded soluble receptor.

SEQ ID NOs: 22 and 23 are constructs of the extracellular domain of IL-20RA fused to a mutated human immunoglobulin gamma 1 constant region produced according to the procedure set forth in example 5. SEQ ID NO: 62 is the predicted mature sequence without the signal sequence. SEQ ID NOs: 20 and 21 are constructs of the extracellular domain of IL-20RB fused to wild type human immunoglobulin kappa light chain constant region produced according to the procedure of example 5. SEQ ID NO: 60 is the predicted mature sequence without the signal sequence. Figure 1 depicts the heterotetramer produced by example 5.

SEQ ID NOs: 52 and 53 are constructs of the extracellular domain of IL-20RA fused to a mutated human immunoglobulin gamma 1 constant region produced according to the procedure set forth in example 12. SEQ ID NO: 54 is the predicted mature sequence without the signal sequence. SEQ ID NOs: 56 and 57 are constructs of the extracellular domain of IL-20RB fused to wild type human immunoglobulin kappa light chain constant region produced according to the procedure of example 12. SEQ ID NO: 58 is the predicted mature sequence without the signal sequence. The resultant heterotetramer is almost identical to that produced by example 5, the primary difference being the absence of a polypeptide linker between the extracellular domains and the beginning of the Ig constant regions, 22 in Figure 1. Hereinafter, the term "extracellular domain of a receptor" means the extracellular domain of the receptor or a portion of the extracellular domain that is necessary for binding to its ligand, in this case the ligand being IL-20.

One can link together the extracellular domains of IL-20RA and IL-20RB in a number of ways such that the resultant soluble receptor can bind to IL-20. Figures 1-8 illustrate a representative number of embodiments of the present invention. Common elements in each of the drawings are given the same number. Figure 1 represents the embodiment of the present invention produced according to example 5 below. The soluble receptor construct, designated 10, is comprised of two IL-20 binding site polypeptide chains designated 12 and 14. Each binding site is comprised of the extracellular domain of IL-20RA, designated 16, and the extracellular domain of IL-20RB designated 18.

The extracellular domain, 16, of IL-20RA is linked to the constant heavy one (CH1) domain, 20, of the human immunoglobulin gamma 1 heavy chain constant region via linker 22, which is SEQ ID NO:72. The CH1 domain, 20, is then linked to the CH2 domain, 24, via hinge region 23. The CH2 domain, 24, is linked to the CH3 domain, 26, via hinge region 25.

Comparing the construct of Figure 1 with SEQ ID NO:22, the extracellular domain, 16, of IL-20RA extends from amino acid residues 36, a valine, to and including amino acid residue 249, a glutamine of SEQ ID NO:22. Polypeptide linker, 22, extends from amino acid residue 250, a glycine to and including amino acid residue 264, a serine, of SEQ ID NO:22. The CH1 domain, 22 of Figure 1, extends from amino acid residue 265, an alanine, to and including amino acid residue 362, a valine, of SEQ ID NO:22. Hinge region 23 of Figure 1 extends from amino acid residue 363, a glutamic acid to and including amino acid residue 377, a proline, of SEQ ID NO: 22. Chains 12 and 14 are disulfide-bonded together by means of disulfide bonds 28 and 30. The disulfide bonds are formed between the heavy chains by the cysteine residues at positions 373 and 376 of SEQ ID NO: 22 of each of the two heavy chains.

Extracellular domain, 18, of 1L-20RB is linked to the constant region of the human kappa light chain (CL), 34 of Figure 1 via polypeptide linker 32, which is the polypeptide SEQ ID NO: 72. The extracellular domain, 18, of IL-20RB extends from amino acid residue 30, a valine, to and including amino acid residue 230, an alanine, of SEQ ID NO: 20. Polypeptide linker, 32, extends from amino acid residue 231, a glycine, to and including amino acid residue 245, a serine, of SEQ ID NO:20. The kappa constant light region, 34, extends from amino acid residue 246, an arginine, to and including the final amino acid residue 352, a cysteine, of SEQ ID NO:20. The cysteine at position 352 of SEQ ID NO: 20 forms a disulfide bond, 36 in Figure 1, with the cysteine at position 367 of SEQ ID NO: 22. The constant light chain 34 is thus linked to the hinge region, 23, by disulfide bond, 36. In this way, the extracellular domain, 16, of IL-20RA is linked to the extracellular domain, 18, of IL-20RB to form a soluble receptor.

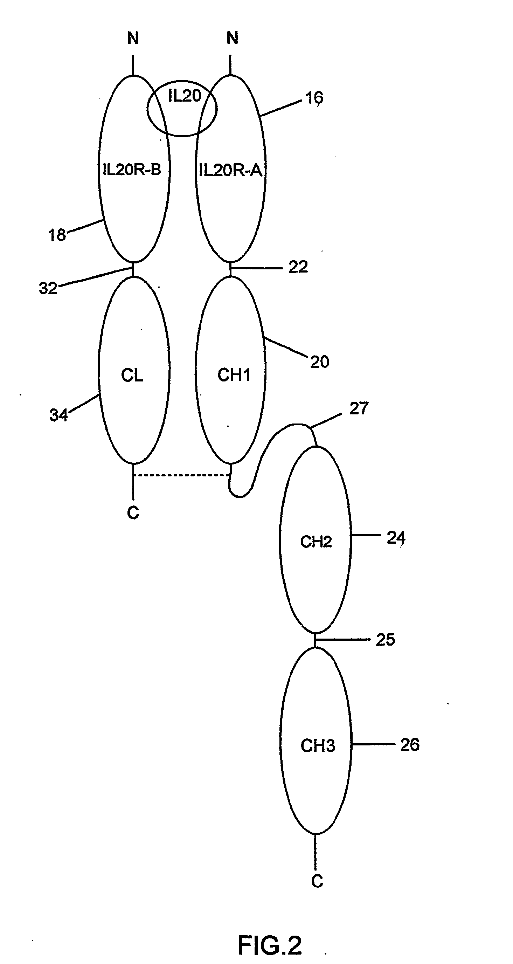

If the cysteine residues at positions 373 and 376 of SEQ ID NO:22 were changed to different amino acid residues, the two IL-20 binding polypeptides, 12 and 14, would not be disulfide bonded together and would form a construct shown in Figure 2. having hinge region, 27.

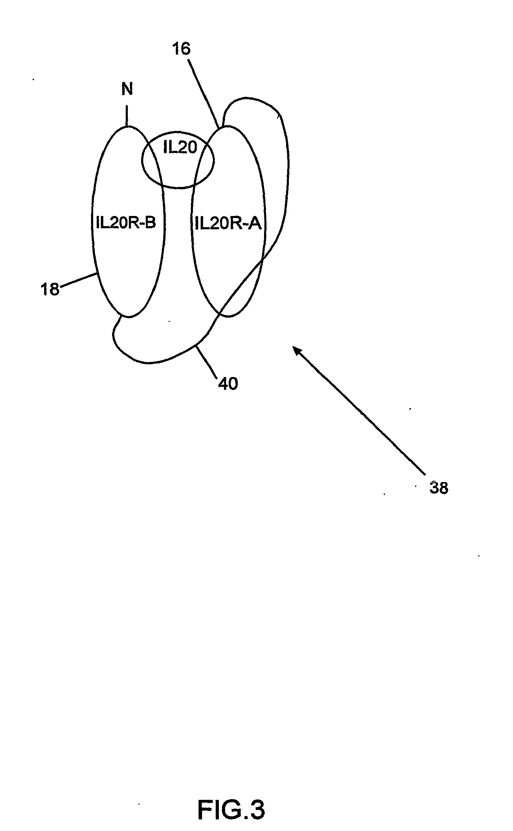

Figure 3 shows a very simple soluble receptor 38 of the present invention wherein extracellular domain, 16, of IL-20RA is connected to the extracellular domain, 18, of IL-20RB by means of a polypeptide linker, 40. The polypeptide linker extends from the amino terminus of extracellular domain, 16, of IL-20RA and is connected to the carboxyl terminus of the extracellular domain, 18, of IL-20RB. The polypeptide linker should be between 100-240 amino acids in length, preferably about 170 amino acid residues in length. A suitable linker would be comprised of glycine and serine residues. A possible linker would be multiple units of SEQ ID NO: 72, preferably about 12.

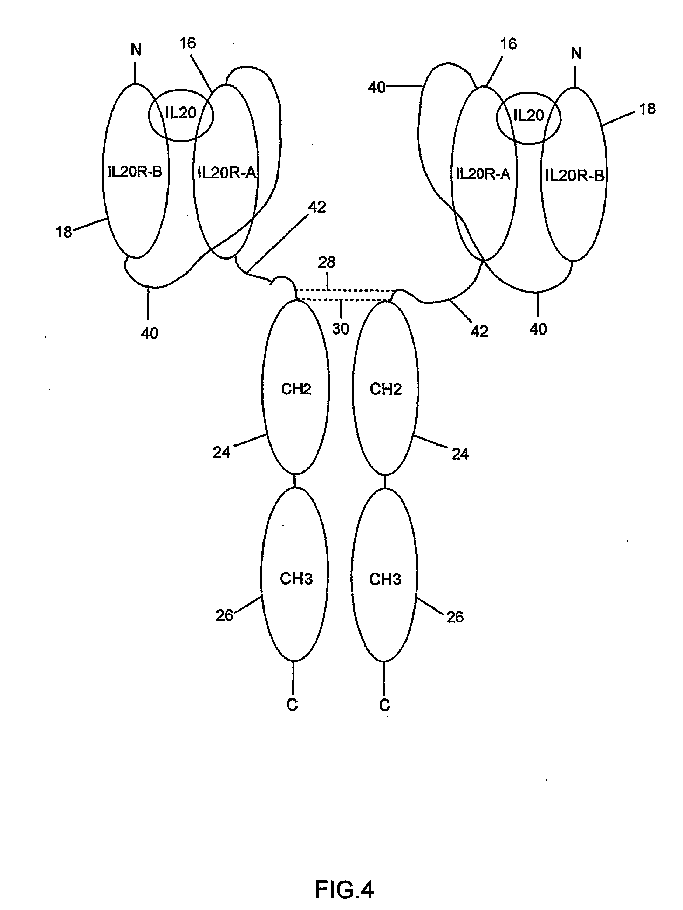

Figure 4 shows an embodiment that has the extracellular domain, 16, of IL-20RA linked to the extracellular domain, 18, of IL-20RB by means of linker 40, as in Figure 3. While the extracellular domain, 16, of IL-20RA is linked to the CH1 domain, 20, as in Figure 1 by means of polypeptide linker 42, which should be about 30 amino acid residues in length. An ideal linker would be comprised of glycine and serine as in SEQ ID NO: 72, and the hinge sequence, 23 of Figure 1.

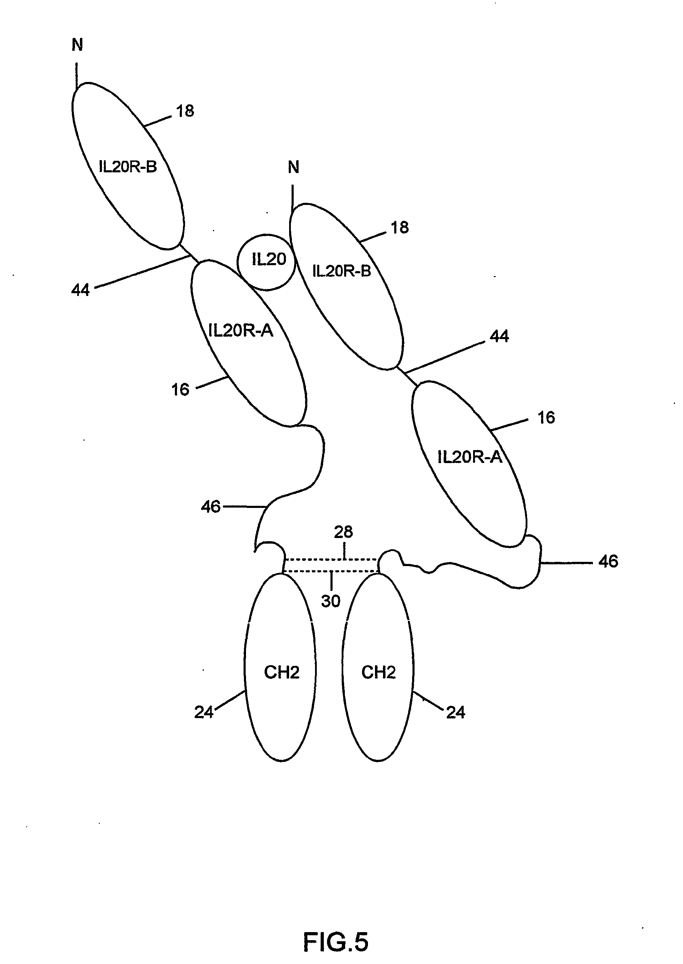

Figure 5 shows another possible embodiment of the present invention. In this embodiment, a polypeptide linker 44 of about 15 amino acid residue, e.g. SEQ ID NO: 72, links the carboxyl terminus of the extracellular domain, 18, of IL-20RB with the amino terminus of the extracellular domain, 16, of IL-20RA. A polypeptide linker 46 of about 30 amino acid residues extends from the carboxy terminus of the extracellular domain, 16, of IL-20RA to the CH2 domain. The carboxyl terminus of linker 46 would preferably be comprised of the hinge region extending from amino acid residue 363, a glutamic acid to and including amino acid residue 377, a proline, of SEQ ID NO: 22. Nonetheless, polypeptide linker 46 would ideally have at least one cysteine residue at its carboxyl terminus so a disulfide bond could be formed.

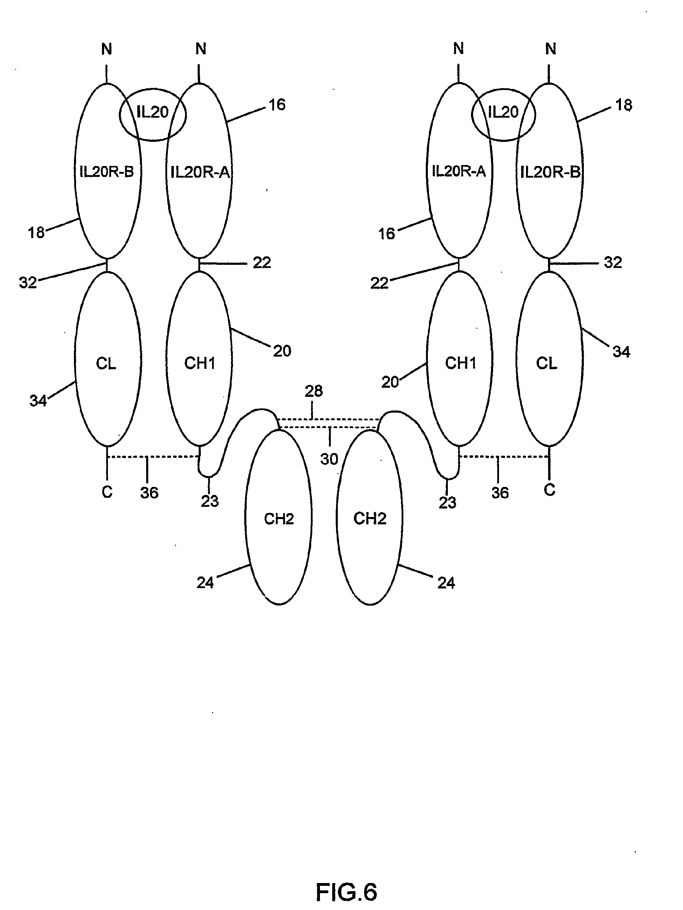

The soluble IL-20 receptor of Figure 6 is identical to that of Figure 1 except for the CH3 domain, 26 of Figure 1, is not present on the embodiment of Figure 6. The CH3 region begins at amino acid residue 488, a glycine, and extends to the last residue 594 of SEQ ID NO: 22.

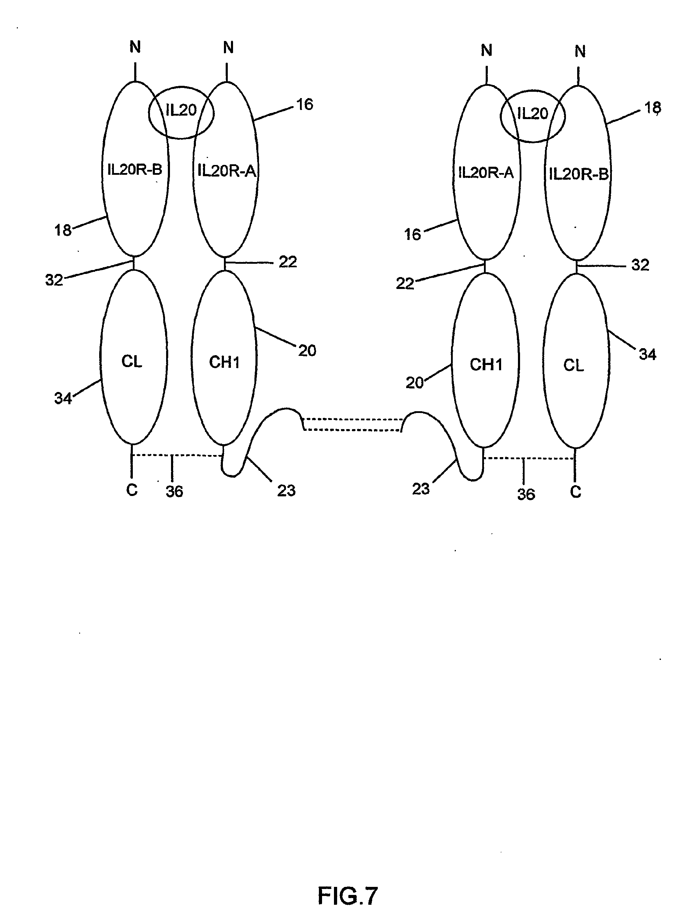

Figure 7 shows a soluble IL-20 receptor construct that is identical to the construct of Figure 1 except both the CH2, and CH3 domains are absent. The CH2 and CH3 domains run from amino acid residue 378, an alanine, to the end of the polypeptide sequence of SEQ ID NO: 22.

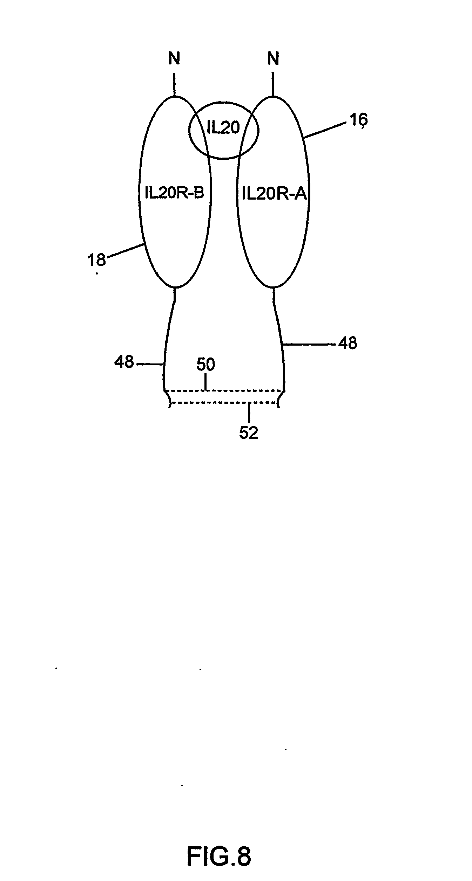

Figure 8 shows a construct wherein both IL-20RA, 16, and IL-20RB have a polypeptide linker, 48, fused to their respective carboxyl termini. Each polypeptide linker has two cysteine residues such that when they are expressed the cysteines form two disulfide bonds, 50 and 52. In this case the polypeptide linker is comprised of the hinge region, 23 in Figure 1. The hinge region is comprised of amino acid residues 363, a glutamine, to and including amino acid residue 377 of SEQ ID NO: 22.

In another aspect of the invention, a method is provided for producing a soluble receptor comprised of extracellular domains of IL-20RA and IL-20RB comprising (a) introducing into a host cell a first DNA sequence comprised of a transcriptional promoter operatively linked to a first secretory signal sequence followed downstream by and in proper reading frame the DNA that encodes the extracellular portion of IL-20RA and the DNA that encodes an immunoglobulin light chain constant region;(b) introducing into the host cell a second DNA construct comprised of a transcriptional promoter operatively linked to a second secretory signal followed downstream by and in proper reading frame a DNA sequence that encodes the extracellular portion of IL-20RB and a DNA sequence that encodes an immunoglobulin heavy chain constant region domain selected from the group consisting of C_{H}1, C_{H}2, C_{H}3 and C_{H}4; (c) growing the host cell in an appropriate growth medium under physiological conditions to allow the secretion of a fusion protein comprised of the extracellular domain of IL-20RA and IL-20RB; and (d) isolating the polypeptide from the host cell. In one embodiment, the second DNA sequence further encodes an immunoglobulin heavy chain hinge region wherein the hinge region is joined to the heavy chain constant region domain. In another embodiment, the second DNA sequence further encodes an immunoglobulin variable region joined upstream of and in proper reading frame with the immunoglobulin heavy chain constant region.

In an alternative embodiment, a method is provided for producing a soluble receptor comprised of the extracellular domains of IL-20RA and IL-20RB comprising (a) introducing into a host cell a first DNA sequence comprised of a transcriptional promoter operatively linked to a first secretory signal sequence followed downstream by and in proper reading frame the DNA that encodes the extracellular portion of IL-20RB and the DNA that encodes an immunoglobulin light chain constant region;(b) introducing into the host cell a second DNA construct comprised of a transcriptional promoter operatively linked to a second secretory signal followed downstream by and in proper reading frame a DNA sequence that encodes the extracellular portion of IL-20RA and a DNA sequence that encodes an immunoglobulin heavy chain constant region domain selected from the group consisting of C_{H}1, C_{H}2, C_{H}3 and C_{H}4; (c) growing the host cell in an appropriate growth medium under physiological conditions to allow the secretion of a dimerized heterodimeric fusion protein comprised of the extracellular domain of IL-20RA and IL-20RB; and (d) isolating the dimerized polypeptide from the host cell. In one embodiment, the second DNA sequence further encodes an immunoglobulin heavy chain hinge region wherein the hinge region is joined to the heavy chain constant region domain. In another embodiment, the second DNA sequence further encodes an immunoglobulin variable region joined upstream of and in proper reading frame with the immunoglobulin heavy chain constant region. (See U.S. Patent No. 5,843,725.)

As used herein, the term "antibodies" includes polyclonal antibodies, affinity-purified polyclonal antibodies, monoclonal antibodies, and antigen-binding fragments, such as F(ab')₂ and Fab proteolytic fragments. Genetically engineered intact antibodies or fragments, such as chimeric antibodies, Fv fragments, single chain antibodies and the like, as well as synthetic antigen-binding peptides and polypeptides, are also included. Non-human antibodies may be humanized by grafting non-human CDRs onto human framework and constant regions, or by incorporating the entire non-human variable domains (optionally "cloaking" them with a human-like surface by replacement of exposed residues, wherein the result is a "veneered" antibody). In some instances, humanized antibodies may retain non-human residues within the human variable region framework domains to enhance proper binding characteristics. Through humanizing antibodies, biological half-life may be increased, and the potential for adverse immune reactions upon administration to humans is reduced. The binding affinity of an antibody can be readily determined by one of ordinary skill in the art, for example, by Scatchard analysis. A variety of assays known to those skilled in the art can be utilized to detect antibodies that bind to protein or peptide. Exemplary assays are described in detail in *Antibodies: A Laboratory Manual,* Harlow and Lane (Eds.) (Cold Spring Harbor Laboratory Press, 1988). Representative examples of such assays include: concurrent immunoelectrophoresis, radioimmunoassay, radioimmuno-precipitation, enzyme-linked immunosorbent assay (ELISA), dot blot or Western blot assay, inhibition or competition assay, and sandwich assay.

We have shown that IL-20 up-regulates IL-8. Inflammatory diseases in which IL-8 plays a significant role, and for which a decrease in IL-8 would be beneficial are, adult respiratory disease (ARD), septic shock, multiple organ failure, inflammatory lung injury such as asthma or bronchitis, bacterial pneumonia, psoriasis and inflammatory bowel disease such as ulcerative colitis and Crohn's disease, eczema, atopic dermatitis and contact dermatitis. Thus, antagonists to IL-20 can be used to treat these diseases.

### Biology of IL-20, Its receptor and Its Role in Psoriasis

Two orphan class II cytokine receptors, both of which are expressed in skin, were identified as IL-20 receptor subunits. Both IL-20 receptor subunits are required for ligand binding, distinguishing their role from that of subunits in the four other known class II cytokine receptors. IL-20RA and IL-20RB are also coexpressed in a number of human tissues besides skin, including ovary, adrenal gland, testis, salivary gland, muscle, lung, kidney, heart and to a lesser degree the small intestine suggesting additional target tissues for IL-20 action. Additionally, we have detected IL-20RA mRNA but not IL-20RB mRNA in several human tissues including stomach, thyroid, pancreas, uterus, brain and prostate suggesting that IL-20RA may partner with other class II receptor subunits. We conclude that the IL-20 heterodimeric receptor is structurally similar to other class II cytokine receptors and is expressed in skin where we have demonstrated activity of the IL-20 ligand.

Two lines of evidence indicate that a role IL-20 and its receptor are involved in psoriasis. This multigenic skin disease is characterized by increased keratinocyte proliferation, altered keratinocyte differentiation, and infiltration of immune cells into the skin. The first line of evidence for a role of IL-20 in psoriasis is that the observed hyperkeratosis and thickened epidermis in the transgenic mice that resemble human psoriatic abnormalities. Decreased numbers of tonofilaments, thought to be related to defective keratinization, are a striking feature of human psoriasis. Intramitochondrial inclusions have been found in both chemically induced and naturally occurring hyperplastic skin conditions in mice. The cause of the inclusions and their effects on mitochondrial function, if any, are unknown. We conclude that IL-20 transgenic mice exhibit many of the characteristics observed in human psoriasis.

A second line of evidence that implicates the IL-20 receptor in psoriasis is that both IL-20RA and IL-20RB mRNA are markedly upregulated in human psoriatic skin compared to normal skin. Both IL-20 receptor subunits are expressed in keratinocytes throughout the epidermis and are also expressed in a subset of immune and endothelial cells. We propose that increased expression of an activated IL-20 receptor may alter the interactions between endothelial cells, immune cells and keratinocytes, leading to dysregulation of keratinocyte proliferation and differentiation.

A crucial step in understanding the function of a novel cytokine is the identification and characterization of its cognate receptor. We have successfully used a structure-based approach to isolate a novel interleukin that ultimately led to the isolation of its receptor. IL-20 stimulates signal transduction in the human keratinocyte HaCaT cell line, supporting a direct action of this novel ligand in skin. In addition, IL-1β, EGF and TNF-α, proteins known to be active in keratinocytes and to be involved with proliferative and pro-inflammatory signals in skin, enhance the response to IL-20. In both HaCaT and BHK cells expressing the IL-20 receptor, IL-20 signals through STAT3.

### Use of Antagonist to IL-20 to Treat Psoriasis

As indicated in the discussion above and the examples below, IL-20 is involved in the pathology of psoriasis. The present invention is in particular a method for treating psoriasis by administering antagonists to IL-20. The antagonists to IL-20 can either be a soluble receptor that binds to IL-20 or antibodies, single chain antibodies or fragments of antibodies that bind to either IL-20 or the IL-20 receptor. The antagonists will thus prevent activation of the IL-20 receptor.

Psoriasis is one of the most common dermatologic diseases, affecting up to 1 to 2 percent of the world's population. It is a chronic inflammatory skin disorder characterized by erythematous, sharply demarcated papules and rounded plaques, covered by silvery micaceous scale. The skin lesions of psoriasis are variably pruritic. Traumatized areas often develop lesions of psoriasis. Additionally, other external factors may exacerbate psoriasis including infections, stress, and medications, e.g. lithium, beta blockers, and anti-malarials.

The most common variety of psoriasis is called plaque type. Patients with plaque-type psoriasis will have stable, slowly growing plaques, which remain basically unchanged for long periods of time. The most common areas for plaque psoriasis to occur are the elbows knees, gluteal cleft, and the scalp. Involvement tends to be symmetrical. Inverse psoriasis affects the intertriginous regions including the axilla, groin, submammary region, and navel, and it also tends to affect the scalp, palms, and soles. The individual lesions are sharply demarcated plaques but may be moist due to their location. Plaque-type psoriasis generally develops slowly and runs an indolent course. It rarely spontaneously remits.

Eruptive psoriasis (guttate psoriasis) is most common in children and young adults. It develops acutely in individuals without psoriasis or in those with chronic plaque psoriasis. Patients present with many small erythematous, scaling papules, frequently after upper respiratory tract infection with beta-hemolytic streptococci. Patients with psoriasis may also develop pustular lesions. These may be localized to the palms and soles or may be generalized and associated with fever, malaise, diarrhea, and arthralgias..

About half of all patients with psoriasis have fingernail involvement, appearing as punctate pitting, nail thickening or subungual hyperkeratosis. About 5 to 10 percent of patients with psoriasis have associated joint complaints, and these are most often found in patients with fingernail involvement. Although some have the coincident occurrence of classic Although some have the coincident occurrence of classic rheumatoid arthritis, many have joint disease that falls into one of five type associated with psoriasis: (1) disease limited to a single or a few small joints (70 percent of cases); (2) a seronegative rheumatoid arthritis-like disease; (3) involvement of the distal interphalangeal joints; (4) severe destructive arthritis with the development of "arthritis mutilans"; and (5) disease limited to the spine.

Psoriasis can be treated by administering antagonists to IL-20. The preferred antagonists are either a soluble receptor to IL-20 or antibodies, antibody fragments or single chain antibodies that bind to either the IL-20 receptor or to IL-20. The antagonists to IL-20 can be administered alone or in combination with other established therapies such as lubricants, keratolytics, topical corticosteroids, topical vitamin D derivatives, anthralin, systemic antimetabolites such as methotrexate, psoralen-ultraviolet-light therapy (PUVA), etretinate, isotretinoin, cyclosporine, and the topical vitamin D3 derivative calcipotriol. The antagonists, in particularly the soluble receptor or the antibodies that bind to IL-20 or the IL-20 receptor can be administered to individual subcutaneously, intravenously, or transdermally using a cream or transdermal patch that contains the antagonist of IL-20. If administered subcutaneously, the antagonist can be injected into one or more psoriatic plaques. If administered transdermally, the antagonists can be administered directly on the plaques using a cream containing the antagonist to IL-20.

### Use of Antagonists to IL-20 to Treat Inflammatory Conditions of the Lung.

Antagonists to IL-20 can be administered to a person who has asthma, bronchitis or cystic fibrosis or other inflammatory lung disease to treat the disease. The antagonists can be administered by any suitable method including intravenous, subcutaneous, bronchial lavage, and the use of inhalant containing an antagonist to IL-20.

### Administration of Antagonists to IL-20

The quantities of antagonists to IL-20 necessary for effective therapy will depend upon many different factors, including means of administration, target site, physiological state of the patient, and other medications administered. Thus, treatment dosages should be titrated to optimize safety and efficacy. Typically, dosages used *in vitro* may provide useful guidance in the amounts useful for *in vivo* administration of these reagents. Animal testing of effective doses for treatment of particular disorders will provide further predictive indication of human dosage. Methods for administration include oral, intravenous, peritoneal, intramuscular, transdermal or administration into the lung or trachea in spray form by means or a nebulizer or atomizer. Pharmaceutically acceptable carriers will include water, saline, buffers to name just a few. Dosage ranges would ordinarily be expected from 1µg to 1000µg per kilogram of body weight per day. A dosage for an average adult of the IL-20 soluble receptor would be about 25 mg given twice weekly as a subcutaneous injection. A dosage of an antibody that binds to IL-20 would be about 25 mg given twice weekly. A mixture of antibodies that bind to the IL-20RA and IL-20RB subunits would be about 25 mg given twice weekly for an adult. Injections could be given at the site of psoriatic lesions for the treatment of psoriasis. For subcutaneous or intravenous administration of the antagonist to IL-20, the antibody or soluble receptor can be in phosphate buffered saline. Also in skin diseases such as psoriasis, the antagonist to IL-20 can be administered via an ointment or transdermal patch. The doses by may be higher or lower as can be determined by a medical doctor with ordinary skill in the art. For a complete discussion of drug formulations and dosage ranges *see Remington's Pharmaceutical Sciences,* 18^{th} Ed., (Mack Publishing Co., Easton, Penn., 1996), and *Goodman and Gilman's: The Pharmacological Bases of Therapeutics,* 9^{th} Ed. (Pergamon Press 1996).

The invention is further illustrated by the following non-limiting examples.

### Example 1

### Up-regulation of IL-8 by IL-20

### Methods:

Normal Human Epidermal neonatal keratinocytes (NHEK) (from Clonetics) at passage 2 were plated and grown to confluency in 12 well tissue culture plates. KGM (Keratinocyte growth media) was purchased from Clonetics. When cells reached confluency, they were washed with KGM media minus growth factors = KBM (keratinocyte basal media). Cells were serum starved in KBM for 72 hours prior to the addition of test compounds. Thrombin at 1 I.U./mL and trypsin at 25nM were used as positive controls. One mL of media/well was added. KBM only was used as the negative control.

IL-20 was made up in KBM media and added at varying concentrations, from 2.5µg/ml down to 618ng/mL in a first experiment and from 2.5µg/mL down to 3ng/mL in a second experiment.

Cells were incubated at 37° C, 5% CO₂ for 48 hours. Supernatants were removed and frozen at -80° C for several days prior to assaying for IL-8 and GM-CSF levels. Human IL-8 Immunoassay kit # D8050 (RandD Systems, Inc.) and human GM-CSF Immunoassay kit # HSGMO (RandD Systems, Inc.) were used to determine cytokine production following manufacturer's instructions.

### Results

The results indicated that the expression of IL-8 and GM-CSF were induced by IL-20.

### Example 2

### Cloning of IL-20RB

### Cloning of IL-20RB coding region

Two PCR primers were designed based on the sequence from International Patent Application No. PCT/US99/03735 (publication no. WO 99/46379) filed on March 8, 1999. SEQ ID NO: 16 contains the ATG (Met1) codon with an EcoRI restriction site, SEQ ID NO: 17 contains the stop codon (TAG) with an XhoI restriction site. The PCR amplification was carried out using a human keratinocyte (HaCaT) cDNA library DNA as a template and SEQ ID NO: 16 and SEQ ID NO: 17 as primers. The PCR reaction was performed as follows: incubation at 94°C for 1 min followed by 30 cycles of 94°C for 30 sec and 68°C for 2 min, after additional 68°C for 4 min, the reaction was stored at 4°C. The PCR products were run on 1% Agarose gel, and a 1 kb DNA band was observed. The PCR products were cut from the gel and the DNA was purified using a QIAquick Gel Extraction Kit (Qiagen). The purified DNA was digested with EcoRI and XhoI, and cloned into a pZP vector that was called pZP7N. A pZP plasmid is a mammalian expression vector containing an expression cassette having the mouse metallothionein-1 promoter, human tPA leader peptide, multiple restriction sites for insertion of coding sequences, a Glu-Glu tag, and a human growth hormone terminator. The plasmid also has an E. coli origin of replication, a mammalian selectable marker expression unit having an SV40 promoter, an enhancer and an origin of replication, as well as a DHFR gene, and the SV40 terminator. Several IL-20RB-pZP7N clones were sequenced. They all contain three non-conservative mutations compared with the sequence of IL-20RB in PCT/US99/03735: (sequence IL-20RB-pZP7N), 146 Pro (CCC) -- Thr (ACC), 148 His (CAT) -- Asp (GAT), and 171 Thr (ACG) -- Arg (AGG).

To verify the three substitutions in IL-20RB-pZP7N clone, PCR amplification was carried out using three difference cDNA sources -- fetal skin marathon cDNA, HaCaT cDNA library DNA, and prostate smooth muscle cDNA library DNA -- as templates. The PCR products were gel purified and sequenced. The sequence of each of the three PCR products was consistent with that of the IL-20RB-pZP7N clone. IL-20RB is SEQ ID NO: 13 and 14, and the mature extracellular domain is SEQ ID NO: 15.

### Example 3

### Binding of IL-20 to IL-20RB/ IL-20RA Heterodimer

A cell-based binding assay was used to verify IL-20 binds to IL-20RAIL-20RB heterodimer.

Expression vectors containing known and orphan Class II cytokine receptors (including IL-20RA and IL-20RB) were transiently transfected into COS cells in various combinations, which were then assayed for their ability to bind biotin-labeled IL-20 protein. The results show IL-20RB- IL-20RA heterodimer is a receptor for IL-20. The procedure used is described below.

The COS cell transfection was performed in a 12-well tissue culture plate as follows: 0.5 µg DNA was mixed with medium containing 5 µl lipofectamine in 92 µl serum free Dulbecco's modified Eagle's medium (DMEM) (55 mg sodium pyruvate, 146 mg L-glutamine, 5 mg transferrin, 2.5 mg insulin, 1 µg selenium and 5 mg fetuin in 500 ml DMEM), incubated at room temperature for 30 minutes and then added to 400 µl serum free DMEM media. This 500 µl mixture was then added to 1.5 x 10⁵ COS cells/well and incubated for 5 hours at 37° C. 500 µl 20% fetal bovine serum (FBS) DMEM media was added and incubated overnight.

The assay, a modification of the "secretion trap" (Davis, S., *et al., Cell 87*: 1161-1169 (1996), was performed as follows: cells were rinsed with PBS/1% bovine serum albumin (BSA) and blocked for 1 hour with TNB (0.1 M Tris-HCI, 0.15 M NaCl and 0.5% Blocking Reagent (NEN Renaissance TSA-Direct Kit Cat# NEL701) in water). This was followed by a one-hour incubation with 3 µg/ml biotinylated IL-20 protein in TNB. Cells were washed with PBS/1% BSA and incubated for another hour with 1:300 diluted streptavidin-HRP (NEN kit) in TNB. Following another wash, cells were fixed for 15 minutes with 1.8% Formaldehyde in phosphate-buffered saline (PBS). Cells were then washed with TNT (0.1 M Tris-HCL; 0.15 M NaCl, and 0.05% Tween-20 in water). Positive binding signals were detected following a five-minute incubation with fluorescein tyramide reagent diluted 1:50 in dilution buffer (NEN kit). Cells were washed with TNT, preserved with Vectashield Mounting Media (Vector Labs) diluted 1:5 in TNT, and visualized using an FITC filter on an inverted fluorescent microscope.

### Example 4

### Up-regulation of Inflammatory Cytokines by IL-20

### Cell Treatment

The human keratinocyte cell line, HaCaT was grown at 37°C to several days post-confluence in T-75 tissue culture flasks. At this point, normal growth media (DMEM + 10% FBS) was removed and replaced with serum-free media. Cells were then incubated for two days at 37°C. DMEM was then removed and four flasks of cells per treatment were treated with one of each of the following conditions for four hours at 37°C: recombinant human (rh) IL-1 alpha at 5 ng/mL, rh IL-1 alpha at 20 ng/mL, rh IL-1 alpha at 5 ng/mL + IL-20 at 1µg/mL, IL-20 at 1µg/mL, or rh IL-10 at 10 ng/mL.

### RNA Isolation

Following cytokine treatment, media was removed and cells were lysed using a guanidium thiocyanate solution. Total RNA was isolated from the cell lysate by an overnight spin on a cesium chloride gradient. The following day, the RNA pellet was resuspended in a TE/SDS solution and ethanol precipitated. RNA was then quantitated using a spectrophotometer, followed by a DNase treatment as per Section V.B. of Clontech's Atlas^{™} cDNA Expression Arrays User Manual (version PT3140-1/PR9X390, published 11/5/99). Quality of RNA samples was verified by purity calculations based on spec readings, and by visualization on agarose gel. Genomic contamination of the RNA samples was ruled out by PCR analysis of the beta-actin gene.

### Probe Synthesis

Clontech's protocols for polyA+ enrichment, probe synthesis and hybridization to Atlas^{™} arrays were followed (see above, plus Atlas^{™} Pure Total RNA Labeling System User Manual, PT3231-1/PR96157, published 6/22/99). Briefly, polyA+ RNA was isolated from 50 mg of total RNA using streptavidin coated magnetic beads (by Clontech, Paolo Alto, CA) and a magnetic particle separator. PolyA+ RNA was then labeled with ^{alpha32}P-dATP via RT-PCR. Clontech CDS primers specific to the 268 genes on the Atlas^{™} human cytokine/receptor array (Cat. #7744-1) were used in the reaction. Labeled probe was isolated using column chromatography and counted in scintillation fluid.

### Array membrane Hybridization

Atlas^{™} arrays were pre-hybridized with Clontech ExpressHyb plus 100 mg/mL heat denatured salmon sperm DNA for at least thirty minutes at 68°C with continuous agitation. Membranes were then hybridized with 1.9 x 10⁶ CPM/mL (a total of 1.14 x 10⁷ CPM) overnight at 68°C with continuous agitation. The following day, membranes were washed for thirty minutes x 4 in 2X SSC, 1% SDS at 68° C, plus for thirty minutes x 1 in 0.1X SSC, 0.5% SDS at 68°C, followed by one final room temperature wash for five minutes in 2X SSC. Array membranes were then placed in Kodak plastic pouches sealed and exposed to a phosphor imager screen overnight at room temperature. The next day, phosphor screens were scanned on a phosphor imager and analyzed using Clontech's AtlasImage^{™} 1.0 software.

### Results

### Genes Up-regulated by IL-20

1. Tumor necrosis factor (TNF) was up-regulated 1.9-2.4 fold by IL-20.
2. Placental growth factors 1 & 2 (PLGF) were up-regulated 1.9-2.0 fold by IL-20.
3. Coagulating factor II receptor was up-regulated 2.0-2.5 fold by IL-20.
4. Calcitonin receptor was up-regulated 2.2-2.3 fold by IL-20.
5. TNF-inducible hyaluronate-binding protein TSG-6 was up-regulated 2.1-2.2 fold by IL-20.
6. Vascular endothelial growth factor (VEGF) receptor-1 precursor, tyrosine-protein kinase receptor (FLT-1) (SFLT) was up-regulated 2.1-2.7 fold by IL-20.
7. MRP-8 (calcium binding protein in macrophages MIF- related) was up-regulated 2.9-4.1 fold by IL-20.
8. MRP-14 (calcium binding protein in macrophages MIF-related) was up-regulated 3.0-3.8 fold by IL-20.
9. Relaxin H2 was up-regulated 3.14 fold by IL-20.
10. Transforming growth factor beta (TGFβ) receptor III 300 kDa was up-regulated 2.4-3.6 fold by IL-20.

### Genes Showing Synergy with IL-20 + IL-1 Treatment

1. Bone morphogenic protein 2a was up-regulated 1.8 fold with IL-20 treatment alone, 2.5 fold with IL-1 treatment alone, and 8.2 fold with both IL-20 and IL-1 treatment together.
2. MRP-8 was up-regulated 2.9 fold with IL-20 treatment alone, 10.7 fold with IL-1 treatment alone and 18.0 fold with both IL-20 and IL-1 treatment together.
3. Erythroid differentiation protein (EDF) was up-regulated 1.9 fold with IL-20 treatment alone, 9.7 fold with IL-1 treatment alone and 19.0 fold with both IL-20 and IL-1 treatment together.
4. MRP-14 (calcium binding protein in macrophages, MIF related) was up-regulated 3.0 fold with IL-20 treatment alone, 12.2 fold with IL-1 treatment alone and 20.3 fold with both 1L-20 and IL-1 treatment together.
5. Heparin-binding EGF-like growth factor was up-regulated 2.0 fold with IL-20 treatment alone, 14 fold with IL-1 treatment alone and 25.0 fold with both IL-20 and IL-1 treatment together.
6. Beta-thromboglobulin-like protein was up-regulated 1.5 fold with IL-20 treatment alone, 15 fold with IL-1 treatment alone and 27 fold with both IL-20 and IL-1 treatment together.
7. Brain-derived neurotrophic factor (BDNF) was up-regulated 1.7 fold with IL-20 treatment alone, 25 fold with IL-1 treatment alone and 48 fold with both IL-20 and IL-1 treatment together.
8. Monocyte chemotactic and activating factor MCAF was up-regulated 1.3 fold with IL-20 treatment alone, 32 fold with IL-1 treatment alone and 56 fold with both IL-20 and IL-1 treatment together.

### Example 5

### IL,20RA/RB receptor-Ig fusion Heterotetramer

The expression vector pEZE3 was used to express the recombinant IL-20 receptor-Ig fusion protein. The plasmid pEZE3 is derived from pDC312. pDC312 was obtained through license from Immunex Corporation. The plasmids pDC312 and pEZE3 contain an EASE segment as described in WO 97/25420. The presence of the EASE segment in an expression vector can improve expression of recombinant proteins two to eight fold in stable cell pools.

The plasmid pEZE3 is a tricistronic expression vector that may be used to express up to three different proteins in mammalian cells, preferably Chinese Hamster Ovary (CHO) cells. The pEZE3 expression unit contains the cytomegalovirus (CMV) enhancer/promoter, the adenovirus tripartite leader sequence, a multiple cloning site for insertion of the coding region for the first recombinant protein, the poliovirus type 2 internal ribosome entry site, a second multiple cloning site for insertion of the coding region for the second recombinant protein, an encephalomyocarditis virus internal ribosome entry site, a coding segment for mouse dihydrofolate reductase, and the SV40 transcription terminator. In addition, pEZE3 contains an E. coli origin of replication and the bacterial beta lactamase gene.

The IL-20 receptor-Ig fusion protein is a disulfide linked heterotetramer consisting of two chains of the extracellular domain of the human IL-20RB fused to the wild type human immunoglobulin kappa light chain constant region and two chains of the human IL-20RA protein extracellular domain fused to a mutated human immunoglobulin gamma 1 constant region. The human immunoglobulin gamma 1 constant region contains amino acid substitutions to reduce FcγRI binding and C1q complement fixation.

The human IL-20RB extracellular domain human immunoglobulin kappa light chain constant region fusion construct was generated by overlap PCR. The IL-20RB coding segment consists of amino acids 1 to 230. The template used for the PCR amplification of the IL-20R segment was generated IL-20RB human kappa light chain constant region expression construct as described below in Example 12. Oligonucleotide primers SEQ ID NO: 24 and SEQ ID NO: 25 were used to amplify the IL-20RB segment. The entire wild type human immunoglobulin kappa light chain constant region was used. The template used for the PCR amplification of the wild type human immunoglobulin kappa light chain constant region segment was generated IL-20RB human kappa light chain constant region expression construct as described in Example 12. Oligonucleotide primers SEQ ID NO: 26 and SEQ ID NO: 27 were used to amplify the wild type human immunoglobulin kappa light chain constant region. The two protein coding domains were linked by overlap PCR using oligonucleotides SEQ ID NO: 24 and SEQ ID NO: 27. A (Gly₄Ser)₃ (SEQ ID NO: 72) peptide linker was inserted between the two protein domains. The (Gly₄Ser)₃ peptide linker was encoded on the PCR primers SEQ ID NO: 26 and SEQ ID NO:25. The resultant IL-20RB extracellular domain/kappa light chain constant region fusion construct is shown by SEQ ID NOs: 20 and 21. The predicted mature polypeptide, minus the signal sequence, is SEQ ID NO: 60. The portion of the extracellular domain of TL-20RB that was actually used was comprised of the amino acid sequence of SEQ ID NO: 61. N-terminal sequencing resulted in the predicted amino acid sequence.

The human IL-20RA extracellular domain human immunoglobulin gamma 1 heavy chain constant region fusion construct was generated by overlap PCR of four separate DNA fragments, each generated by separate PCR amplification reactions. The first fragment contained an optimized tPA (tissue plasminogen activator) signal sequence. The tPA signal sequence was amplified using oligonucleotide primers SEQ ID NO: 28 and SEQ ID NO: 29 using an in-house previously generated expression vector as the template. The second fragment contained the IL-20RA extracellular domain-coding region consisting of amino acids 30 to 243 of SEQ ID NO: 11. Oligonucleotide primers SEQ ID NO: 30 and SEQ ID NO: 31 were used to amplify this IL-20RA segment using a previously generated clone of IL-20RA as the template.

The human gamma 1 heavy chain constant region was generated from 2 segments. The first segment containing the C_{H}1 domain was amplified using oligonucleotide primers SEQ ID NO: 32 and SEQ ID NO: 33 using a clone of the wild type human gamma 1 heavy chain constant region as the template. The second segment containing the remaining hinge, C_{H}2, and C_{H}3 domains of the human immunoglobulin gamma 1 heavy chain constant region was generated by PCR amplification using oligonucleotide primers SEQ ID NO: 34 and SEQ ID NO: 35. The template used for this PCR amplification was from a previously generated human gamma 1 Fc construct that contained codons for amino acid substitutions to reduce FcγRI binding and C1q complement fixation as described in Example 12.

The four protein coding domains were linked by overlap PCR using oligonucleotides SEQ ID NO: 28 and SEQ ID NO: 35. A (Gly₄Ser)₃ peptide linker was inserted between the IL-20RA and CH1 protein domains. The (Gly₄Ser)₃ peptide linker was encoded on the PCR primers SEQ ID NO: 32 and SEQ ID NO: 31. The IL-20RA extracellular domain/ domain human immunoglobulin gamma 1 heavy constant region fusion protein and DNA sequence are shown in SEQ ID NOs: 22 and 23. The predicted mature polypeptide sequence, minus the signal sequence, is SEQ ID NO: 62. The portion of extracellular domain of IL-20RA that was actually used was comprised of SEQ ID NO: 63.

The 1L-20RB extracellular domain human immunoglobulin kappa light chain constant region fusion coding segment was cloned into the second MCS while the human IL-20RA extracellular domain human immunoglobulin gamma 1 heavy chain constant region fusion coding segment was cloned into the first MCS of pEZE3. The plasmid was used to transfect CHO cells. The cells were selected in medium without hypoxanthine or thymidine and the transgene was amplified using methotrexate. The presence of protein was assayed by Western blotting using anti human gamma 1 heavy chain constant region and anti human kappa light chain antibodies. N-terminal sequencing revealed that the optimized tPA leader was not completely cleaved. The observed mass indicated that the first residue of the polypeptide sequence to be pyroglutamic acid, and the N-terminal sequence appears to be pyroEEIHAELRRFRRVPCVSGG (SEQ ID NO: 64), the underlined portion being remnants of the tPA leader.

### Example 6

### IL-20 Transgenic Phenotype

Both human and mouse IL-20 were overexpressed in transgenic mice using a variety of promoters. The liver-specific mouse albumin promoter, directing expression of human IL-20, was used initially in an attempt to achieve circulating levels of protein. Subsequent studies were conducted using the keratin 14 (K14) promoter, which primarily targets expression to the epidermis and other stratified squamous epithelia; the mouse metallothionein-1 promoter, which gives a broad expression pattern; and the EµLCK promoter, which drives expression in cells of the lymphoid lineage. Similar results were obtained in all four cases, possibly because these promoters all give rise to circulating levels of IL-20.

In all cases, transgenic pups expressing the IL-20 transgene were smaller than non-transgenic littermates, had a shiny appearance with tight, wrinkled skin and died within the first few days after birth. Pups had milk in their stomachs indicating that they were able to suckle. These mice had swollen extremities, tail, nostril and mouth regions and had difficulty moving. In addition, the mice were frail, lacked visible adipose tissue and had delayed ear and toe development. Low expression levels in liver (less than 100 mRNA molecules/cell) were sufficient for both the neonatal lethality and skin abnormalities. Transgenic mice without a visible phenotype either did not express the transgene, did not express it at detectable levels, or were mosaic.

Histologic analysis of the skin of the IL-20 transgenic mice showed a thickened epidermis, hyperkeratosis and a compact stratum corneum compared to non-transgenic littermates. Serocellular crusts (scabs) were observed occasionally. Electron microscopic (EM) analysis of skin from transgenic mice showed intramitochondrial lipoid inclusions, mottled keratohyaline granules, and relatively few tonofilaments similar to that observed in human psoriatic skin and in mouse skin disease models. In addition, many of the transgenic mice had apoptotic thymic lymphocytes. No other abnormalities were detected by histopathological analysis. These histological and EM results support and extend the observed gross skin alterations.

### Example 7

### Specificity and Affinity of IL-20 for Its Receptor

The specificity and affinity of IL-20 for its receptor was determined using BHK cells stably transfected with IL-20RA, IL-20RB or both receptor subunits. Binding assays using radiolabeled ligand demonstrated that IL-20 bound to BHK transfectants expressing both IL-20RA and IL-20RB but not to untransfected cells nor to transfectants expressing either receptor subunit alone. Binding of ¹²⁵I-labeled IL-20 was eliminated in the presence of 100-fold excess of unlabeled IL-20 but not with 100-fold excess of the unrelated cytokine, IL-21. The binding affinity (k_{D}) of IL-20 to the IL-20RA/IL-20RB heterodimeric receptor was determined to be approximately 1.5 nM.

### Example 8

### IL-20 receptor Activation

To determine if IL-20 binding leads to receptor activation, the factor-dependent pre-B cell line BaF3 was co-transfected with IL-20RA and IL-20RB and treated with IL-20 at various concentrations. IL-20 stimulated proliferation in a dose-dependent manner and gave a detectable signal at 1.1 pM, with a half maximal response at 3.4 pM. We note that the IL-20 concentration for the half maximal proliferative response in BaF3 cells is 1000X lower than that for half maximal binding affinity in BHK cells. Possible explanations for this large difference include the use of different cell lines, different receptor expression levels and different assay outputs. IL-20 also stimulated signal transduction in the biologically relevant human keratinocyte cell line HaCaT, which naturally expresses IL-20RA and IL-20RB. Therefore, IL-20 binds and activates the heterodimeric 1L-20RA/IL-20RB receptor at concentrations expected for a cytokine. While the negative controls containing untransfected BaF3

### Example 9

### Expression Analysis of IL-20RA and IL-20RB

RT-PCR analysis was performed on a variety of human tissues to determine the expression pattern of IL-20RA and IL-20RB. Both receptor subunits are most highly expressed in skin and testis. The significant result is that IL-20RA and IL-20RB are both expressed in skin, where they have been shown to mediate the IL-20-induced response. Both IL-20RA and IL-20RB are also both expressed in monocytes, lung, ovary, muscle, testis, adrenal gland, heart, salivary gland and placenta. IL-20RA is also in brain, kidney, liver, colon, small intestine, stomach, thyroid, pancreas, uterus and prostate while IL-20RB is not.

### Example 10

### IL-20RA and IL-20RB mRNA are Up-regulated in Psoriasis

*In situ* hybridization was used to determine whether IL-20 receptor expression is altered in psoriasis. Skin samples from four psoriasis patients and three unaffected patients were assayed with probes specific for the two-receptor subunit mRNAs. All four psoriatic skin samples had high levels of IL-20RA and IL-20RB mRNA in keratinocytes whereas normal skin samples did not have detectable levels of either receptor subunit mRNA. Positive signals in psoriatic skin were also observed in mononuclear immune cells and in endothelial cells in a subset of vessels. Therefore, both IL-20RA and IL-20RB are expressed in keratinocytes, immune cells and endothelial cells, the major cell types thought to interact in psoriasis.

### Example 11

### Cloning of Mouse IL-20RA

A cross-species hybridization probe was generated which contained the full-length cDNA fragment encoding human IL-20RA. A Southern blot of mouse genomic DNA and Northern blots of mouse RNA were performed to demonstrate that the human IL-20RA cDNA could specifically hybridize to mouse sequences. The Northern blot results indicated that mouse IL-20RA RNA was present in mouse embryo day 15 and 17 as well as heart, brain, lung, liver, kidney, testes, spleen, thymus, liver, stomach, and small intestine.

The human IL-20RA full length DNA hybridization probe was used to screen a mouse genomic library. The library, which was obtained from Clontech (Palo Alto, CA), was generated from an MboI partial digest of mouse genomic DNA and cloned into the BamHI site of Lambda bacteriophage EMBL3 SP6/T7. Positive bacteriophage was plaque purified and bacteriophage DNA was prepared using Promega's Wizard Lambda Preps DNA Purification System. Two genomic restriction enzyme fragments, a 5.7 kb EcoRI fragment and an 8.0 kb SacI fragment, were generated from the positive bacteriophage and subcloned into pBluescript. DNA sequence analysis revealed the presence of 3 exons from the mouse ortholog to human IL-20RA.

PCR primers from the 5' UTR, SEQ ID NO: 40, and 3' UTR, SEQ ID NO: 41, were designed to generate a full-length mouse IL-20RA sequence by PCR amplification. Mouse embryo 15day plus 17 day cDNA was used as the template for the PCR amplification. PCR products were subcloned and sequenced for confirmation. The mouse sequences are SEQ ID NOs: 36 and 37. The mature extracellular domain is comprised of SEQ ID NO: 38.

### Example 12

### Construction of an IL-20 Receptor Heterotetramer

A vector expressing a secreted hIL-20RA/hIL-20B heterodimer was constructed. In this construct, the extracellular domain of hIL-20RA was fused to the heavy chain of IgG gamma 1 (IgGγ1), while the extracellular portion of IL-20RB was fused to human kappa light chain (human κ light chain).

### Construction of IgG gamma 1 and human κ light fusion vectors

The heavy chain of IgGγ1 was cloned into the Zem229R mammalian expression vector (ATCC deposit No. 69447) such that any extracellular portion of a receptor having a 5' EcoRI and 3' NheI site can be cloned in, resulting in an N-terminal extracellular domain-C-terminal IgGγ1 fusion. The IgGγ1 fragment used in this construct was made by using PCR to isolate the IgGγ1 sequence from a Clontech human fetal liver cDNA library as template. A PCR reaction using oligos SEQ ID NO: 42 and SEQ ID NO: 43 was run as follows: 40 cycles of 94° for 60 sec., 53°C for 60 sec., and 72° for 120 sec.; and 72°C for 7 minutes. PCR products were separated by agarose gel electrophoresis and purified using a QiaQuick^{™} (Qiagen Inc., Valencia, CA) gel extraction kit. The isolated, 990 bp, DNA fragment was digested with MluI and EcoRI (Boerhinger-Mannheim), extracted with QiaQuick^{™} gel extraction kit and ligated with oligos SEQ ID NO: 44 and SEQ ID NO: 45, which comprise an MluI/EcoRI linker, into Zem229R previously digested with Mlul and EcoRI using standard molecular biology techniques disclosed herein. This generic cloning vector was called Vector#76 hIgGgamma1 w/Ch1 #786 Zem229R (Vector #76). The polynucleotide sequence of the extracellular domain of hIL-20RA fused to the heavy chain of IgG gamma 1 is show in SEQ ID NO: 52 and the corresponding polypeptide sequence shown in SEQ ID NO: 53, the mature polypeptide, minus the signal sequence being comprised of SEQ ID NO: 54. The portion of the extracellular domain of IL-20RA used was comprised of SEQ ID NO: 55.

The human κ light chain was cloned in the Zem228R mammalian expression vector (ATCC deposit No. 69446) such that any extracellular portion of a receptor having a 5' EcoRI site and a 3' KpnI site can be cloned in, resulting in an N-terminal extracellular domain-C-terminal human κ light chain fusion. The human κ light chain fragment used in this construct was made by using PCR to isolate the human κ light chain sequence from the same Clontech hFetal Liver cDNA library used above. A PCR reaction was run using oligos SEQ ID NO: 46 and SEQ ID NO: 47. PCR products were separated by agarose gel electrophoresis and purified using a QiaQuick^{™} (Qiagen) gel extraction kit. The isolated, 315 bp, DNA fragment was digested with MluI and EcoRI (Boerhinger-Mannheim), extracted with QiaQuick^{™} gel extraction kit and ligated with the MluI/EcoRI linker described above, into Zem228R previously digested with MluI and EcoRI using standard molecular biology techniques disclosed herein. This generic cloning vector was called Vector #77 hκlight #774 Zem228R (Vector #77). The polynucleotide sequence of the extracellular portion of IL-20RB fused to human kappa light chain is shown in SEQ ID NO: 56 and the corresponding polypeptide sequence shown in SEQ ID NO: 57, the mature polypeptide, minus the signal sequence, is comprised of SEQ ID NO: 58. The portion of the extracellular domain of IL-20RB actually used was comprised of SEQ ID NO: 59.

### Insertion of hIL-20RA and IL-20RB extracellular domains into fusion vector constructs

Using the construction vectors above, a construct having human IL-20RA fused to IgGγ1 was made. This construction was done by using PCR to obtain human IL-20RA receptor from hIL-20RA /IgG Vector #102 with oligos SEQ ID NO: 48 and SEQ ID NO: 49 under conditions described as follows: 30 cycles of 94°C for 60 sec., 57°C for 60 sec., and 72°C for 120 sec.; and 72°C for 7 min. The resulting PCR product was digested with EcoRI and NheI, gel purified, as described herein, and ligated into a previously EcoRI and NheI digested and band-purified Vector #76 (above). The resulting vector was sequenced to confirm that the human IL-20Rα /IgG gamma 1 fusion (hIL-20RA /Ch1 IgG) was correct. The hIL-20RA /Ch1 IgG gamma 1 #1825 Zem229R vector was called vector #195. The IL-20RA/Ch1 IgGγ1 sequence thus obtained is depicted by SEQ ID NOs: 52 and 53. N-terminal sequencing indicated the presence of the predicted mature polypeptide sequence of SEQ ID NO: 54.

A separate construct having IL-20RB fused to κ light was also constructed. The IL-20RB/human κ light chain construction was performed as above by PCRing from DR1/7N-4 with oligos SEQ ID NO: 50 and SEQ ID NO: 51, digesting the resulting band with EcoRI and KpnI and then ligating this product into a previously EcoRI and KpnI digested and band-purified Vec#77 (above). The resulting vector was sequenced to confirm that the IL-20RB/ human κ light chain fusion (IL-20RB/κlight) was correct. This IL-20RB//κlight construct is shown by SEQ ID NOs: 56 and 57. N-terminal sequencing of the resultant polypeptide indicated the presence of the predicted mature amino acid sequence comprised of SEQ ID NO: 58. SEQ ID NO:59 is the mature portion of the extracellular domain of IL-20RB used.

### Co-expression of the human IL-20RA and human IL-20RB receptors

Approximately 16µg of each of vectors #194 and #195, above, were co-transfected into BHK-570 cells (ATCC No. CRL-10314) using Lipofectamine^{™} reagent (Gibco/BRL), as per manufacturer's instructions. The transfected cells were selected for 10 days in DMEM + 5%FBS (Gibco/BRL) containing 1µM of methotrexate (MTX) (Sigma, St. Louis, MO) and 0.5mg/ml G418 (Gibco/BRL) for 10 days. The resulting pool of transfectants was selected again in 10µM MTX and 0.5mg/ml G418 for 10 days.

The resulting pool of doubly selected cells was used to generate protein. Three factories (Nunc, Denmark) of this pool were used to generate 8 L of serum free conditioned medium. This conditioned media was passed over a 1 ml protein-A column and eluted in (10) 750 microliter fractions. 4 of these fractions found to have the highest concentration were pooled and dialyzed (10 kD MW cutoff) against PBS. Finally, the dialyzed material was analyzed by BCA (Pierce) and found to have a concentration of 317 µg/ml. A total of 951 µg was obtained from this 8 L purification.

### Example 13

### IL-20 Binding Activates STAT3 in the HaCaT Keratinocyte Cell Line

IL-20 binds cell lines transfected with both subunits of its receptor. However, these cell lines overexpress the IL-20 receptor relative to its normal level and their relevance to the physiological role of IL-20 is unclear. The human HaCaT keratinocyte cell line, which expresses endogenous IL-20RA and IL-20RB was used to examine IL-20 signal transduction in a biologically relevant cell type. HaCaT cells were infected with recombinant adenovirus containing a reporter construct to allow detection of intracellular signaling. The construct consists of the firefly luciferase gene driven by promoter/enhancer sequences comprised of the serum response element (SRE) and signal transducers and activators of transduction elements (STATs). This assay system detects productive ligand-receptor interactions and indicates possible downstream signal transduction components involved in receptor activation. Treatment with IL-20 alone resulted in a dose-dependent increase in luciferase activity with a half maximal response occurring at approximately 2.3 nM. Subsequent luciferase reporter assays using adenovirus vectors containing only the SRE element or only the STAT elements produced detectable reporter activation only through STATs.

To determine if other cytokines act in concert with IL-20, HaCaT cells were treated with IL-20 alone or in combination with a single submaximal dose of EGF, IL-1β, or TNFα. In the presence of each of these three proteins, IL-20 treatment resulted in a dose-dependent increase in luciferase activity. IL-20 in combination with IL-1β results in a half-maximal response at approximately 0.5 nM, about five-fold lower than with IL-20 alone. In addition, activation of the reporter gene is detectable at 0.1 nM IL-20, a dose that is at least tenfold lower than the IL-20 dose required alone.

BHK cells transfected with IL-20RA, IL-20RB or both receptor subunits were used to determine whether receptor pairing was required for IL-20 stimulation of STAT-luciferase. As was the case with binding assays, only cells transfected with both receptor subunits responded to IL-20 and did so with a half-maximal response of 5.7 pM. We note that the IL-20 concentration for the half-maximal response in BHK cells is 400-fold lower than that for half-maximal response in HaCaT cells. It is likely that a lower concentration of IL-20 is needed for half-maximal response in BHK cells, as compared to HaCaT cells, due to higher receptor levels in the BHK IL-20 receptor transfectants.

A nuclear translocation assay was used to identify STAT proteins involved in IL-20 action. Both HaCaT cells, with endogenous IL-20 receptors, and BHK cells transfected with IL-20RA and IL-20RB, were treated with IL-20 protein and translocation of STAT3 and STAT1 transcription factors from the cytoplasm to the nucleus was assayed by immunofluorescence.

In unstimulated HaCaT cells, STAT3 staining was predominantly in the cytosol. Treatment of HaCaT cells with IL-20 resulted in a distinct accumulation of STAT3 in the nucleus. Nuclear translocation of STAT3 in response to increasing concentrations of IL-20 occurred with a half-maximal IL-20 concentration of 7 nM. In contrast to STAT3 translocation, HaCaT cells treated with IL-20 did not show any detectable nuclear accumulation of STAT1.

BHK cells transfected with IL-20RA and IL-20RB were used to confirm that the IL-20 receptor was required for IL-20 stimulation of STAT3 nuclear translocation. In BHK cells lacking the IL-20 receptor, STAT3 remained cytosolic following treatment with IL-20. In contrast, in BHK cells transfected with the IL-20 receptor, STAT3 translocated to the nucleus in response to IL-20. Again, STAT1 remained cytosolic regardless of IL-20 treatment or IL-20 receptor expression. We conclude that the IL-20 receptor is required for IL-20-mediated STAT3 activation.

## Claims

1. Use of a soluble receptor that binds to an IL-20 polypeptide, wherein the IL-20 polypeptide comprises an amino acid sequence selected from the group consisting of SEQ ID NOs: 1, 2, 3, 4, 5, 6, 7, 8 and 9, for the manufacture of a medicament for treating an inflammatory disease, wherein said soluble receptor comprises: a first subunit, said first subunit comprising an amino acid sequence selected from SEQ ID NOs: 11, 12, 55, 63 and 65, or a fragment thereof that binds to an IL-20 polypeptide; and a second subunit, said second subunit comprising an amino acid sequence selected from SEQ ID NOs: 14, 15, 19, 59, 61, 67, 68 and 69, or a fragment thereof that binds to an IL-20 polypeptide;
wherein said inflammatory disease is a skin disease.

2. Use according to Claim 1, wherein the first subunit is fused to a constant region heavy chain of an immunoglobulin (Ig) molecule, and the second subunit is fused to a constant region of a light chain of an Ig molecule.

3. Use according to Claim 1 wherein the skin disease is selected from the group consisting of melanoma, psoriasis, eczema, atopic dermatitis and contact dermatitis.

## Patentansprüche

1. Verwendung eines löslichen Rezeptors, der an ein IL-20-Polypeptid bindet, wobei das IL-20-Polypeptid eine Aminosäuresequenz umfaßt, die ausgewählt ist aus der Gruppe, bestehend aus SEQ ID NOs: 1, 2, 3, 4, 5, 6, 7, 8 und 9 zur Herstellung eines Medikaments zur Behandlung einer entzündlichen Erkrankung, wobei der lösliche Rezeptor umfaßt: eine erste Untereinheit, wobei diese erste Untereinheit eine Aminosäuresequenz, ausgewählt aus den SEQ ID NOs: 11, 12, 55, 63 und 65 oder ein Fragment davon, umfaßt, das an ein IL-20-Polypeptid bindet; und eine zweite Untereinheit, wobei diese zweite Untereinheit eine Aminosäuresequenz, ausgewählt aus den SEQ ID NOs: 14, 15, 19, 59, 61, 67, 68 und 69 oder ein Fragment davon, umfaßt, das an ein IL-20-Polypeptid bindet;
wobei die entzündliche Erkrankung eine Hauterkrankung ist.

2. Verwendung nach Anspruch 1, wobei die erste Untereinheit an eine konstante Region der schweren Kette eines Immunoglobulin-(Ig)-Moleküls fusioniert ist und die zweite Untereinheit an eine konstante Region einer leichten Kette eines Ig-Moleküls fusioniert ist.

3. Verwendung nach Anspruch 1, wobei die Hauterkrankung ausgewählt ist aus der Gruppe, bestehend aus Melanom, Psoriasis, Ekzem, atopischer Dermatitis und Kontaktdermatitis.

## Revendications

1. Utilisation d'un récepteur soluble qui se lie à un polypeptide de l'IL-20, dans laquelle le polypeptide de l'IL-20 comprend une séquence d'acides aminés choisie dans le groupe formé par les SEQ ID N° : 1, 2, 3, 4, 5, 6, 7, 8 et 9, pour la fabrication d'un médicament destiné au traitement d'une maladie inflammatoire, où ledit récepteur soluble comprend : une première sous-unité, ladite première sous-unité comprenant une séquence d'acides aminés choisie parmi les SEQ ID N° : 11, 12, 55, 63 et 65, ou un fragment de celles-ci qui se lie à un polypeptide de l'IL-20 ; et une seconde sous-unité, ladite seconde sous-unité comprenant une séquence d'acides aminés choisie parmi les SEQ ID N° : 14, 15, 19, 59, 61, 67, 68 et 69, ou un fragment de celles-ci qui se lie à un polypeptide de l'IL-20 ;
dans laquelle ladite maladie inflammatoire est une maladie de la peau.

2. Utilisation selon la revendication 1, dans laquelle la première sous-unité est fusionnée à une région constante d'une chaîne lourde d'une molécule d'immunoglobuline (Ig), et la seconde sous-unité est fusionnée à une région constante d'une chaîne légère d'une molécule d'Ig.

3. Utilisation selon la revendication 1, dans laquelle la maladie de la peau est choisie dans le groupe formé par un mélanome, un psoriasis, un eczéma, une dermatite atopique et une dermatite de contact.


### SEQUENCE LISTING

<110> ZymoGenetics, Inc.
<120> Method for Treating Inflammation
<130> 99-108PC
<150> 09/470,898
   <151> 1999-12-23
<150> 60/213,341
   <151> 2000-06-22
<160> 72
<170> FastSEQ for Windows Version 3.0
<210> 1
   <211> 176
   <212> PRT
   <213> Homo sapiens
<400> 1
<210> 2
   <211> 152
   <212> PRT
   <213> Homo sapiens
<400> 2
   Leu Lys Thr Leu Asn Leu Gly Ser Cys Val Ile Ala Thr Asn Leu Gln
   1 5 10 15
   Glu Ile Arg Asn Gly Phe Ser Asp Ile Arg Gly Ser Val Gln Ala Lys
   20 25 30
   Asp Gly Asn Ile Asp Ile Arg Ile Leu Arg Arg Thr Glu Ser Leu Gln
   35 40 45
   Asp Thr Lys Pro Ala Asn Arg Cys Cys Leu Leu Arg His Leu Leu Arg
   50 55 60
   Leu Tyr Leu Asp Arg Val Phe Lys Asn Tyr Gln Thr Pro Asp His Tyr
   65 70 75 80
   Thr Leu Arg Lys Ile Ser Ser Leu Ala Asn Ser Phe Leu Thr Ile Lys
   85 90 95
   Lys Asp Leu Arg Leu Cys His Ala His Met Thr Cys His Cys Gly Glu
   100 105 110
   Glu Ala Met Lys Lys Tyr Ser Gln Ile Leu Ser His Phe Glu Lys Leu
   115 120 125
   Glu Pro Gln Ala Ala Val Val Lys Ala Leu Gly Glu Leu Asp Ile Leu
   130 135 140
   Leu Gln Trp Met Glu Glu Thr Glu
   145 150
<210> 3
   <211> 151
   <212> PRT
   <213> Homo sapiens
<400> 3
   Met Lys Ala Ser Ser Leu Ala Phe Ser Leu Leu Ser Ala Ala Phe Tyr
   1               5                   10                  15
   Leu Leu Trp Thr Pro Ser Thr Gly Leu Lys Thr Leu Asn Leu Gly Ser
               20                  25                  30
   Cys Val Ile Ala Thr Asn Leu Gln Glu Ile Arg Asn Gly Phe Ser Asp
           35                  40                  45
   Ile Arg Gly Ser Val Gln Ala Lys Asp Gly Asn Ile Asp Ile Arg Ile
       50                  55                  60
   Leu Arg Arg Thr Glu Ser Leu Gln Asp Thr Lys Pro Ala Asn Arg Cys
   65                  70                  75                  80
   Cys Leu Leu Arg His Leu Leu Arg Leu Tyr Leu Asp Arg Val Phe Lys
                   85                  90                  95
   Asn Tyr Gln Thr Pro Asp His Tyr Thr Leu Arg Lys Ile Ser Ser Leu
               100                 105                 110
   Ala Asn Ser Phe Leu Thr Ile Lys Lys Asp Leu Arg Leu Cys Leu Glu
           115                 120                 125
   Pro Gln Ala Ala Val Val Lys Ala Leu Gly Glu Leu Asp Ile Leu Leu
       130                 135                 140
   Gln Trp Met Glu Glu Thr Glu
   145                 150
<210> 4
   <211> 127
   <212> PRT
   <213> Homo sapiens
<400> 4
   Leu Lys Thr Leu Asn Leu Gly Ser Cys Val Ile Ala Thr Asn Leu Gln
   1               5                   10                  15
   Glu Ile Arg Asn Gly Phe Ser Asp Ile Arg Gly Ser Val Gln Ala Lys
               20                  25                  30
   Asp Gly Asn Ile Asp Ile Arg Ile Leu Arg Arg Thr Glu Ser Leu Gln
           35                  40                  45
   Asp Thr Lys Pro Ala Asn Arg Cys Cys Leu Leu Arg His Leu Leu Arg
       50                  55                  60
   Leu Tyr Leu Asp Arg Val Phe Lys Asn Tyr Gln Thr Pro Asp His Tyr
   65                  70                  75                  80
   Thr Leu Arg Lys Ile Ser Ser Leu Ala Asn Ser Phe Leu Thr Ile Lys
                   85                  90                  95
   Lys Asp Leu Arg Leu Cys Leu Glu Pro Gln Ala Ala Val Val Lys Ala
               100                 105                 110
   Leu Gly Glu Leu Asp Ile Leu Leu Gln Trp Met Glu Glu Thr Glu
           115                 120                 125
<210> 5
   <211> 176
   <212> PRT
   <213> Mus musculis
<400> 5
   Met Lys Gly Phe Gly Leu Ala Phe Gly Leu Phe Ser Ala Val Gly Phe
   1 5 10 15
   Leu Leu Trp Thr Pro Leu Thr Gly Leu Lys Thr Leu His Leu Gly Ser
   20 25 30
   Cys Val Ile Thr Ala Asn Leu Gln Ala Ile Gln Lys Glu Phe Ser Glu
   35 40 45
   Ile Arg Asp Ser Val Gln Ala Glu Asp Thr Asn Ile Asp Ile Arg Ile
   50 55 60
   Leu Arg Thr Thr Glu Ser Leu Lys Asp Ile Lys Ser Leu Asp Arg Cys
   65 70 75 80
   Cys Phe Leu Arg His Leu Val Arg Phe Tyr Leu Asp Arg Val Phe Lys
   85 90 95
   Val Tyr Gln Thr Pro Asp His His Thr Leu Arg Lys Ile Ser Ser Leu
   100 105 110
   Ala Asn Ser Phe Leu Ile Ile Lys Lys Asp Leu Ser Val Cys His Ser
   115 120 125
   His Met Ala Cys His Cys Gly Glu Glu Ala Met Glu Lys Tyr Asn Gln
   130 135 140
   Ile Leu Ser His Phe Ile Glu Leu Glu Leu Gln Ala Ala Val Val Lys
   145 150 155 160
   Ala Leu Gly Glu Leu Gly Ile Leu Leu Arg Trp Met Glu Glu Met Leu
   165 170 175
<210> 6
   <211> 152
   <212> PRT
   <213> Mus musculis
<400> 6
   Leu Lys Thr Leu His Leu Gly Ser Cys Val Ile Thr Ala Asn Leu Gln
   1 5 10 15
   Ala Ile Gln Lys Glu Phe Ser Glu Ile Arg Asp Ser Val Gln Ala Glu
   20 25 30
   Asp Thr Asn Ile Asp Ile Arg Ile Leu Arg Thr Thr Glu Ser Leu Lys
   35 40 45
   Asp Ile Lys Ser Leu Asp Arg Cys Cys Phe Leu Arg His Leu Val Arg
   50 55 60
   Phe Tyr Leu Asp Arg Val Phe Lys Val Tyr Gln Thr Pro Asp His His
   65 70 75 80
   Thr Leu Arg Lys Ile Ser Ser Leu Ala Asn Ser Phe Leu Ile Ile Lys
   85 90 95
   Lys Asp Leu Ser Val Cys His Ser His Met Ala Cys His Cys Gly Glu
   100 105 110
   Glu Ala Met Glu Lys Tyr Asn Gln Ile Leu Ser His Phe Ile Glu Leu
   115 120 125
   Glu Leu Gln Ala Ala Val Val Lys Ala Leu Gly Glu Leu Gly Ile Leu
   130 135 140
   Leu Arg Trp Met Glu Glu Met Leu
   145 150
<210> 7
   <211> 144
   <212> PRT
   <213> Mus musculis
<400> 7
   Cys Val Ile Thr Ala Asn Leu Gln Ala Ile Gln Lys Glu Phe Ser Glu
   1 5 10 15
   Ile Arg Asp Ser Val Gln Ala Glu Asp Thr Asn Ile Asp Ile Arg Ile
   20 25 30
   Leu Arg Thr Thr Glu Ser Leu Lys Asp Ile Lys Ser Leu Asp Arg Cys
   35 40 45
   Cys Phe Leu Arg His Leu Val Arg Phe Tyr Leu Asp Arg Val Phe Lys
   50 55 60
   Val Tyr Gln Thr Pro Asp His His Thr Leu Arg Lys Ile Ser Ser Leu
   65 70 75 80
   Ala Asn Ser Phe Leu Ile Ile Lys Lys Asp Leu Ser Val Cys His Ser
   85 90 95
   His Met Ala Cys His Cys Gly Glu Glu Ala Met Glu Lys Tyr Asn Gln
   100 105 110
   Ile Leu Ser His Phe Ile Glu Leu Glu Leu Gln Ala Ala Val Val Lys
   115 120 125
   Ala Leu Gly Glu Leu Gly Ile Leu Leu Arg Trp Met Glu Glu Met Leu
   130 135 140
<210> 8
   <211> 154
   <212> PRT
   <213> Mus musculis
<400> 8
   Met Lys Gly Phe Gly Leu Ala Phe Gly Leu Phe Ser Ala Val Gly Phe
    1               5                  10                  15
   Leu Leu Trp Thr Pro Leu Thr Gly Leu Lys Thr Leu His Leu Gly Ser
               20                  25                  30
   Cys Val Ile Thr Ala Asn Leu Gln Ala Ile Gln Lys Glu Phe Ser Glu
           35                  40                  45
   Ile Arg Asp Ser Val Ser Leu Asp Arg Cys Cys Phe Leu Arg His Leu
       50                  55                  60
   Val Arg Phe Tyr Leu Asp Arg Val Phe Lys Val Tyr Gln Thr Pro Asp
   65                  70                  75                  80
   His His Thr Leu Arg Lys Ile Ser Ser Leu Ala Asn Ser Phe Leu Ile
                   85                  90                  95
   Ile Lys Lys Asp Leu Ser Val Cys His Ser His Met Ala Cys His Cys
               100                 105                 110
   Gly Glu Glu Ala Met Glu Lys Tyr Asn Gln Ile Leu Ser His Phe Ile
           115                 120                 125
   Glu Leu Glu Leu Gln Ala Ala Val Val Lys Ala Leu Gly Glu Leu Gly
       130                 135                 140
   Ile Leu Leu Arg Trp Met Glu Glu Met Leu
   145                 150
<210> 9
   <211> 130
   <212> PRT
   <213> Homo sapiens
<400> 9
   Leu Lys Thr Leu His Leu Gly Ser Cys Val Ile Thr Ala Asn Leu Gln
    1               5                  10                  15
   Ala Ile Gln Lys Glu Phe Ser Glu Ile Arg Asp Ser Val Ser Leu Asp
               20                  25                  30
   Arg Cys Cys Phe Leu Arg His Leu Val Arg Phe Tyr Leu Asp Arg Val
           35                  40                  45
   Phe Lys Val Tyr Gln Thr Pro Asp His His Thr Leu Arg Lys Ile Ser
       50                  55                  60
   Ser Leu Ala Asn Ser Phe Leu Ile Ile Lys Lys Asp Leu Ser Val Cys
   65                  70                  75                  80
   His Ser His Met Ala Cys His Cys Gly Glu Glu Ala Met Glu Lys Tyr
                   85                  90                  95
   Asn Gln Ile Leu Ser His Phe Ile Glu Leu Glu Leu Gln Ala Ala Val
               100                 105                 110
   Val Lys Ala Leu Gly Glu Leu Gly Ile Leu Leu Arg Trp Met Glu Glu
           115                 120                 125
   Met Leu
       130
<210> 10
   <211> 3516
   <212> DNA
   <213> Homo sapiens
<220>
   <221> CDS
   <222> (237)...(1895)
<400> 10
   tccagctggg tagccggggg agcgcgcgtg ggggctccgc gagtcgctcg cccttggttt    60
   ctggggaagc ctgggggacg cggctgtggc ggaggcgccc tgggactcag gtcgcctgga   120
   gcgtggcacg cagagcccca ggcgcggagc tgaggccgcg cggccgcgct tggccccagc   180
   gggcgtggga ctgagcagtc tgctgccccc cgacatgtga cccagccccg ccgccc atg   239
   cgg gct ccc ggc cgc ccg gcc ctg cgg ccg ctg ccg ctg ccg ccg ctg     287
   ctg ctg ttg ctc ctg gcg gcg cct tgg gga cgg gca gtt ccc tgt gtc     335
   tct ggt ggt ttg cct aaa cct gca aac atc acc ttc tta tcc atc aac     383
   atg aag aat gtc cta caa tgg act cca cca gag ggt ctt caa gga gtt     431
   aaa gtt act tac act gtg cag tat ttc ata tat ggg caa aag aaa tgg     479
   ctg aat aaa tca gaa tgc aga aat atc aat aga acc tac tgt gat ctt     527
   tct gct gaa act tct gac tac gaa cac cag tat tat gcc aaa gtt aag      575
   gcc att tgg gga aca aag tgt tcc aaa tgg gct gaa agt gga cgg ttc      623
   tat cct ttt tta gaa aca caa att ggc cca cca gag gtg gca ctg act      671
   aca gat gag aag tcc att tct gtt gtc ctg aca gct cca gag aag tgg      719
   aag aga aat cca gaa gac ctt cct gtt tcc atg caa caa ata tac tcc      767
   aat ctg aag tat aac gtg tct gtg ttg aat act aaa tca aac aga acg      815
   tgg tcc cag tgt gtg acc aac cac acg ctg gtg ctc acc tgg ctg gag      863
   ccg aac act ctt tac tgc gta cac gtg gag tcc ttc gtc cca ggg ccc      911
   cct cgc cgt gct cag cct tct gag aag cag tgt gcc agg act ttg aaa      959
   gat caa tca tca gag ttc aag gct aaa atc atc ttc tgg tat gtt ttg      1007
   ccc ata tct att acc gtg ttt ctt ttt tct gtg atg ggc tat tcc atc      1055
   tac cga tat atc cac gtt ggc aaa gag aaa cac cca gca aat ttg att      1103
   ttg att tat gga aat gaa ttt gac aaa aga ttc ttt gtg cct gct gaa      1151
   aaa atc gtg att aac ttt atc acc ctc aat atc tcg gat gat tct aaa      1199
   att tct cat cag gat atg agt tta ctg gga aaa agc agt gat gta tcc      1247
   agc ctt aat gat cct cag ccc agc ggg aac ctg agg ccc cct cag gag      1295
   gaa gag gag gtg aaa cat tta ggg tat gct tcg cat ttg atg gaa att      1343
   ttt tgt gac tct gaa gaa aac acg gaa ggt act tct ttc acc cag caa      1391
   gag tcc ctc agc aga aca ata ccc ccg gat aaa aca gtc att gaa tat      1439
   gaa tat gat gtc aga acc act gac att tgt gcg ggg cct gaa gag cag      1487
   gag ctc agt ttg cag gag gag gtg tcc aca caa gga aca tta ttg gag      1535
   tcg cag gca gcg ttg gca gtc ttg ggc ccg caa acg tta cag tac tca      1583
   tac acc cct cag ctc caa gac tta gac ccc ctg gcg cag gag cac aca      1631
   gac tcg gag gag ggg ccg gag gaa gag cca tcg acg acc ctg gtc gac      1679
   tgg gat ccc caa act ggc agg ctg tgt att cct tcg ctg tcc agc ttc      1727
   gac cag gat tca gag ggc tgc gag cct tct gag ggg gat ggg ctc gga      1775
   gag gag ggt ctt cta tct aga ctc tat gag gag ccg gct cca gac agg      1823
   cca cca gga gaa aat gaa acc tat ctc atg caa ttc atg gag gaa tgg      1871
   ggg tta tat gtg cag atg gaa aac tgatgccaac acttcctttt gccttttgtt     1925
   tcctgtgcaa acaagtgagt cacccctttg atcccagcca taaagtacct gggatgaaag     1985
   aagttttttc cagtttgtca gtgtctgtga gaattactta tttcttttct ctattctcat     2045
   agcacgtgtg tgattggttc atgcatgtag gtctcttaac aatgatggtg ggcctctgga     2105
   gtccaggggc tggccggttg ttctatgcag agaaagcagt caataaatgt ttgccagact     2165
   gggtgcagaa tttattcagg tgggtgtact ctggcctctt ggttcattat tttcaaacaa     2225
   gcacacttgt acaattattt tctgggtact tcccatatgc acatagcact gtaaaaaata     2285
   tttcccaaag atcactcatt ttataaatac cactttttca gaattgggtt tattgcgagc     2345
   aggaggagat acttaaaaca tgcacatata ccaggttggt ggtaagttgg tcacatgtga     2405
   aaacctcaac tatttaatca tcatgattca tattttgagt gaatacatca ggcacagacc     2465
   ttcatgatat cacacactct tggctacttt aagaggccat ctttaatact ttatgagtag     2525
   ttctggagtg taaacataaa cgagtattct tttgtagtca gaaaagtgtc ctctcaataa     2585
   tttagtaggg gcttattgtc tctcaaaact aacctaaaag aaaatgacac attttataat 2645
   agaatattac atttatttct ggaagtgtgt tttcaaaaag atatttacat agtctgtaaa 2705
   ctagaaagtg ttaggtaaag ctctaggtta ctgtgttact attataatat taaacattcg 2765
   aataggcagt cgttcaaaga ctctttggaa tatctatgaa tgaatatcct ctattcttat 2825
   aatattaaaa cccataagta aatataggac atacaagaga aatgagttaa atgactatgt 2885
   aagggagagt ttattaaaat ttgatgaaat ttactgtagg aactaaacta tgccataaaa 2945
   caatagcttt ctagttcatt tccagtaact gttcccatct cctttaccac ttgttaagaa 3005
   aattaaattc ttcagtcacg ctgctttaaa atgggacaaa atctattaag ttgaaccata 3065
   tataattgtg gatatttggc tgtttttaat ctgacaagca gtaacttcat atggtttgcc 3125
   ttaatatata tttgttttag tcatgaactc ataatccatt gatgctcttt catgagaaga 3185
   gatatgaccc atatttcctt attgatatta ttggtacagg cagacaaccc tggtaggaga 3245
   gatggattct ggggtcatga cctttcgtga ttatccgcaa atgcaaacag tttcagatct 3305
   aatggtttaa tttagggagt aattatatta atcagagtgt tctgttattc tcaatcttta 3365
   tagaaacgat tctgctggtt ttgaagaaca gatgtattac actaactgta aaagtagttc 3425
   aagagtgaga aagaataaat tgttattaag agcaaaagaa aaataaagtg attgatgata 3485
   aaaaaaaaaa aaaaaaagcg gccgcctcga g 3516
<210> 11
   <211> 553
   <212> PRT
   <213> Homo sapiens
<400> 11
<210> 12
   <211> 221
   <212> PRT
   <213> Homo sapiens
<400> 12
   Val Pro Cys Val Ser Gly Gly Leu Pro Lys Pro Ala Asn Ile Thr Phe
    1               5                  10                  15
   Leu Ser Ile Asn Met Lys Asn Val Leu Gln Trp Thr Pro Pro Glu Gly
               20                  25                  30
   Leu Gln Gly Val Lys Val Thr Tyr Thr Val Gln Tyr Phe Ile Tyr Gly
           35                  40                  45
   Gln Lys Lys Trp Leu Asn Lys Ser Glu Cys Arg Asn Ile Asn Arg Thr
       50                  55                  60
   Tyr Cys Asp Leu Ser Ala Glu Thr Ser Asp Tyr Glu His Gln Tyr Tyr
   65                  70                  75                  80
   Ala Lys Val Lys Ala Ile Trp Gly Thr Lys Cys Ser Lys Trp Ala Glu
                   85                  90                  95
   Ser Gly Arg Phe Tyr Pro Phe Leu Glu Thr Gln Ile Gly Pro Pro Glu
               100                 105                 110
   Val Ala Leu Thr Thr Asp Glu Lys Ser Ile Ser Val Val Leu Thr Ala
           115                 120                 125
   Pro Glu Lys Trp Lys Arg Asn Pro Glu Asp Leu Pro Val Ser Met Gln
       130                 135                 140
   Gln Ile Tyr Ser Asn Leu Lys Tyr Asn Val Ser Val Leu Asn Thr Lys
   145                 150                 155                 160
   Ser Asn Arg Thr Trp Ser Gln Cys Val Thr Asn His Thr Leu Val Leu
                   165                 170                 175
   Thr Trp Leu Glu Pro Asn Thr Leu Tyr Cys Val His Val Glu Ser Phe
               180                 185                 190
   Val Pro Gly Pro Pro Arg Arg Ala Gln Pro Ser Glu Lys Gln Cys Ala
           195                 200                 205
   Arg Thr Leu Lys Asp Gln Ser Ser Glu Phe Lys Ala Lys
       210                 215                 220
<210> 13
   <211> 971
   <212> DNA
   <213> Homo sapiens
<220>
   <221> CDS
   <222> (18)...(950)
<400> 13
   gaattcgagt ctaccaa atg cag act ttc aca atg gtt cta gaa gaa atc     50
                      Met Gln Thr Phe Thr Met Val Leu Glu Glu Ile
                       1               5                   10
   tgg aca agt ctt ttc atg tgg ttt ttc tac gca ttg att cca tgt ttg    98
   Trp Thr Ser Leu Phe Met Trp Phe Phe Tyr Ala Leu Ile Pro Cys Leu
               15                  20                  25
   ctc aca gat gaa gtg gcc att ctg cct gcc cct cag aac ctc tct gta   146
   Leu Thr Asp Glu Val Ala Ile Leu Pro Ala Pro Gln Asn Leu Ser Val
           30                  35                  40
   ctc tca acc aac atg aag cat ctc ttg atg tgg agc cca gtg atc gcg   194
   Leu Ser Thr Asn Met Lys His Leu Leu Met Trp Ser Pro Val Ile Ala
       45                  50                  55
   cct gga gaa aca gtg tac tat tct gtc gaa tac cag ggg gag tac gag   242
   Pro Gly Glu Thr Val Tyr Tyr Ser Val Glu Tyr Gln Gly Glu Tyr Glu
   60                  65                  70                  75
   agc ctg tac acg agc cac atc tgg atc ccc agc agc tgg tgc tca ctc   290
   Ser Leu Tyr Thr Ser His Ile Trp Ile Pro Ser Ser Trp Cys Ser Leu
                   80                  85                  90
   act gaa ggt cct gag tgt gat gtc act gat gac atc acg gcc act gtg   338
   Thr Glu Gly Pro Glu Cys Asp Val Thr Asp Asp Ile Thr Ala Thr Val
               95                  100                 105
   cca tac aac ctt cgt gtc agg gcc aca ttg ggc tca cag acc tca gcc   386
   Pro Tyr Asn Leu Arg Val Arg Ala Thr Leu Gly Ser Gln Thr Ser Ala
           110                 115                 120
   tgg agc atc ctg aag cat ccc ttt aat aga aac tca acc atc ctt acc      434
   Trp Ser Ile Leu Lys His Pro Phe Asn Arg Asn Ser Thr Ile Leu Thr
       125                 130                 135
   cga cct ggg atg gag atc acc aaa gat ggc ttc cac ctg gtt att gag      482
   Arg Pro Gly Met Glu Ile Thr Lys Asp Gly Phe His Leu Val Ile Glu
   140                 145                 150                 155
   ctg gag gac ctg ggg ccc cag ttt gag ttc ctt gtg gcc tac tgg agg      530
   Leu Glu Asp Leu Gly Pro Gln Phe Glu Phe Leu Val Ala Tyr Trp Arg
                   160                 165                 170
   agg gag cct ggt gcc gag gaa cat gtc aaa atg gtg agg agt ggg ggt      578
   Arg Glu Pro Gly Ala Glu Glu His Val Lys Met Val Arg Ser Gly Gly
               175                 180                 185
   att cca gtg cac cta gaa acc atg gag cca ggg gct gca tac tgt gtg      626
   Ile Pro Val His Leu Glu Thr Met Glu Pro Gly Ala Ala Tyr Cys Val
           190                 195                 200
   aag gcc cag aca ttc gtg aag gcc att ggg agg tac agc gcc ttc agc      674
   Lys Ala Gln Thr Phe Val Lys Ala Ile Gly Arg Tyr Ser Ala Phe Ser
       205                 210                 215
   cag aca gaa tgt gtg gag gtg caa gga gag gcc att ccc ctg gta ctg      722
   Gln Thr Glu Cys Val Glu Val Gln Gly Glu Ala Ile Pro Leu Val Leu
   220                 225                 230                 235
   gcc ctg ttt gcc ttt gtt ggc ttc atg ctg atc ctt gtg gtc gtg cca      770
   Ala Leu Phe Ala Phe Val Gly Phe Met Leu Ile Leu Val Val Val Pro
                   240                 245                 250
   ctg ttc gtc tgg aaa atg ggc cgg ctg ctc cag tac tcc tgt tgc ccc      818
   Leu Phe Val Trp Lys Met Gly Arg Leu Leu Gln Tyr Ser Cys Cys Pro
               255                 260                 265
   gtg gtg gtc ctc cca gac acc ttg aaa ata acc aat tca ccc cag aag      866
   Val Val Val Leu Pro Asp Thr Leu Lys Ile Thr Asn Ser Pro Gln Lys
           270                 275                 280
   tta atc agc tgc aga agg gag gag gtg gat gcc tgt gcc acg gct gtg      914
   Leu Ile Ser Cys Arg Arg Glu Glu Val Asp Ala Cys Ala Thr Ala Val
       285                 290                 295
   atg tct cct gag gaa ctc ctc agg gcc tgg atc tca taggtttgcg          960
   Met Ser Pro Glu Glu Leu Leu Arg Ala Trp Ile Ser
   300                 305                 310
   gaaggctcga g                                                        971
<210> 14
   <211> 311
   <212> PRT
   <213> Homo sapiens
<400> 14
   Met Gln Thr Phe Thr Met Val Leu Glu Glu Ile Trp Thr Ser Leu Phe
   1 5 10 15
   Met Trp Phe Phe Tyr Ala Leu Ile Pro Cys Leu Leu Thr Asp Glu Val
   20 25 30
   Ala Ile Leu Pro Ala Pro Gln Asn Leu Ser Val Leu Ser Thr Asn Met
   35 40 45
   Lys His Leu Leu Met Trp Ser Pro Val Ile Ala Pro Gly Glu Thr Val
   50 55 60
   Tyr Tyr Ser Val Glu Tyr Gln Gly Glu Tyr Glu Ser Leu Tyr Thr Ser
   65 70 75 80
   His Ile Trp Ile Pro Ser Ser Trp Cys Ser Leu Thr Glu Gly Pro Glu
   85 90 95
   Cys Asp Val Thr Asp Asp Ile Thr Ala Thr Val Pro Tyr Asn Leu Arg
   100 105 110
   Val Arg Ala Thr Leu Gly Ser Gln Thr Ser Ala Trp Ser Ile Leu Lys
   115 120 125
   His Pro Phe Asn Arg Asn Ser Thr Ile Leu Thr Arg Pro Gly Met Glu
   130 135 140
   Ile Thr Lys Asp Gly Phe His Leu Val Ile Glu Leu Glu Asp Leu Gly
   145 150 155 160
   Pro Gln Phe Glu Phe Leu Val Ala Tyr Trp Arg Arg Glu Pro Gly Ala
   165 170 175
   Glu Glu His Val Lys Met Val Arg Ser Gly Gly Ile Pro Val His Leu
   180 185 190
   Glu Thr Met Glu Pro Gly Ala Ala Tyr Cys Val Lys Ala Gln Thr Phe
   195 200 205
   Val Lys Ala Ile Gly Arg Tyr Ser Ala Phe Ser Gln Thr Glu Cys Val
   210 215 220
   Glu Val Gln Gly Glu Ala Ile Pro Leu Val Leu Ala Leu Phe Ala Phe
   225 230 235 240
   Val Gly Phe Met Leu Ile Leu Val Val Val Pro Leu Phe Val Trp Lys
   245 250 255
   Met Gly Arg Leu Leu Gln Tyr Ser Cys Cys Pro Val Val Val Leu Pro
   260 265 270
   Asp Thr Leu Lys Ile Thr Asn Ser Pro Gln Lys Leu Ile Ser Cys Arg
   275 280 285
   Arg Glu Glu Val Asp Ala Cys Ala Thr Ala Val Met Ser Pro Glu Glu
   290 295 300
   Leu Leu Arg Ala Trp Ile Ser
   305 310
<210> 15
   <211> 203
   <212> PRT
   <213> Homo sapiens
<400> 15
   Asp Glu Val Ala Ile Leu Pro Ala Pro Gln Asn Leu Ser Val Leu Ser
    1               5                  10                  15
   Thr Asn Met Lys His Leu Leu Met Trp Ser Pro Val Ile Ala Pro Gly
               20                  25                  30
   Glu Thr Val Tyr Tyr Ser Val Glu Tyr Gln Gly Glu Tyr Glu Ser Leu
           35                  40                  45
   Tyr Thr Ser His Ile Trp Ile Pro Ser Ser Trp Cys Ser Leu Thr Glu
       50                  55                  60
   Gly Pro Glu Cys Asp Val Thr Asp Asp Ile Thr Ala Thr Val Pro Tyr
   65                  70                  75                  80
   Asn Leu Arg Val Arg Ala Thr Leu Gly Ser Gln Thr Ser Ala Trp Ser
                   85                  90                  95
   Ile Leu Lys His Pro Phe Asn Arg Asn Ser Thr Ile Leu Thr Arg Pro
               100                 105                 110
   Gly Met Glu Ile Thr Lys Asp Gly Phe His Leu Val Ile Glu Leu Glu
           115                 120                 125
   Asp Leu Gly Pro Gln Phe Glu Phe Leu Val Ala Tyr Trp Arg Arg Glu
       130                 135                 140
   Pro Gly Ala Glu Glu His Val Lys Met Val Arg Ser Gly Gly Ile Pro
   145                 150                 155                 160
   Val His Leu Glu Thr Met Glu Pro Gly Ala Ala Tyr Cys Val Lys Ala
                   165                 170                 175
   Gln Thr Phe Val Lys Ala Ile Gly Arg Tyr Ser Ala Phe Ser Gln Thr
               180                 185                 190
   Glu Cys Val Glu Val Gln Gly Glu Ala Ile Pro
           195                 200
<210> 16
   <211> 33
   <212> DNA
   <213> Homo sapiens
<400> 16
   gcgaattcga gtctaccaaa tgcagacttt cac 33
<210> 17
   <211> 32
   <212> DNA
   <213> Homo sapiens
<400> 17
   cgctcgagcc ttccgcaaac ctatgagatc ca 32
<210> 18
   <211> 1379
   <212> DNA
   <213> Homo sapiens
<220>
   <221> CDS
   <222> (132)...(1034)
<400> 18
<210> 19
   <211> 301
   <212> PRT
   <213> Homo sapiens
<400> 19
   Met Gln Thr Phe Thr Met Val Leu Glu Glu Ile Trp Thr Ser Leu Phe
    1               5                  10                  15
   Met Trp Phe Phe Tyr Ala Leu Ile Pro Cys Leu Leu Thr Asp Glu Val
               20                  25                  30
   Ala Ile Leu Pro Ala Pro Gln Asn Leu Ser Val Leu Ser Thr Asn Met
           35                  40                  45
   Lys His Leu Leu Met Trp Ser Pro Val Ile Ala Pro Gly Glu Thr Val
       50                  55                  60
   Tyr Tyr Ser Val Glu Tyr Gln Gly Glu Tyr Glu Ser Leu Tyr Thr Ser
   65                  70                  75                  80
   His Ile Trp Ile Pro Ser Ser Trp Cys Ser Leu Thr Glu Gly Pro Glu
   85 90 95
   Cys Asp Val Thr Asp Asp Ile Thr Ala Thr Val Pro Tyr Asn Leu Arg
   100 105 110
   Val Arg Ala Thr Leu Gly Ser Gln Thr Ser Ala Trp Ser Ile Leu Lys
   115 120 125
   His Pro Phe Asn Arg Asn Ser Thr Ile Leu Thr Arg Pro Gly Met Glu
   130 135 140
   Ile Pro Lys His Gly Phe His Leu Val Ile Glu Leu Glu Asp Leu Gly
   145 150 155 160
   Pro Gln Phe Glu Phe Leu Val Ala Tyr Trp Thr Arg Glu Pro Gly Ala
   165 170 175
   Glu Glu His Val Lys Met Val Arg Ser Gly Gly Ile Pro Val His Leu
   180 185 190
   Glu Thr Met Glu Pro Gly Ala Ala Tyr Cys Val Lys Ala Gln Thr Phe
   195 200 205
   Val Lys Ala Ile Gly Arg Tyr Ser Ala Phe Ser Gln Thr Glu Cys Val
   210 215 220
   Glu Val Gln Gly Glu Ala Ile Pro Leu Val Leu Ala Leu Phe Ala Phe
   225 230 235 240
   Val Gly Phe Met Leu Ile Leu Val Val Val Pro Leu Phe Val Trp Lys
   245 250 255
   Met Gly Arg Leu Leu Gln Tyr Ser Cys Cys Pro Val Val Val Leu Pro
   260 265 270
   Asp Thr Leu Lys Ile Thr Asn Ser Pro Gln Val Asn Gln Leu Gln Lys
   275 280 285
   Gly Gly Gly Gly Cys Leu Cys His Gly Cys Asp Val Ser
   290 295 300
<210> 20
   <211> 1081
   <212> DNA
   <213> Homo sapiens
<220>
   <221> CDS
   <222> (9)...(1067)
<400> 20
   ggccggcc atg cag act ttc aca atg gtt cta gaa gaa atc tgg aca agt 50
   Met Gln Thr Phe Thr Met Val Leu Glu Glu Ile Trp Thr Ser
   1 5 10
   ctt ttc atg tgg ttt ttc tac gca ttg att cca tgt ttg ctc aca gat      98
   Leu Phe Met Trp Phe Phe Tyr Ala Leu Ile Pro Cys Leu Leu Thr Asp
    15                  20                  25                  30
   gaa gtg gcc att ctg cct gcc cct cag aac ctc tct gta ctc tca acc     146
   Glu Val Ala Ile Leu Pro Ala Pro Gln Asn Leu Ser Val Leu Ser Thr
                    35                  40                  45
   aac atg aag cat ctc ttg atg tgg agc cca gtg atc gcg cct gga gaa     194
   Asn Met Lys His Leu Leu Met Trp Ser Pro Val Ile Ala Pro Gly Glu
                50                  55                  60
   aca gtg tac tat tct gtc gaa tac cag ggg gag tac gag agc ctg tac     242
   Thr Val Tyr Tyr Ser Val Glu Tyr Gln Gly Glu Tyr Glu Ser Leu Tyr
            65                  70                  75
   acg agc cac atc tgg atc ccc agc agc tgg tgc tca ctc act gaa ggt     290
   Thr Ser His Ile Trp Ile Pro Ser Ser Trp Cys Ser Leu Thr Glu Gly
        80                  85                  90
   cct gag tgt gat gtc act gat gac atc acg gcc act gtg cca tac aac     338
   Pro Glu Cys Asp Val Thr Asp Asp Ile Thr Ala Thr Val Pro Tyr Asn
    95                 100                 105                 110
   ctt cgt gtc agg gcc aca ttg ggc tca cag acc tca gcc tgg agc atc     386
   Leu Arg Val Arg Ala Thr Leu Gly Ser Gln Thr Ser Ala Trp Ser Ile
                   115                 120                 125
   ctg aag cat ccc ttt aat aga aac tca acc atc ctt acc cga cct ggg     434
   Leu Lys His Pro Phe Asn Arg Asn Ser Thr Ile Leu Thr Arg Pro Gly
               130                 135                 140
   atg gag atc ccc aaa cat ggc ttc cac ctg gtt att gag ctg gag gac     482
   Met Glu Ile Pro Lys His Gly Phe His Leu Val Ile Glu Leu Glu Asp
           145                 150                 155
   ctg ggg ccc cag ttt gag ttc ctt gtg gcc tac tgg acg agg gag cct     530
   Leu Gly Pro Gln Phe Glu Phe Leu Val Ala Tyr Trp Thr Arg Glu Pro
       160                 165                 170
   ggt gcc gag gaa cat gtc aaa atg gtg agg agt ggg ggt att cca gtg     578
   Gly Ala Glu Glu His Val Lys Met Val Arg Ser Gly Gly Ile Pro Val
   175                 180                 185                 190
   cac cta gaa acc atg gag cca ggg gct gca tac tgt gtg aag gcc cag      626
   His Leu Glu Thr Met Glu Pro Gly Ala Ala Tyr Cys Val Lys Ala Gln
                   195                 200                 205
   aca ttc gtg aag gcc att ggg agg tac agc gcc ttc agc cag aca gaa      674
   Thr Phe Val Lys Ala Ile Gly Arg Tyr Ser Ala Phe Ser Gln Thr Glu
               210                 215                 220
   tgt gtg gag gtg caa gga gag gcc gga ggt ggt ggc agt gga ggc ggc      722
   Cys Val Glu Val Gln Gly Glu Ala Gly Gly Gly Gly Ser Gly Gly Gly
           225                 230                 235
   ggt agc gga ggc ggt ggc agt cga act gtg gct gca cca tct gtc ttc      770
   Gly Ser Gly Gly Gly Gly Ser Arg Thr Val Ala Ala Pro Ser Val Phe
       240                 245                 250
   atc ttc ccg cca tct gat gag cag ttg aaa tct gga act gcc tct gtt      818
   Ile Phe Pro Pro Ser Asp Glu Gln Leu Lys Ser Gly Thr Ala Ser Val
   255                 260                 265                 270
   gtg tgc ctg ctg aat aac ttc tat ccc aga gag gcc aaa gta cag tgg      866
   Val Cys Leu Leu Asn Asn Phe Tyr Pro Arg Glu Ala Lys Val Gln Trp
                   275                 280                 285
   aag gtg gat aac gcc ctc caa tcg ggt aac tcc cag gag agt gtc aca      914
   Lys Val Asp Asn Ala Leu Gln Ser Gly Asn Ser Gln Glu Ser Val Thr
               290                 295                 300
   gag cag gac agc aag gac agc acc tac agc ctc agc agc acc ctg acg      962
   Glu Gln Asp Ser Lys Asp Ser Thr Tyr Ser Leu Ser Ser Thr Leu Thr
           305                 310                 315
   ctg agc aaa gca gac tac gag aaa cac aaa gtc tac gcc tgc gaa gtc     1010
   Leu Ser Lys Ala Asp Tyr Glu Lys His Lys Val Tyr Ala Cys Glu Val
       320                 325                 330
   acc cat cag ggc ctg agc tcg ccc gtc aca aag agc ttc aac agg gga     1058
   Thr His Gln Gly Leu Ser Ser Pro Val Thr Lys Ser Phe Asn Arg Gly
   335                 340                 345                 350
   gag tgt taa tctagaggcg cgcc                                         1081
   Glu Cys  *
<210> 21
   <211> 352
   <212> PRT
   <213> Homo sapiens
<400> 21
   Met Gln Thr Phe Thr Met Val Leu Glu Glu Ile Trp Thr Ser Leu Phe
   1               5                   10                  15
   Met Trp Phe Phe Tyr Ala Leu Ile Pro Cys Leu Leu Thr Asp Glu Val
               20                  25                  30
   Ala Ile Leu Pro Ala Pro Gln Asn Leu Ser Val Leu Ser Thr Asn Met
           35                  40                  45
   Lys His Leu Leu Met Trp Ser Pro Val Ile Ala Pro Gly Glu Thr Val
       50                  55                  60
   Tyr Tyr Ser Val Glu Tyr Gln Gly Glu Tyr Glu Ser Leu Tyr Thr Ser
   65                  70                  75                  80
   His Ile Trp Ile Pro Ser Ser Trp Cys Ser Leu Thr Glu Gly Pro Glu
                   85                  90                  95
   Cys Asp Val Thr Asp Asp Ile Thr Ala Thr Val Pro Tyr Asn Leu Arg
               100                 105                 110
   Val Arg Ala Thr Leu Gly Ser Gln Thr Ser Ala Trp Ser Ile Leu Lys
           115                 120                 125
   His Pro Phe Asn Arg Asn Ser Thr Ile Leu Thr Arg Pro Gly Met Glu
       130                 135                 140
   Ile Pro Lys His Gly Phe His Leu Val Ile Glu Leu Glu Asp Leu Gly
   145                 150                 155                 160
   Pro Gln Phe Glu Phe Leu Val Ala Tyr Trp Thr Arg Glu Pro Gly Ala
                   165                 170                 175
   Glu Glu His Val Lys Met Val Arg Ser Gly Gly Ile Pro Val His Leu
               180                 185                 190
   Glu Thr Met Glu Pro Gly Ala Ala Tyr Cys Val Lys Ala Gln Thr Phe
           195                 200                 205
   Val Lys Ala Ile Gly Arg Tyr Ser Ala Phe Ser Gln Thr Glu Cys Val
       210                 215                 220
   Glu Val Gln Gly Glu Ala Gly Gly Gly Gly Ser Gly Gly Gly Gly Ser
   225                 230                 235                 240
   Gly Gly Gly Gly Ser Arg Thr Val Ala Ala Pro Ser Val Phe Ile Phe
                   245                 250                 255
   Pro Pro Ser Asp Glu Gln Leu Lys Ser Gly Thr Ala Ser Val Val Cys
               260                 265                 270
   Leu Leu Asn Asn Phe Tyr Pro Arg Glu Ala Lys Val Gln Trp Lys Val
           275                 280                 285
   Asp Asn Ala Leu Gln Ser Gly Asn Ser Gln Glu Ser Val Thr Glu Gln
       290                 295                 300
   Asp Ser Lys Asp Ser Thr Tyr Ser Leu Ser Ser Thr Leu Thr Leu Ser
   305                 310                 315                 320
   Lys Ala Asp Tyr Glu Lys His Lys Val Tyr Ala Cys Glu Val Thr His
                   325                 330                 335
   Gln Gly Leu Ser Ser Pro Val Thr Lys Ser Phe Asn Arg Gly Glu Cys
               340                 345                 350
<210> 22
   <211> 1801
   <212> DNA
   <213> Homo sapiens
<220>
   <221> CDS
   <222> (8)...(1789)
<400> 22
   gtcgacc atg gat gca atg aag aga ggg ctc tgc tgt gtg ctg ctg ctg       49
   tgt ggc gcc gtc ttc gtt tcg ctc agc cag gaa atc cat gcc gag ttg       97
   aga cgc ttc cgt aga gtt ccc tgt gtc tct ggt ggt ttg cct aaa cct      145
   gca aac atc acc ttc tta tcc atc aac atg aag aat gtc cta caa tgg      193
   act cca cca gag ggt ctt caa gga gtt aaa gtt act tac act gtg cag      241
   tat ttc ata tat ggg caa aag aaa tgg ctg aat aaa tca gaa tgc aga      289
   aat atc aat aga acc tac tgt gat ctt tct gct gaa act tct gac tac       337
   gaa cac cag tat tat gcc aaa gtt aag gcc att tgg gga aca aag tgt       385
   tcc aaa tgg gct gaa agt gga cgg ttc tat cct ttt tta gaa aca caa       433
   att ggc cca cca gag gtg gca ctg act aca gat gag aag tcc att tct       481
   gtt gtc ctg aca gct cca gag aag tgg aag aga aat cca gaa gac ctt       529
   cct gtt tcc atg caa caa ata tac tcc aat ctg aag tat aac gtg tct       577
   gtg ttg aat act aaa tca aac aga acg tgg tcc cag tgt gtg acc aac       625
   cac acg ctg gtg ctc acc tgg ctg gag ccg aac act ctt tac tgc gta       673
   cac gtg gag tcc ttc gtc cca ggg ccc cct cgc cgt gct cag cct tct       721
   gag aag cag tgt gcc agg act ttg aaa gat caa ggt gga ggc ggt tca       769
   ggc gga ggt ggc tct ggc ggt ggc gga tcg gcc tcc acc aag ggc cca       817
   tcg gtc ttc ccc ctg gca ccc tcc tcc aag agc acc tct ggg ggc aca 865
   gcg gcc ctg ggc tgc ctg gtc aag gac tac ttc ccc gaa ccg gtg acg 913
   gtg tcg tgg aac tca ggc gcc ctg acc agc ggc gtg cac acc ttc ccg 961
   gct gtc cta cag tcc tca gga ctc tac tcc ctc agc agc gtg gtg acc 1009
   gtg ccc tcc agc agc ttg ggc acc cag acc tac atc tgc aac gtg aat 1057
   cac aag ccc agc aac acc aag gtg gac aag aaa gtt gag ccc aaa tct 1105
   tgt gac aaa act cac aca tgc cca ccg tgc cca gca cct gaa gcc gag 1153
   ggg gca ccg tca gtc ttc ctc ttc ccc cca aaa ccc aag gac acc ctc 1201
   atg atc tcc cgg acc cct gag gtc aca tgc gtg gtg gtg gac gtg agc 1249
   cac gaa gac cct gag gtc aag ttc aac tgg tac gtg gac ggc gtg gag 1297
   gtg cat aat gcc aag aca aag ccg cgg gag gag cag tac aac agc acg 1345
   tac cgt gtg gtc agc gtc ctc acc gtc ctg cac cag gac tgg ctg aat      1393
   ggc aag gag tac aag tgc aag gtc tcc aac aaa gcc ctc cca tcc tcc      1441
   atc gag aaa acc atc tcc aaa gcc aaa ggg cag ccc cga gaa cca cag      1489
   gtg tac acc ctg ccc cca tcc cgg gat gag ctg acc aag aac cag gtc      1537
   agc ctg acc tgc ctg gtc aaa ggc ttc tat ccc agc gac atc gcc gtg      1585
   gag tgg gag agc aat ggg cag ccg gag aac aac tac aag acc acg cct      1633
   ccc gtg ctg gac tcc gac ggc tcc ttc ttc ctc tac agc aag ctc acc      1681
   gtg gac aag agc agg tgg cag cag ggg aac gtc ttc tca tgc tcc gtg      1729
   atg cat gag gct ctg cac aac cac tac acg cag aag agc ctc tcc ctg      1777
   tct ccg ggt aaa taatctagat ct                                        1801
<210> 23
   <211> 594
   <212> PRT
   <213> Homo sapiens
<400> 23
<210> 24
   <211> 29
   <212> DNA
   <213> Homo sapiens
<400> 24
   ggccggccat gcagactttc acaatggtt 29
<210> 25
   <211> 52
   <212> DNA
   <213> homo sapiens
<400> 25
   tccgctaccg ccgcctccac tgccaccacc tccggcctct ccttgcacct cc 52
<210> 26
   <211> 53
   <212> DNA
   <213> Homo sapiens
<400> 26
   gtggaggcgg cggtagcgga ggcggtggca gtcgaactgt ggctgcacca tct 53
<210> 27
   <211> 38
   <212> DNA
   <213> Homo sapiens
<400> 27
   ggcgcgcctc tagattaaca ctctcccctg ttgaagct 38
<210> 28
   <211> 30
   <212> DNA
   <213> Homo sapiens
<400> 28
   gtcgaccatg gatgcaatga agagagggct 30
<210> 29
   <211> 30
   <212> DNA
   <213> Homo sapiens
<400> 29
   cacagggaac tctacggaag cgtctcaact 30
<210> 30
   <211> 33
   <212> DNA
   <213> Homo sapiens
<400> 30
   cttccgtaga gttccctgtg tctctggtgg ttt 33
<210> 31
   <211> 53
   <212> DNA
   <213> Homo sapiens
<400> 31
   gccagagcca cctccgcctg aaccgcctcc accttgatct ttcaaagtcc tgg 53
<210> 32
   <211> 51
   <212> DNA
   <213> Homo sapiens
<400> 32
   caggcggagg tggctctggc ggtggcggat cggcctccac caagggccca t 51
<210> 33
   <211> 20
   <212> DNA
   <213> Homo sapiens
<400> 33
   ctgggcacgg tgggcatgtg 20
<210> 34
   <211> 20
   <212> DNA
   <213> Homo sapiens
<400> 34
   cacatgccca ccgtgcccag 20
<210> 35
   <211> 31
   <212> DNA
   <213> Homo sapiens
<400> 35
   agatctagat tatttacccg gagacaggga g 31
<210> 36
   <211> 1806
   <212> DNA
   <213> Mus musculus
<220>
   <221> CDS
   <222> (38)...(1675)
<400> 36
   cgccgcgttc ccgagatgtg acccgaactg acagccc atg cac act ccc ggg acc     55
                                            Met His Thr Pro Gly Thr
                                            1               5
   ccg gcg ccg ggc cac ccg gac ccg ccg cca ctg ttg ctg ctc acg ctg     103
   Pro Ala Pro Gly His Pro Asp Pro Pro Pro Leu Leu Leu Leu Thr Leu
               10                  15                  20
   ctt ctg ctg ctg gcc gct tcg gga cgc gca gtt cct tgt gtc ttc tgt     151
   Leu Leu Leu Leu Ala Ala Ser Gly Arg Ala Val Pro Cys Val Phe Cys
           25                  30                  35
   ggt ttg cct aaa cct aca aat atc acc ttc tta tcc atc aac atg aag     199
   Gly Leu Pro Lys Pro Thr Asn Ile Thr Phe Leu Ser Ile Asn Met Lys
       40                  45                  50
   aat gtc ctg cat tgg aat cca cca gag agt cta cac gga gtt gaa gtc     247
   Asn Val Leu His Trp Asn Pro Pro Glu Ser Leu His Gly Val Glu Val
   55                  60                  65                  70
   aca tac act gtg caa tat ttc ata tat ggg cag aag aaa tgg ctg aat     295
   Thr Tyr Thr Val Gln Tyr Phe Ile Tyr Gly Gln Lys Lys Trp Leu Asn
                   75                  80                  85
   gcc tct aaa tgc ggg agt atc aac agg acc tac tgt gac ctt tct gtt     343
   Ala Ser Lys Cys Gly Ser Ile Asn Arg Thr Tyr Cys Asp Leu Ser Val
               90                  95                  100
   gag acc tca gac tat gaa cac cag ttc tat gcc aaa gtg aag gcc att      391
   Glu Thr Ser Asp Tyr Glu His Gln Phe Tyr Ala Lys Val Lys Ala Ile
           105                 110                 115
   tgg gaa gcc agg tgc tcc gaa tgg gcc gag acg gaa cgc ttc tat cct      439
   Trp Glu Ala Arg Cys Ser Glu Trp Ala Glu Thr Glu Arg Phe Tyr Pro
       120                 125                 130
   ttc ttg gaa act caa gtc agc cca cca gag att gcc ctg aca act ggc      487
   Phe Leu Glu Thr Gln Val Ser Pro Pro Glu Ile Ala Leu Thr Thr Gly
   135                 140                 145                 150
   gag aag tcc atc tct att gcc ctg aca gca cca gag aag tgg aaa aga      535
   Glu Lys Ser Ile Ser Ile Ala Leu Thr Ala Pro Glu Lys Trp Lys Arg
                   155                 160                 165
   aat cca caa gac cac act gtt tct atg caa cag ata tac ccc aat ttg      583
   Asn Pro Gln Asp His Thr Val Ser Met Gln Gln Ile Tyr Pro Asn Leu
               170                 175                 180
   aag tac aat gtg tct gtg tat aac act aag tcg aga aga acg tgg tcc      631
   Lys Tyr Asn Val Ser Val Tyr Asn Thr Lys Ser Arg Arg Thr Trp Ser
           185                 190                 195
   cag tgt gtc acc aac agc aca ctg gtc ctc agc tgg ctg gag ccc aac      679
   Gln Cys Val Thr Asn Ser Thr Leu Val Leu Ser Trp Leu Glu Pro Asn
       200                 205                 210
   act ctg tat tgt gtc cac gtg gag tcc ctt gtc cca ggg ccc cct cgc      727
   Thr Leu Tyr Cys Val His Val Glu Ser Leu Val Pro Gly Pro Pro Arg
   215                 220                 225                 230
   ctc ccg atg cct tct cag aag cag tgc atc agt act ttg gaa gtt caa      775
   Leu Pro Met Pro Ser Gln Lys Gln Cys Ile Ser Thr Leu Glu Val Gln
                   235                 240                 245
   aca tca gca tgg aag gct aaa gtc atc ttc tgg tat gtc ttc ctc aca      823
   Thr Ser Ala Trp Lys Ala Lys Val Ile Phe Trp Tyr Val Phe Leu Thr
               250                 255                 260
   tct gtt atc gtg ttt ctt ttc tcc gca att ggc tac ttg gtt tac cgt      871
   Ser Val Ile Val Phe Leu Phe Ser Ala Ile Gly Tyr Leu Val Tyr Arg
           265                 270                 275
   tac atc cat gtt ggc aag gaa aaa cac cca gca aat ttg gta ctg att      919
   Tyr Ile His Val Gly Lys Glu Lys His Pro Ala Asn Leu Val Leu Ile
       280                 285                 290
   tat aga aat gaa att ggc aca aga gtc ttt gaa cct act gaa aca atc      967
   Tyr Arg Asn Glu Ile Gly Thr Arg Val Phe Glu Pro Thr Glu Thr Ile
   295                 300                 305                 310
   aca ctt aat ttt atc acc ttc agt atg ttg gat gat act aaa att tct     1015
   Thr Leu Asn Phe Ile Thr Phe Ser Met Leu Asp Asp Thr Lys Ile Ser
                   315                 320                 325
   cca aag gat atg aat tta ctg gac aaa agc agt gat gac atc agt gtt     1063
   Pro Lys Asp Met Asn Leu Leu Asp Lys Ser Ser Asp Asp Ile Ser Val
               330                 335                 340
   aat gac cct gag cac aat gag gcc tgg gag ccg cac tgg gag gag gtg     1111
   Asn Asp Pro Glu His Asn Glu Ala Trp Glu Pro His Trp Glu Glu Val
           345                 350                 355
   gag ggg caa cat tta gga tgc tct tcg cat ttg atg gac gct gtc tgt     1159
   Glu Gly Gln His Leu Gly Cys Ser Ser His Leu Met Asp Ala Val Cys
       360                 365                 370
   ggt gct gag caa aga gac gga gac acc tcc cta acc cag cat ggg tgg     1207
   Gly Ala Glu Gln Arg Asp Gly Asp Thr Ser Leu Thr Gln His Gly Trp
   375                 380                 385                 390
   ctt aac agc acc atc ccc aca gga gag aca gac act gag cct caa tac     1255
   Leu Asn Ser Thr Ile Pro Thr Gly Glu Thr Asp Thr Glu Pro Gln Tyr
                   395                 400                 405
   aaa gtc cta agt gac ttc tac ggg gag ggt gaa atc caa ctg tcc tgt     1303
   Lys Val Leu Ser Asp Phe Tyr Gly Glu Gly Glu Ile Gln Leu Ser Cys
               410                 415                 420
   gag ccg gaa gag gcg gcc aga aca gag aaa ata tct gag cca ctg gtg     1351
   Glu Pro Glu Glu Ala Ala Arg Thr Glu Lys Ile Ser Glu Pro Leu Val
           425                 430                 435
   act tca gca aac ttg gac cca cag ctt gaa gac cta cat cac ctg ggt     1399
   Thr Ser Ala Asn Leu Asp Pro Gln Leu Glu Asp Leu His His Leu Gly
       440                 445                 450
   cag gag cat act gtc tcc gag gat ggg cca gag gaa gag aca tct ata      1447
   Gln Glu His Thr Val Ser Glu Asp Gly Pro Glu Glu Glu Thr Ser Ile
   455                 460                 465                 470
   aca gta gtg gat tgg gac cct caa act ggc agg ctg tgt atc cct tcc      1495
   Thr Val Val Asp Trp Asp Pro Gln Thr Gly Arg Leu Cys Ile Pro Ser
                   475                 480                 485
   tta cct atc ttt ggc cgt gat cct gag aac tat ggt cat tat gag aga      1543
   Leu Pro Ile Phe Gly Arg Asp Pro Glu Asn Tyr Gly His Tyr Glu Arg
               490                 495                 500
   gac cag ctc tta gag ggt ggc ctt ttg tct aga ctc tat gag aac cag      1591
   Asp Gln Leu Leu Glu Gly Gly Leu Leu Ser Arg Leu Tyr Glu Asn Gln
           505                 510                 515
   gca cct gac aag cca gag aaa gaa aat gaa aac tgt ctc aca cgg ttt      1639
   Ala Pro Asp Lys Pro Glu Lys Glu Asn Glu Asn Cys Leu Thr Arg Phe
       520                 525                 530
   atg gag gaa tgg ggg tta cat gta caa atg gaa agc tagtgccagg           1685
   Met Glu Glu Trp Gly Leu His Val Gln Met Glu Ser
   535                 540                 545
   ctttctgttg actgccaaca aatgaaggaa ccatcccagg gggtgaacag tgttcaggtt    1745
   atcagtgtca gcaatgagac tgttctctct gttcatgaac tttgtcagcc ctgcctcatc    1805
   c                                                                    1806
<210> 37
   <211> 546
   <212> PRT
   <213> Mus musculus
<400> 37
   Met His Thr Pro Gly Thr Pro Ala Pro Gly His Pro Asp Pro Pro Pro
    1               5                  10                  15
   Leu Leu Leu Leu Thr Leu Leu Leu Leu Leu Ala Ala Ser Gly Arg Ala
               20                  25                  30
   Val Pro Cys Val Phe Cys Gly Leu Pro Lys Pro Thr Asn Ile Thr Phe
           35                  40                  45
   Leu Ser Ile Asn Met Lys Asn Val Leu His Trp Asn Pro Pro Glu Ser
       50                  55                  60
   Leu His Gly Val Glu Val Thr Tyr Thr Val Gln Tyr Phe Ile Tyr Gly
   65                  70                  75                  80
   Gln Lys Lys Trp Leu Asn Ala Ser Lys Cys Gly Ser Ile Asn Arg Thr
                   85                  90                  95
   Tyr Cys Asp Leu Ser Val Glu Thr Ser Asp Tyr Glu His Gln Phe Tyr
               100                 105                 110
   Ala Lys Val Lys Ala Ile Trp Glu Ala Arg Cys Ser Glu Trp Ala Glu
           115                 120                 125
   Thr Glu Arg Phe Tyr Pro Phe Leu Glu Thr Gln Val Ser Pro Pro Glu
       130                 135                 140
   Ile Ala Leu Thr Thr Gly Glu Lys Ser Ile Ser Ile Ala Leu Thr Ala
   145                 150                 155                 160
   Pro Glu Lys Trp Lys Arg Asn Pro Gln Asp His Thr Val Ser Met Gln
                   165                 170                 175
   Gln Ile Tyr Pro Asn Leu Lys Tyr Asn Val Ser Val Tyr Asn Thr Lys
               180                 185                 190
   Ser Arg Arg Thr Trp Ser Gln Cys Val Thr Asn Ser Thr Leu Val Leu
           195                 200                 205
   Ser Trp Leu Glu Pro Asn Thr Leu Tyr Cys Val His Val Glu Ser Leu
       210                 215                 220
   Val Pro Gly Pro Pro Arg Leu Pro Met Pro Ser Gln Lys Gln Cys Ile
   225                 230                 235                 240
   Ser Thr Leu Glu Val Gln Thr Ser Ala Trp Lys Ala Lys Val Ile Phe
                   245                 250                 255
   Trp Tyr Val Phe Leu Thr Ser Val Ile Val Phe Leu Phe Ser Ala Ile
               260                 265                 270
   Gly Tyr Leu Val Tyr Arg Tyr Ile His Val Gly Lys Glu Lys His Pro
           275                 280                 285
   Ala Asn Leu Val Leu Ile Tyr Arg Asn Glu Ile Gly Thr Arg Val Phe
       290                 295                 300
   Glu Pro Thr Glu Thr Ile Thr Leu Asn Phe Ile Thr Phe Ser Met Leu
   305                 310                 315                 320
   Asp Asp Thr Lys Ile Ser Pro Lys Asp Met Asn Leu Leu Asp Lys Ser
                   325                 330                 335
   Ser Asp Asp Ile Ser Val Asn Asp Pro Glu His Asn Glu Ala Trp Glu
               340                 345                 350
   Pro His Trp Glu Glu Val Glu Gly Gln His Leu Gly Cys Ser Ser His
           355                 360                 365
   Leu Met Asp Ala Val Cys Gly Ala Glu Gln Arg Asp Gly Asp Thr Ser
       370                 375                 380
   Leu Thr Gln His Gly Trp Leu Asn Ser Thr Ile Pro Thr Gly Glu Thr
   385 390 395 400
   Asp Thr Glu Pro Gln Tyr Lys Val Leu Ser Asp Phe Tyr Gly Glu Gly
   405 410 415
   Glu Ile Gln Leu Ser Cys Glu Pro Glu Glu Ala Ala Arg Thr Glu Lys
   420 425 430
   Ile Ser Glu Pro Leu Val Thr Ser Ala Asn Leu Asp Pro Gln Leu Glu
   435 440 445
   Asp Leu His His Leu Gly Gln Glu His Thr Val Ser Glu Asp Gly Pro
   450 455 460
   Glu Glu Glu Thr Ser Ile Thr Val Val Asp Trp Asp Pro Gln Thr Gly
   465 470 475 480
   Arg Leu Cys Ile Pro Ser Leu Pro Ile Phe Gly Arg Asp Pro Glu Asn
   485 490 495
   Tyr Gly His Tyr Glu Arg Asp Gln Leu Leu Glu Gly Gly Leu Leu Ser
   500 505 510
   Arg Leu Tyr Glu Asn Gln Ala Pro Asp Lys Pro Glu Lys Glu Asn Glu
   515 520 525
   Asn Cys Leu Thr Arg Phe Met Glu Glu Trp Gly Leu His Val Gln Met
   530 535 540
   Glu Ser
   545
<210> 38
   <211> 217
   <212> PRT
   <213> Mus musculus
<400> 38
   Val Pro Cys Val Phe Cys Gly Leu Pro Lys Pro Thr Asn Ile Thr Phe
   1 5 10 15
   Leu Ser Ile Asn Met Lys Asn Val Leu His Trp Asn Pro Pro Glu Ser
   20 25 30
   Leu His Gly Val Glu Val Thr Tyr Thr Val Gln Tyr Phe Ile Tyr Gly
   35 40 45
   Gln Lys Lys Trp Leu Asn Ala Ser Lys Cys Gly Ser Ile Asn Arg Thr
   50 55 60
   Tyr Cys Asp Leu Ser Val Glu Thr Ser Asp Tyr Glu His Gln Phe Tyr
   65 70 75 80
   Ala Lys Val Lys Ala Ile Trp Glu Ala Arg Cys Ser Glu Trp Ala Glu
   85 90 95
   Thr Glu Arg Phe Tyr Pro Phe Leu Glu Thr Gln Val Ser Pro Pro Glu
   100 105 110
   Ile Ala Leu Thr Thr Gly Glu Lys Ser Ile Ser Ile Ala Leu Thr Ala
   115 120 125
   Pro Glu Lys Trp Lys Arg Asn Pro Gln Asp His Thr Val Ser Met Gln
   130 135 140
   Gln Ile Tyr Pro Asn Leu Lys Tyr Asn Val Ser Val Tyr Asn Thr Lys
   145 150 155 160
   Ser Arg Arg Thr Trp Ser Gln Cys Val Thr Asn Ser Thr Leu Val Leu
   165 170 175
   Ser Trp Leu Glu Pro Asn Thr Leu Tyr Cys Val His Val Glu Ser Leu
   180 185 190
   Val Pro Gly Pro Pro Arg Leu Pro Met Pro Ser Gln Lys Gln Cys Ile
   195 200 205
   Ser Thr Leu Glu Val Gln Thr Ser Ala
   210 215
<210> 39
   <211> 514
   <212> PRT
   <213> Mus musculus
<400> 39
   Val Pro Cys Val Phe Cys Gly Leu Pro Lys Pro Thr Asn Ile Thr Phe
   1 5 10 15
   Leu Ser Ile Asn Met Lys Asn Val Leu His Trp Asn Pro Pro Glu Ser
   20 25 30
   Leu His Gly Val Glu Val Thr Tyr Thr Val Gln Tyr Phe Ile Tyr Gly
   35 40 45
   Gln Lys Lys Trp Leu Asn Ala Ser Lys Cys Gly Ser Ile Asn Arg Thr
   50 55 60
   Tyr Cys Asp Leu Ser Val Glu Thr Ser Asp Tyr Glu His Gln Phe Tyr
   65 70 75 80
   Ala Lys Val Lys Ala Ile Trp Glu Ala Arg Cys Ser Glu Trp Ala Glu
   85 90 95
   Thr Glu Arg Phe Tyr Pro Phe Leu Glu Thr Gln Val Ser Pro Pro Glu
   100 105 110
   Ile Ala Leu Thr Thr Gly Glu Lys Ser Ile Ser Ile Ala Leu Thr Ala
   115 120 125
   Pro Glu Lys Trp Lys Arg Asn Pro Gln Asp His Thr Val Ser Met Gln
   130 135 140
   Gln Ile Tyr Pro Asn Leu Lys Tyr Asn Val Ser Val Tyr Asn Thr Lys
   145 150 155 160
   Ser Arg Arg Thr Trp Ser Gln Cys Val Thr Asn Ser Thr Leu Val Leu
   165 170 175
   Ser Trp Leu Glu Pro Asn Thr Leu Tyr Cys Val His Val Glu Ser Leu
               180                 185                 190
   Val Pro Gly Pro Pro Arg Leu Pro Met Pro Ser Gln Lys Gln Cys Ile
           195                 200                 205
   Ser Thr Leu Glu Val Gln Thr Ser Ala Trp Lys Ala Lys Val Ile Phe
       210                 215                 220
   Trp Tyr Val Phe Leu Thr Ser Val Ile Val Phe Leu Phe Ser Ala Ile
   225                 230                 235                 240
   Gly Tyr Leu Val Tyr Arg Tyr Ile His Val Gly Lys Glu Lys His Pro
                   245                 250                 255
   Ala Asn Leu Val Leu Ile Tyr Arg Asn Glu Ile Gly Thr Arg Val Phe
               260                 265                 270
   Glu Pro Thr Glu Thr Ile Thr Leu Asn Phe Ile Thr Phe Ser Met Leu
           275                 280                 285
   Asp Asp Thr Lys Ile Ser Pro Lys Asp Met Asn Leu Leu Asp Lys Ser
       290                 295                 300
   Ser Asp Asp Ile Ser Val Asn Asp Pro Glu His Asn Glu Ala Trp Glu
   305                 310                 315                 320
   Pro His Trp Glu Glu Val Glu Gly Gln His Leu Gly Cys Ser Ser His
                   325                 330                 335
   Leu Met Asp Ala Val Cys Gly Ala Glu Gln Arg Asp Gly Asp Thr Ser
               340                 345                 350
   Leu Thr Gln His Gly Trp Leu Asn Ser Thr Ile Pro Thr Gly Glu Thr
           355                 360                 365
   Asp Thr Glu Pro Gln Tyr Lys Val Leu Ser Asp Phe Tyr Gly Glu Gly
       370                 375                 380
   Glu Ile Gln Leu Ser Cys Glu Pro Glu Glu Ala Ala Arg Thr Glu Lys
   385                 390                 395                 400
   Ile Ser Glu Pro Leu Val Thr Ser Ala Asn Leu Asp Pro Gln Leu Glu
                   405                 410                 415
   Asp Leu His His Leu Gly Gln Glu His Thr Val Ser Glu Asp Gly Pro
               420                 425                 430
   Glu Glu Glu Thr Ser Ile Thr Val Val Asp Trp Asp Pro Gln Thr Gly
           435                 440                 445
   Arg Leu Cys Ile Pro Ser Leu Pro Ile Phe Gly Arg Asp Pro Glu Asn
       450                 455                 460
   Tyr Gly His Tyr Glu Arg Asp Gln Leu Leu Glu Gly Gly Leu Leu Ser
   465                 470                 475                 480
   Arg Leu Tyr Glu Asn Gln Ala Pro Asp Lys Pro Glu Lys Glu Asn Glu
                   485                 490                 495
   Asn Cys Leu Thr Arg Phe Met Glu Glu Trp Gly Leu His Val Gln Met
               500                 505                 510
   Glu Ser
<210> 40
   <211> 18
   <212> DNA
   <213> Mus musculus
<400> 40
   cgccgcgttc ccgagatg 18
<210> 41
   <211> 24
   <212> DNA
   <213> Mus musculus
<400> 41
   ggatgaggca gggctgacaa agtt 24
<210> 42
   <211> 36
   <212> DNA
   <213> Homo sapiens
<400> 42
   acttgtggaa ttcgctagca ccaagggccc atcggt 36
<210> 43
   <211> 32
   <212> DNA
   <213> Homo sapiens
<400> 43
   gcctagaacg cgttcattta cccggagaca gg 32
<210> 44
   <211> 8
   <212> DNA
   <213> Homo sapiens
<400> 44
   aattgaga 8
<210> 45
   <211> 8
   <212> DNA
   <213> Homo sapiens
<400> 45
   cgcgtctc 8
<210> 46
   <211> 37
   <212> DNA
   <213> Homo sapiens
<400> 46
   gtcacttgaa ttcggtaccg cctctgttgt gtgcctg 37
<210> 47
   <211> 32
   <212> DNA
   <213> Homo sapiens
<400> 47
   gacctgaacg cgtctaacac tctcccctgt tg 32
<210> 48
   <211> 38
   <212> DNA
   <213> Homo sapiens
<400> 48
   tcagtcggaa ttcgcagaag ccatgcgggc tcccggcc 38
<210> 49
   <211> 35
   <212> DNA
   <213> Homo sapiens
<400> 49
   ctgtgacgct agcctctgat gattgatctt tcaaa 35
<210> 50
   <211> 43
   <212> DNA
   <213> Homo sapiens
<400> 50
   gatgtctgaa ttcgcagaag ccatgcagac tttcacaatg gtt 43
<210> 51
   <211> 86
   <212> DNA
   <213> Homo sapiens
<400> 51
   aagacggtac cagatttcaa ctgctcatca gatggcggga agatgaagac agatggtgca 60
   gccacagtgg cctctccttg cacctc 86
<210> 52
   <211> 1720
   <212> DNA
   <213> Homo sapiens
<220>
   <221> CDS
   <222> (1)...(1713)
<400> 52
   atg cgg gct ccc ggc cgc ccg gcc ctg cgg ccg ctg ctg ctg ttg ctc      48
   Met Arg Ala Pro Gly Arg Pro Ala Leu Arg Pro Leu Leu Leu Leu Leu
    1               5                  10                  15
   ctg gcg gcg cct tgg gga cgg gca gtt ccc tgt gtc tct ggt ggt ttg      96
   Leu Ala Ala Pro Trp Gly Arg Ala Val Pro Cys Val Ser Gly Gly Leu
               20                  25                  30
   cct aaa cct gca aac atc acc ttc tta tcc atc aac atg aag aat gtc     144
   Pro Lys Pro Ala Asn Ile Thr Phe Leu Ser Ile Asn Met Lys Asn Val
           35                  40                  45
   cta caa tgg act cca cca gag ggt ctt caa gga gtt aaa gtt act tac     192
   Leu Gln Trp Thr Pro Pro Glu Gly Leu Gln Gly Val Lys Val Thr Tyr
       50                  55                  60
   act gtg cag tat ttc ata tat ggg caa aag aaa tgg ctg aat aaa tca     240
   Thr Val Gln Tyr Phe Ile Tyr Gly Gln Lys Lys Trp Leu Asn Lys Ser
   65                  70                  75                  80
   gaa tgc aga aat atc aat aga acc tac tgt gat ctt tct gct gaa act     288
   Glu Cys Arg Asn Ile Asn Arg Thr Tyr Cys Asp Leu Ser Ala Glu Thr
                   85                  90                  95
   tct gac tac gaa cac cag tat tat gcc aaa gtt aag gcc att tgg gga      336
   Ser Asp Tyr Glu His Gln Tyr Tyr Ala Lys Val Lys Ala Ile Trp Gly
               100                 105                 110
   aca aag tgt tcc aaa tgg gct gaa agt gga cgg ttc tat cct ttt tta      384
   Thr Lys Cys Ser Lys Trp Ala Glu Ser Gly Arg Phe Tyr Pro Phe Leu
           115                 120                 125
   gaa aca caa att ggc cca cca gag gtg gca ctg act aca gat gag aag      432
   Glu Thr Gln Ile Gly Pro Pro Glu Val Ala Leu Thr Thr Asp Glu Lys
       130                 135                 140
   tcc att tct gtt gtc ctg aca gct cca gag aag tgg aag aga aat cca      480
   Ser Ile Ser Val Val Leu Thr Ala Pro Glu Lys Trp Lys Arg Asn Pro
   145                 150                 155                 160
   gaa gac ctt cct gtt tcc atg caa caa ata tac tcc aat ctg aag tat      528
   Glu Asp Leu Pro Val Ser Met Gln Gln Ile Tyr Ser Asn Leu Lys Tyr
                   165                 170                 175
   aac gtg tct gtg ttg aat act aaa tca aac aga acg tgg tcc cag tgt      576
   Asn Val Ser Val Leu Asn Thr Lys Ser Asn Arg Thr Trp Ser Gln Cys
               180                 185                 190
   gtg acc aac cac acg ctg gtg ctc acc tgg ctg gag ccg aac act ctt      624
   Val Thr Asn His Thr Leu Val Leu Thr Trp Leu Glu Pro Asn Thr Leu
           195                 200                 205
   tac tgc gta cac gtg gag tcc ttc gtc cca ggg ccc cct cgc cgt gct      672
   Tyr Cys Val His Val Glu Ser Phe Val Pro Gly Pro Pro Arg Arg Ala
       210                 215                 220
   cag cct tct gag aag cag tgt gcc agg act ttg aaa gat caa tca tca      720
   Gln Pro Ser Glu Lys Gln Cys Ala Arg Thr Leu Lys Asp Gln Ser Ser
   225                 230                 235                 240
   gag gct agc acc aag ggc cca tcg gtc ttc ccc ctg gca ccc tcc tcc      768
   Glu Ala Ser Thr Lys Gly Pro Ser Val Phe Pro Leu Ala Pro Ser Ser
                   245                 250                 255
   aag agc acc tct ggg ggc aca gcg gcc ctg ggc tgc ctg gtc aag gac     816
   Lys Ser Thr Ser Gly Gly Thr Ala Ala Leu Gly Cys Leu Val Lys Asp
               260                 265                 270
   tac ttc ccc gaa ccg gtg acg gtg tcg tgg aac tca ggc gcc ctg acc     864
   Tyr Phe Pro Glu Pro Val Thr Val Ser Trp Asn Ser Gly Ala Leu Thr
           275                 280                 285
   agc ggc gtg cac acc ttc ccg gct gtc cta cag tcc tca gga ctc tac     912
   Ser Gly Val His Thr Phe Pro Ala Val Leu Gln Ser Ser Gly Leu Tyr
       290                 295                 300
   tcc ctc agc agc gtg gtg acc gtg ccc tcc agc agc ttg ggc acc cag     960
   Ser Leu Ser Ser Val Val Thr Val Pro Ser Ser Ser Leu Gly Thr Gln
   305                 310                 315                 320
   acc tac atc tgc aac gtg aat cac aag ccc agc aac acc aag gtg gac    1008
   Thr Tyr Ile Cys Asn Val Asn His Lys Pro Ser Asn Thr Lys Val Asp
                   325                 330                 335
   aag aaa gtt gag ccc aaa tct tgt gac aaa act cac aca tgc cca ccg    1056
   Lys Lys Val Glu Pro Lys Ser Cys Asp Lys Thr His Thr Cys Pro Pro
               340                 345                 350
   tgc cca gca cct gaa ctc ctg ggg gga ccg tca gtc ttc ctc ttc ccc    1104
   Cys Pro Ala Pro Glu Leu Leu Gly Gly Pro Ser Val Phe Leu Phe Pro
           355                 360                 365
   cca aaa ccc aag gac acc ctc atg atc tcc cgg acc cct gag gtc aca    1152
   Pro Lys Pro Lys Asp Thr Leu Met Ile Ser Arg Thr Pro Glu Val Thr
       370                 375                 380
   tgc gtg gtg gtg gac gtg agc cac gaa gac cct gag gtc aag ttc aac    1200
   Cys Val Val Val Asp Val Ser His Glu Asp Pro Glu Val Lys Phe Asn
   385                 390                 395                 400
   tgg tac gtg gac ggc gtg gag gtg cat aat gcc aag aca aag ccg cgg    1248
   Trp Tyr Val Asp Gly Val Glu Val His Asn Ala Lys Thr Lys Pro Arg
                   405                 410                 415
   gag gag cag tac aac agc acg tac cgt gtg gtc agc gtc ctc acc gtc    1296
   Glu Glu Gln Tyr Asn Ser Thr Tyr Arg Val Val Ser Val Leu Thr Val
               420                 425                 430
   ctg cac cag gac tgg ctg aat ggc aag gag tac aag tgc aag gtc tcc     1344
   Leu His Gln Asp Trp Leu Asn Gly Lys Glu Tyr Lys Cys Lys Val Ser
           435                 440                 445
   aac aaa gcc ctc cca gcc ccc atc gag aaa acc atc tcc aaa gcc aaa     1392
   Asn Lys Ala Leu Pro Ala Pro Ile Glu Lys Thr Ile Ser Lys Ala Lys
       450                 455                 460
   ggg cag ccc cga gaa cca cag gtg tac acc ctg ccc cca tcc cgg gat     1440
   Gly Gln Pro Arg Glu Pro Gln Val Tyr Thr Leu Pro Pro Ser Arg Asp
   465                 470                 475                 480
   gag ctg acc aag aac cag gtc agc ctg acc tgc ctg gtc aaa ggc ttc     1488
   Glu Leu Thr Lys Asn Gln Val Ser Leu Thr Cys Leu Val Lys Gly Phe
                   485                 490                 495
   tat ccc agc gac atc gcc gtg gag tgg gag agc aat ggg cag ccg gag     1536
   Tyr Pro Ser Asp Ile Ala Val Glu Trp Glu Ser Asn Gly Gln Pro Glu
               500                 505                 510
   aac aac tac aag acc acg cct ccc gtg ctg gac tcc gac ggc tcc ttc     1584
   Asn Asn Tyr Lys Thr Thr Pro Pro Val Leu Asp Ser Asp Gly Ser Phe
           515                 520                 525
   ttc ctc tac agc aag ctc acc gtg gac aag agc agg tgg cag cag ggg     1632
   Phe Leu Tyr Ser Lys Leu Thr Val Asp Lys Ser Arg Trp Gln Gln Gly
       530                 535                 540
   aac gtc ttc tca tgc tcc gtg atg cat gag gct ctg cac aac cac tac     1680
   Asn Val Phe Ser Cys Ser Val Met His Glu Ala Leu His Asn His Tyr
   545                 550                 555                 560
   acg cag aag agc ctc tcc ctg tct ccg ggt aaa tgacgcg                 1720
   Thr Gln Lys Ser Leu Ser Leu Ser Pro Gly Lys
                   565                 570
<210> 53
   <211> 571
   <212> PRT
   <213> Homo sapiens
<400> 53
<210> 54
   <211> 547
   <212> PRT
   <213> Homo sapiens
<400> 54
   Val Pro Cys Val Ser Gly Gly Leu Pro Lys Pro Ala Asn Ile Thr Phe
   1               5                   10                  15
   Leu Ser Ile Asn Met Lys Asn Val Leu Gln Trp Thr Pro Pro Glu Gly
               20                  25                  30
   Leu Gln Gly Val Lys Val Thr Tyr Thr Val Gln Tyr Phe Ile Tyr Gly
           35                  40                  45
   Gln Lys Lys Trp Leu Asn Lys Ser Glu Cys Arg Asn Ile Asn Arg Thr
   50 55 60
   Tyr Cys Asp Leu Ser Ala Glu Thr Ser Asp Tyr Glu His Gln Tyr Tyr
   65 70 75 80
   Ala Lys Val Lys Ala Ile Trp Gly Thr Lys Cys Ser Lys Trp Ala Glu
   85 90 95
   Ser Gly Arg Phe Tyr Pro Phe Leu Glu Thr Gln Ile Gly Pro Pro Glu
   100 105 110
   Val Ala Leu Thr Thr Asp Glu Lys Ser Ile Ser Val Val Leu Thr Ala
   115 120 125
   Pro Glu Lys Trp Lys Arg Asn Pro Glu Asp Leu Pro Val Ser Met Gln
   130 135 140
   Gln Ile Tyr Ser Asn Leu Lys Tyr Asn Val Ser Val Leu Asn Thr Lys
   145 150 155 160
   Ser Asn Arg Thr Trp Ser Gln Cys Val Thr Asn His Thr Leu Val Leu
   165 170 175
   Thr Trp Leu Glu Pro Asn Thr Leu Tyr Cys Val His Val Glu Ser Phe
   180 185 190
   Val Pro Gly Pro Pro Arg Arg Ala Gln Pro Ser Glu Lys Gln Cys Ala
   195 200 205
   Arg Thr Leu Lys Asp Gln Ser Ser Glu Ala Ser Thr Lys Gly Pro Ser
   210 215 220
   Val Phe Pro Leu Ala Pro Ser Ser Lys Ser Thr Ser Gly Gly Thr Ala
   225 230 235 240
   Ala Leu Gly Cys Leu Val Lys Asp Tyr Phe Pro Glu Pro Val Thr Val
   245 250 255
   Ser Trp Asn Ser Gly Ala Leu Thr Ser Gly Val His Thr Phe Pro Ala
   260 265 270
   Val Leu Gln Ser Ser Gly Leu Tyr Ser Leu Ser Ser Val Val Thr Val
   275 280 285
   Pro Ser Ser Ser Leu Gly Thr Gln Thr Tyr Ile Cys Asn Val Asn His
   290 295 300
   Lys Pro Ser Asn Thr Lys Val Asp Lys Lys Val Glu Pro Lys Ser Cys
   305 310 315 320
   Asp Lys Thr His Thr Cys Pro Pro Cys Pro Ala Pro Glu Leu Leu Gly
   325 330 335
   Gly Pro Ser Val Phe Leu Phe Pro Pro Lys Pro Lys Asp Thr Leu Met
   340 345 350
   Ile Ser Arg Thr Pro Glu Val Thr Cys Val Val Val Asp Val Ser His
   355 360 365
   Glu Asp Pro Glu Val Lys Phe Asn Trp Tyr Val Asp Gly Val Glu Val
   370 375 380
   His Asn Ala Lys Thr Lys Pro Arg Glu Glu Gln Tyr Asn Ser Thr Tyr
   385 390 395 400
   Arg Val Val Ser Val Leu Thr Val Leu His Gln Asp Trp Leu Asn Gly
   405 410 415
   Lys Glu Tyr Lys Cys Lys Val Ser Asn Lys Ala Leu Pro Ala Pro Ile
   420 425 430
   Glu Lys Thr Ile Ser Lys Ala Lys Gly Gln Pro Arg Glu Pro Gln Val
   435 440 445
   Tyr Thr Leu Pro Pro Ser Arg Asp Glu Leu Thr Lys Asn Gln Val Ser
   450 455 460
   Leu Thr Cys Leu Val Lys Gly Phe Tyr Pro Ser Asp Ile Ala Val Glu
   465 470 475 480
   Trp Glu Ser Asn Gly Gln Pro Glu Asn Asn Tyr Lys Thr Thr Pro Pro
   485 490 495
   Val Leu Asp Ser Asp Gly Ser Phe Phe Leu Tyr Ser Lys Leu Thr Val
   500 505 510
   Asp Lys Ser Arg Trp Gln Gln Gly Asn Val Phe Ser Cys Ser Val Met
   515 520 525
   His Glu Ala Leu His Asn His Tyr Thr Gln Lys Ser Leu Ser Leu Ser
   530 535 540
   Pro Gly Lys
   545
<210> 55
   <211> 217
   <212> PRT
   <213> Homo sapiens
<400> 55
   Val Pro Cys Val Ser Gly Gly Leu Pro Lys Pro Ala Asn Ile Thr Phe
   1 5 10 15
   Leu Ser Ile Asn Met Lys Asn Val Leu Gln Trp Thr Pro Pro Glu Gly
   20 25 30
   Leu Gln Gly Val Lys Val Thr Tyr Thr Val Gln Tyr Phe Ile Tyr Gly
   35 40 45
   Gln Lys Lys Trp Leu Asn Lys Ser Glu Cys Arg Asn Ile Asn Arg Thr
   50 55 60
   Tyr Cys Asp Leu Ser Ala Glu Thr Ser Asp Tyr Glu His Gln Tyr Tyr
   65 70 75 80
   Ala Lys Val Lys Ala Ile Trp Gly Thr Lys Cys Ser Lys Trp Ala Glu
   85 90 95
   Ser Gly Arg Phe Tyr Pro Phe Leu Glu Thr Gln Ile Gly Pro Pro Glu
   100 105 110
   Val Ala Leu Thr Thr Asp Glu Lys Ser Ile Ser Val Val Leu Thr Ala
           115                 120                 125
   Pro Glu Lys Trp Lys Arg Asn Pro Glu Asp Leu Pro Val Ser Met Gln
       130                 135                 140
   Gln Ile Tyr Ser Asn Leu Lys Tyr Asn Val Ser Val Leu Asn Thr Lys
   145                 150                 155                 160
   Ser Asn Arg Thr Trp Ser Gln Cys Val Thr Asn His Thr Leu Val Leu
                   165                 170                 175
   Thr Trp Leu Glu Pro Asn Thr Leu Tyr Cys Val His Val Glu Ser Phe
               180                 185                 190
   Val Pro Gly Pro Pro Arg Arg Ala Gln Pro Ser Glu Lys Gln Cys Ala
           195                 200                 205
   Arg Thr Leu Lys Asp Gln Ser Ser Glu
       210                 215
<210> 56
   <211> 1011
   <212> DNA
   <213> Homo sapiens
<220>
   <221> CDS
   <222> (1)...(1008)
<400> 56
   atg cag act ttc aca atg gtt cta gaa gaa atc tgg aca agt ctt ttc      48
   Met Gln Thr Phe Thr Met Val Leu Glu Glu Ile Trp Thr Ser Leu Phe
    1               5                  10                  15
   atg tgg ttt ttc tac gca ttg att cca tgt ttg ctc aca gat gaa gtg      96
   Met Trp Phe Phe Tyr Ala Leu Ile Pro Cys Leu Leu Thr Asp Glu Val
               20                  25                  30
   gcc att ctg cct gcc cct cag aac ctc tct gta ctc tca acc aac atg     144
   Ala Ile Leu Pro Ala Pro Gln Asn Leu Ser Val Leu Ser Thr Asn Met
           35                  40                  45
   aag cat ctc ttg atg tgg agc cca gtg atc gcg cct gga gaa aca gtg     192
   Lys His Leu Leu Met Trp Ser Pro Val Ile Ala Pro Gly Glu Thr Val
       50                  55                  60
   tac tat tct gtc gaa tac cag ggg gag tac gag agc ctg tac acg agc     240
   Tyr Tyr Ser Val Glu Tyr Gln Gly Glu Tyr Glu Ser Leu Tyr Thr Ser
    65                  70                  75                  80
   cac atc tgg atc ccc agc agc tgg tgc tca ctc act gaa ggt cct gag      288
   His Ile Trp Ile Pro Ser Ser Trp Cys Ser Leu Thr Glu Gly Pro Glu
                    85                  90                  95
   tgt gat gtc act gat gac atc acg gcc act gtg cca tac aac ctt cgt      336
   Cys Asp Val Thr Asp Asp Ile Thr Ala Thr Val Pro Tyr Asn Leu Arg
               100                 105                 110
   gtc agg gcc aca ttg ggc tca cag acc tca gcc tgg agc atc ctg aag      384
   Val Arg Ala Thr Leu Gly Ser Gln Thr Ser Ala Trp Ser Ile Leu Lys
           115                 120                 125
   cat ccc ttt aat aga aac tca acc atc ctt acc cga cct ggg atg gag      432
   His Pro Phe Asn Arg Asn Ser Thr Ile Leu Thr Arg Pro Gly Met Glu
       130                 135                 140
   atc acc aaa gat ggc ttc cac ctg gtt att gag ctg gag gac ctg ggg      480
   Ile Thr Lys Asp Gly Phe His Leu Val Ile Glu Leu Glu Asp Leu Gly
   145                 150                 155                 160
   ccc cag ttt gag ttc ctt gtg gcc tac tgg agg agg gag cct ggt gcc      528
   Pro Gln Phe Glu Phe Leu Val Ala Tyr Trp Arg Arg Glu Pro Gly Ala
                   165                 170                 175
   gag gaa cat gtc aaa atg gtg agg agt ggg ggt att cca gtg cac cta      576
   Glu Glu His Val Lys Met Val Arg Ser Gly Gly Ile Pro Val His Leu
               180                 185                 190
   gaa acc atg gag cca ggg gct gca tac tgt gtg aag gcc cag aca ttc      624
   Glu Thr Met Glu Pro Gly Ala Ala Tyr Cys Val Lys Ala Gln Thr Phe
           195                 200                 205
   gtg aag gcc att ggg agg tac agc gcc ttc agc cag aca gaa tgt gtg      672
   Val Lys Ala Ile Gly Arg Tyr Ser Ala Phe Ser Gln Thr Glu Cys Val
       210                 215                 220
   gag gtg caa gga gag gcc act gtg gct gca cca tct gtc ttc atc ttc      720
   Glu Val Gln Gly Glu Ala Thr Val Ala Ala Pro Ser Val Phe Ile Phe
   225                 230                 235                 240
   ccg cca tct gat gag cag ttg aaa tct ggt acc gcc tct gtt gtg tgc      768
   Pro Pro Ser Asp Glu Gln Leu Lys Ser Gly Thr Ala Ser Val Val Cys
                   245                 250                 255
   ctg ctg aat aac ttc tat ccc aga gag gcc aaa gta cag tgg aag gtg      816
   Leu Leu Asn Asn Phe Tyr Pro Arg Glu Ala Lys Val Gln Trp Lys Val
               260                 265                 270
   gat aac gcc ctc caa tcg ggt aac tcc cag gag agt gtc aca gag cag      864
   Asp Asn Ala Leu Gln Ser Gly Asn Ser Gln Glu Ser Val Thr Glu Gln
           275                 280                 285
   gac agc aag gac agc acc tac agc ctc agc agc acc ctg acg ctg agc      912
   Asp Ser Lys Asp Ser Thr Tyr Ser Leu Ser Ser Thr Leu Thr Leu Ser
       290                 295                 300
   aaa gca gac tac gag aaa cac aaa gtc tac gcc tgc gaa gtc acc cat      960
   Lys Ala Asp Tyr Glu Lys His Lys Val Tyr Ala Cys Glu Val Thr His
   305                 310                 315                 320
   cag ggc ctg agc tcg ccc gtc aca aag agc ttc aac agg gga gag tgt     1008
   Gln Gly Leu Ser Ser Pro Val Thr Lys Ser Phe Asn Arg Gly Glu Cys
                   325                 330                 335
   tag                                                                 1011
<210> 57
   <211> 336
   <212> PRT
   <213> Homo sapiens
<400> 57
   Met Gln Thr Phe Thr Met Val Leu Glu Glu Ile Trp Thr Ser Leu Phe
    1               5                  10                  15
   Met Trp Phe Phe Tyr Ala Leu Ile Pro Cys Leu Leu Thr Asp Glu Val
               20                  25                  30
   Ala Ile Leu Pro Ala Pro Gln Asn Leu Ser Val Leu Ser Thr Asn Met
           35                  40                  45
   Lys His Leu Leu Met Trp Ser Pro Val Ile Ala Pro Gly Glu Thr Val
       50                  55                  60
   Tyr Tyr Ser Val Glu Tyr Gln Gly Glu Tyr Glu Ser Leu Tyr Thr Ser
   65                  70                  75                  80
   His Ile Trp Ile Pro Ser Ser Trp Cys Ser Leu Thr Glu Gly Pro Glu
                   85                  90                  95
   Cys Asp Val Thr Asp Asp Ile Thr Ala Thr Val Pro Tyr Asn Leu Arg
               100                 105                 110
   Val Arg Ala Thr Leu Gly Ser Gln Thr Ser Ala Trp Ser Ile Leu Lys
           115                 120                 125
   His Pro Phe Asn Arg Asn Ser Thr Ile Leu Thr Arg Pro Gly Met Glu
       130                 135                 140
   Ile Thr Lys Asp Gly Phe His Leu Val Ile Glu Leu Glu Asp Leu Gly
   145                 150                 155                 160
   Pro Gln Phe Glu Phe Leu Val Ala Tyr Trp Arg Arg Glu Pro Gly Ala
                   165                 170                 175
   Glu Glu His Val Lys Met Val Arg Ser Gly Gly Ile Pro Val His Leu
               180                 185                 190
   Glu Thr Met Glu Pro Gly Ala Ala Tyr Cys Val Lys Ala Gln Thr Phe
           195                 200                 205
   Val Lys Ala Ile Gly Arg Tyr Ser Ala Phe Ser Gln Thr Glu Cys Val
       210                 215                 220
   Glu Val Gln Gly Glu Ala Thr Val Ala Ala Pro Ser Val Phe Ile Phe
   225                 230                 235                 240
   Pro Pro Ser Asp Glu Gln Leu Lys Ser Gly Thr Ala Ser Val Val Cys
                   245                 250                 255
   Leu Leu Asn Asn Phe Tyr Pro Arg Glu Ala Lys Val Gln Trp Lys Val
               260                 265                 270
   Asp Asn Ala Leu Gln Ser Gly Asn Ser Gln Glu Ser Val Thr Glu Gln
           275                 280                 285
   Asp Ser Lys Asp Ser Thr Tyr Ser Leu Ser Ser Thr Leu Thr Leu Ser
       290                 295                 300
   Lys Ala Asp Tyr Glu Lys His Lys Val Tyr Ala Cys Glu Val Thr His
   305                 310                 315                 320
   Gln Gly Leu Ser Ser Pro Val Thr Lys Ser Phe Asn Arg Gly Glu Cys
                   325                 330                 335
<210> 58
   <211> 307
   <212> PRT
   <213> Homo sapiens
<400> 58
   Asp Glu Val Ala Ile Leu Pro Ala Pro Gln Asn Leu Ser Val Leu Ser
   1 5 10 15
   Thr Asn Met Lys His Leu Leu Met Trp Ser Pro Val Ile Ala Pro Gly
   20 25 30
   Glu Thr Val Tyr Tyr Ser Val Glu Tyr Gln Gly Glu Tyr Glu Ser Leu
   35 40 45
   Tyr Thr Ser His Ile Trp Ile Pro Ser Ser Trp Cys Ser Leu Thr Glu
   50 55 60
   Gly Pro Glu Cys Asp Val Thr Asp Asp Ile Thr Ala Thr Val Pro Tyr
   65 70 75 80
   Asn Leu Arg Val Arg Ala Thr Leu Gly Ser Gln Thr Ser Ala Trp Ser
   85 90 95
   Ile Leu Lys His Pro Phe Asn Arg Asn Ser Thr Ile Leu Thr Arg Pro
   100 105 110
   Gly Met Glu Ile Thr Lys Asp Gly Phe His Leu Val Ile Glu Leu Glu
   115 120 125
   Asp Leu Gly Pro Gln Phe Glu Phe Leu Val Ala Tyr Trp Arg Arg Glu
   130 135 140
   Pro Gly Ala Glu Glu His Val Lys Met Val Arg Ser Gly Gly Ile Pro
   145 150 155 160
   Val His Leu Glu Thr Met Glu Pro Gly Ala Ala Tyr Cys Val Lys Ala
   165 170 175
   Gln Thr Phe Val Lys Ala Ile Gly Arg Tyr Ser Ala Phe Ser Gln Thr
   180 185 190
   Glu Cys Val Glu Val Gln Gly Glu Ala Thr Val Ala Ala Pro Ser Val
   195 200 205
   Phe Ile Phe Pro Pro Ser Asp Glu Gln Leu Lys Ser Gly Thr Ala Ser
   210 215 220
   Val Val Cys Leu Leu Asn Asn Phe Tyr Pro Arg Glu Ala Lys Val Gln
   225 230 235 240
   Trp Lys Val Asp Asn Ala Leu Gln Ser Gly Asn Ser Gln Glu Ser Val
   245 250 255
   Thr Glu Gln Asp Ser Lys Asp Ser Thr Tyr Ser Leu Ser Ser Thr Leu
   260 265 270
   Thr Leu Ser Lys Ala Asp Tyr Glu Lys His Lys Val Tyr Ala Cys Glu
   275 280 285
   Val Thr His Gln Gly Leu Ser Ser Pro Val Thr Lys Ser Phe Asn Arg
   290 295 300
   Gly Glu Cys
   305
<210> 59
   <211> 201
   <212> PRT
   <213> Homo sapiens
<400> 59
   Asp Glu Val Ala Ile Leu Pro Ala Pro Gln Asn Leu Ser Val Leu Ser
    1               5                  10                  15
   Thr Asn Met Lys His Leu Leu Met Trp Ser Pro Val Ile Ala Pro Gly
               20                  25                  30
   Glu Thr Val Tyr Tyr Ser Val Glu Tyr Gln Gly Glu Tyr Glu Ser Leu
           35                  40                  45
   Tyr Thr Ser His Ile Trp Ile Pro Ser Ser Trp Cys Ser Leu Thr Glu
       50                  55                  60
   Gly Pro Glu Cys Asp Val Thr Asp Asp Ile Thr Ala Thr Val Pro Tyr
   65                  70                  75                  80
   Asn Leu Arg Val Arg Ala Thr Leu Gly Ser Gln Thr Ser Ala Trp Ser
                   85                  90                  95
   Ile Leu Lys His Pro Phe Asn Arg Asn Ser Thr Ile Leu Thr Arg Pro
               100                 105                 110
   Gly Met Glu Ile Thr Lys Asp Gly Phe His Leu Val Ile Glu Leu Glu
           115                 120                 125
   Asp Leu Gly Pro Gln Phe Glu Phe Leu Val Ala Tyr Trp Arg Arg Glu
       130                 135                 140
   Pro Gly Ala Glu Glu His Val Lys Met Val Arg Ser Gly Gly Ile Pro
   145                 150                 155                 160
   Val His Leu Glu Thr Met Glu Pro Gly Ala Ala Tyr Cys Val Lys Ala
                   165                 170                 175
   Gln Thr Phe Val Lys Ala Ile Gly Arg Tyr Ser Ala Phe Ser Gln Thr
               180                 185                 190
   Glu Cys Val Glu Val Gln Gly Glu Ala
           195                 200
<210> 60
   <211> 323
   <212> PRT
   <213> Homo sapiens
<400> 60
   Asp Glu Val Ala Ile Leu Pro Ala Pro Gln Asn Leu Ser Val Leu Ser
   1 5 10 15
   Thr Asn Met Lys His Leu Leu Met Trp Ser Pro Val Ile Ala Pro Gly
   20 25 30
   Glu Thr Val Tyr Tyr Ser Val Glu Tyr Gln Gly Glu Tyr Glu Ser Leu
   35 40 45
   Tyr Thr Ser His Ile Trp Ile Pro Ser Ser Trp Cys Ser Leu Thr Glu
   50 55 60
   Gly Pro Glu Cys Asp Val Thr Asp Asp Ile Thr Ala Thr Val Pro Tyr
   65 70 75 80
   Asn Leu Arg Val Arg Ala Thr Leu Gly Ser Gln Thr Ser Ala Trp Ser
   85 90 95
   Ile Leu Lys His Pro Phe Asn Arg Asn Ser Thr Ile Leu Thr Arg Pro
   100 105 110
   Gly Met Glu Ile Pro Lys His Gly Phe His Leu Val Ile Glu Leu Glu
   115 120 125
   Asp Leu Gly Pro Gln Phe Glu Phe Leu Val Ala Tyr Trp Thr Arg Glu
   130 135 140
   Pro Gly Ala Glu Glu His Val Lys Met Val Arg Ser Gly Gly Ile Pro
   145 150 155 160
   Val His Leu Glu Thr Met Glu Pro Gly Ala Ala Tyr Cys Val Lys Ala
   165 170 175
   Gln Thr Phe Val Lys Ala Ile Gly Arg Tyr Ser Ala Phe Ser Gln Thr
   180 185 190
   Glu Cys Val Glu Val Gln Gly Glu Ala Gly Gly Gly Gly Ser Gly Gly
   195 200 205
   Gly Gly Ser Gly Gly Gly Gly Ser Arg Thr Val Ala Ala Pro Ser Val
   210 215 220
   Phe Ile Phe Pro Pro Ser Asp Glu Gln Leu Lys Ser Gly Thr Ala Ser
   225 230 235 240
   Val Val Cys Leu Leu Asn Asn Phe Tyr Pro Arg Glu Ala Lys Val Gln
   245 250 255
   Trp Lys Val Asp Asn Ala Leu Gln Ser Gly Asn Ser Gln Glu Ser Val
   260 265 270
   Thr Glu Gln Asp Ser Lys Asp Ser Thr Tyr Ser Leu Ser Ser Thr Leu
   275 280 285
   Thr Leu Ser Lys Ala Asp Tyr Glu Lys His Lys Val Tyr Ala Cys Glu
   290 295 300
   Val Thr His Gln Gly Leu Ser Ser Pro Val Thr Lys Ser Phe Asn Arg
   305 310 315 320
   Gly Glu Cys
<210> 61
   <211> 201
   <212> PRT
   <213> Homo sapiens
<400> 61
   Asp Glu Val Ala Ile Leu Pro Ala Pro Gln Asn Leu Ser Val Leu Ser
   1 5 10 15
   Thr Asn Met Lys His Leu Leu Met Trp Ser Pro Val Ile Ala Pro Gly
   20 25 30
   Glu Thr Val Tyr Tyr Ser Val Glu Tyr Gln Gly Glu Tyr Glu Ser Leu
   35 40 45
   Tyr Thr Ser His Ile Trp Ile Pro Ser Ser Trp Cys Ser Leu Thr Glu
   50 55 60
   Gly Pro Glu Cys Asp Val Thr Asp Asp Ile Thr Ala Thr Val Pro Tyr
   65 70 75 80
   Asn Leu Arg Val Arg Ala Thr Leu Gly Ser Gln Thr Ser Ala Trp Ser
   85 90 95
   Ile Leu Lys His Pro Phe Asn Arg Asn Ser Thr Ile Leu Thr Arg Pro
   100 105 110
   Gly Met Glu Ile Pro Lys His Gly Phe His Leu Val Ile Glu Leu Glu
   115 120 125
   Asp Leu Gly Pro Gln Phe Glu Phe Leu Val Ala Tyr Trp Thr Arg Glu
   130 135 140
   Pro Gly Ala Glu Glu His Val Lys Met Val Arg Ser Gly Gly Ile Pro
   145 150 155 160
   Val His Leu Glu Thr Met Glu Pro Gly Ala Ala Tyr Cys Val Lys Ala
   165 170 175
   Gln Thr Phe Val Lys Ala Ile Gly Arg Tyr Ser Ala Phe Ser Gln Thr
   180 185 190
   Glu Cys Val Glu Val Gln Gly Glu Ala
   195 200
<210> 62
   <211> 559
   <212> PRT
   <213> Homo sapiens
<400> 62
   Val Pro Cys Val Ser Gly Gly Leu Pro Lys Pro Ala Asn Ile Thr Phe
    1               5                  10                  15
   Leu Ser Ile Asn Met Lys Asn Val Leu Gln Trp Thr Pro Pro Glu Gly
               20                  25                  30
   Leu Gln Gly Val Lys Val Thr Tyr Thr Val Gln Tyr Phe Ile Tyr Gly
           35                  40                  45
   Gln Lys Lys Trp Leu Asn Lys Ser Glu Cys Arg Asn Ile Asn Arg Thr
       50                  55                  60
   Tyr Cys Asp Leu Ser Ala Glu Thr Ser Asp Tyr Glu His Gln Tyr Tyr
   65                  70                  75                  80
   Ala Lys Val Lys Ala Ile Trp Gly Thr Lys Cys Ser Lys Trp Ala Glu
                   85                  90                  95
   Ser Gly Arg Phe Tyr Pro Phe Leu Glu Thr Gln Ile Gly Pro Pro Glu
               100                 105                 110
   Val Ala Leu Thr Thr Asp Glu Lys Ser Ile Ser Val Val Leu Thr Ala
   115 120 125
   Pro Glu Lys Trp Lys Arg Asn Pro Glu Asp Leu Pro Val Ser Met Gln
   130 135 140
   Gln Ile Tyr Ser Asn Leu Lys Tyr Asn Val Ser Val Leu Asn Thr Lys
   145 150 155 160
   Ser Asn Arg Thr Trp Ser Gln Cys Val Thr Asn His Thr Leu Val Leu
   165 170 175
   Thr Trp Leu Glu Pro Asn Thr Leu Tyr Cys Val His Val Glu Ser Phe
   180 185 190
   Val Pro Gly Pro Pro Arg Arg Ala Gln Pro Ser Glu Lys Gln Cys Ala
   195 200 205
   Arg Thr Leu Lys Asp Gln Gly Gly Gly Gly Ser Gly Gly Gly Gly Ser
   210 215 220
   Gly Gly Gly Gly Ser Ala Ser Thr Lys Gly Pro Ser Val Phe Pro Leu
   225 230 235 240
   Ala Pro Ser Ser Lys Ser Thr Ser Gly Gly Thr Ala Ala Leu Gly Cys
   245 250 255
   Leu Val Lys Asp Tyr Phe Pro Glu Pro Val Thr Val Ser Trp Asn Ser
   260 265 270
   Gly Ala Leu Thr Ser Gly Val His Thr Phe Pro Ala Val Leu Gln Ser
   275 280 285
   Ser Gly Leu Tyr Ser Leu Ser Ser Val Val Thr Val Pro Ser Ser Ser
   290 295 300
   Leu Gly Thr Gln Thr Tyr Ile Cys Asn Val Asn His Lys Pro Ser Asn
   305 310 315 320
   Thr Lys Val Asp Lys Lys Val Glu Pro Lys Ser Cys Asp Lys Thr His
   325 330 335
   Thr Cys Pro Pro Cys Pro Ala Pro Glu Ala Glu Gly Ala Pro Ser Val
   340 345 350
   Phe Leu Phe Pro Pro Lys Pro Lys Asp Thr Leu Met Ile Ser Arg Thr
   355 360 365
   Pro Glu Val Thr Cys Val Val Val Asp Val Ser His Glu Asp Pro Glu
   370 375 380
   Val Lys Phe Asn Trp Tyr Val Asp Gly Val Glu Val His Asn Ala Lys
   385 390 395 400
   Thr Lys Pro Arg Glu Glu Gln Tyr Asn Ser Thr Tyr Arg Val Val Ser
   405 410 415
   Val Leu Thr Val Leu His Gln Asp Trp Leu Asn Gly Lys Glu Tyr Lys
   420 425 430
   Cys Lys Val Ser Asn Lys Ala Leu Pro Ser Ser Ile Glu Lys Thr Ile
   435 440 445
   Ser Lys Ala Lys Gly Gln Pro Arg Glu Pro Gln Val Tyr Thr Leu Pro
   450 455 460
   Pro Ser Arg Asp Glu Leu Thr Lys Asn Gln Val Ser Leu Thr Cys Leu
   465 470 475 480
   Val Lys Gly Phe Tyr Pro Ser Asp Ile Ala Val Glu Trp Glu Ser Asn
   485 490 495
   Gly Gln Pro Glu Asn Asn Tyr Lys Thr Thr Pro Pro Val Leu Asp Ser
   500 505 510
   Asp Gly Ser Phe Phe Leu Tyr Ser Lys Leu Thr Val Asp Lys Ser Arg
   515 520 525
   Trp Gln Gln Gly Asn Val Phe Ser Cys Ser Val Met His Glu Ala Leu
   530 535 540
   His Asn His Tyr Thr Gln Lys Ser Leu Ser Leu Ser Pro Gly Lys
   545 550 555
<210> 63
   <211> 214
   <212> PRT
   <213> Homo sapiens
<400> 63
   Val Pro Cys Val Ser Gly Gly Leu Pro Lys Pro Ala Asn Ile Thr Phe
   1 5 10 15
   Leu Ser Ile Asn Met Lys Asn Val Leu Gln Trp Thr Pro Pro Glu Gly
   20 25 30
   Leu Gln Gly Val Lys Val Thr Tyr Thr Val Gln Tyr Phe Ile Tyr Gly
   35 40 45
   Gln Lys Lys Trp Leu Asn Lys Ser Glu Cys Arg Asn Ile Asn Arg Thr
   50 55 60
   Tyr Cys Asp Leu Ser Ala Glu Thr Ser Asp Tyr Glu His Gln Tyr Tyr
   65 70 75 80
   Ala Lys Val Lys Ala Ile Trp Gly Thr Lys Cys Ser Lys Trp Ala Glu
   85 90 95
   Ser Gly Arg Phe Tyr Pro Phe Leu Glu Thr Gln Ile Gly Pro Pro Glu
   100 105 110
   Val Ala Leu Thr Thr Asp Glu Lys Ser Ile Ser Val Val Leu Thr Ala
   115 120 125
   Pro Glu Lys Trp Lys Arg Asn Pro Glu Asp Leu Pro Val Ser Met Gln
   130 135 140
   Gln Ile Tyr Ser Asn Leu Lys Tyr Asn Val Ser Val Leu Asn Thr Lys
   145 150 155 160
   Ser Asn Arg Thr Trp Ser Gln Cys Val Thr Asn His Thr Leu Val Leu
   165 170 175
   Thr Trp Leu Glu Pro Asn Thr Leu Tyr Cys Val His Val Glu Ser Phe
   180 185 190
   Val Pro Gly Pro Pro Arg Arg Ala Gln Pro Ser Glu Lys Gln Cys Ala
   195 200 205
   Arg Thr Leu Lys Asp Gln
   210
<210> 64
   <211> 19
   <212> PRT
   <213> Homo sapiens
<400> 64
   Glu Glu Ile His Ala Glu Leu Arg Arg Phe Arg Arg Val Pro Cys Val
   1 5 10 15
   Ser Gly Gly
<210> 65
   <211> 207
   <212> PRT
   <213> Homo sapiens
<400> 65
   Leu Pro Lys Pro Ala Asn Ile Thr Phe Leu Ser Ile Asn Met Lys Asn
    1               5                  10                  15
   Val Leu Gln Trp Thr Pro Pro Glu Gly Leu Gln Gly Val Lys Val Thr
               20                  25                  30
   Tyr Thr Val Gln Tyr Phe Ile Tyr Gly Gln Lys Lys Trp Leu Asn Lys
           35                  40                  45
   Ser Glu Cys Arg Asn Ile Asn Arg Thr Tyr Cys Asp Leu Ser Ala Glu
       50                  55                  60
   Thr Ser Asp Tyr Glu His Gln Tyr Tyr Ala Lys Val Lys Ala Ile Trp
   65                  70                  75                  80
   Gly Thr Lys Cys Ser Lys Trp Ala Glu Ser Gly Arg Phe Tyr Pro Phe
                   85                  90                  95
   Leu Glu Thr Gln Ile Gly Pro Pro Glu Val Ala Leu Thr Thr Asp Glu
               100                 105                 110
   Lys Ser Ile Ser Val Val Leu Thr Ala Pro Glu Lys Trp Lys Arg Asn
           115                 120                 125
   Pro Glu Asp Leu Pro Val Ser Met Gln Gln Ile Tyr Ser Asn Leu Lys
       130                 135                 140
   Tyr Asn Val Ser Val Leu Asn Thr Lys Ser Asn Arg Thr Trp Ser Gln
   145                 150                 155                 160
   Cys Val Thr Asn His Thr Leu Val Leu Thr Trp Leu Glu Pro Asn Thr
                   165                 170                 175
   Leu Tyr Cys Val His Val Glu Ser Phe Val Pro Gly Pro Pro Arg Arg
               180                 185                 190
   Ala Gln Pro Ser Glu Lys Gln Cys Ala Arg Thr Leu Lys Asp Gln
           195                 200                 205
<210> 66
   <211> 150
   <212> PRT
   <213> Homo sapiens
<400> 66
   Cys Arg Asn Ile Asn Arg Thr Tyr Cys Asp Leu Ser Ala Glu Thr Ser
   1               5                   10                  15
   Asp Tyr Glu His Gln Tyr Tyr Ala Lys Val Lys Ala Ile Trp Gly Thr
               20                  25                  30
   Lys Cys Ser Lys Trp Ala Glu Ser Gly Arg Phe Tyr Pro Phe Leu Glu
           35                  40                  45
   Thr Gln Ile Gly Pro Pro Glu Val Ala Leu Thr Thr Asp Glu Lys Ser
       50                  55                  60
   Ile Ser Val Val Leu Thr Ala Pro Glu Lys Trp Lys Arg Asn Pro Glu
   65                  70                  75                  80
   Asp Leu Pro Val Ser Met Gln Gln Ile Tyr Ser Asn Leu Lys Tyr Asn
                   85                  90                  95
   Val Ser Val Leu Asn Thr Lys Ser Asn Arg Thr Trp Ser Gln Cys Val
               100                 105                 110
   Thr Asn His Thr Leu Val Leu Thr Trp Leu Glu Pro Asn Thr Leu Tyr
           115                 120                 125
   Cys Val His Val Glu Ser Phe Val Pro Gly Pro Pro Arg Arg Ala Gln
       130                 135                 140
   Pro Ser Glu Lys Gln Cys
   145                 150
<210> 67
   <211> 196
   <212> PRT
   <213> Homo sapiens
<400> 67
   Leu Pro Ala Pro Gln Asn Leu Ser Val Leu Ser Thr Asn Met Lys His
   1               5                   10                  15
   Leu Leu Met Trp Ser Pro Val Ile Ala Pro Gly Glu Thr Val Tyr Tyr
   20 25 30
   Ser Val Glu Tyr Gln Gly Glu Tyr Glu Ser Leu Tyr Thr Ser His Ile
   35 40 45
   Trp Ile Pro Ser Ser Trp Cys Ser Leu Thr Glu Gly Pro Glu Cys Asp
   50 55 60
   Val Thr Asp Asp Ile Thr Ala Thr Val Pro Tyr Asn Leu Arg Val Arg
   65 70 75 80
   Ala Thr Leu Gly Ser Gln Thr Ser Ala Trp Ser Ile Leu Lys His Pro
   85 90 95
   Phe Asn Arg Asn Ser Thr Ile Leu Thr Arg Pro Gly Met Glu Ile Thr
   100 105 110
   Lys Asp Gly Phe His Leu Val Ile Glu Leu Glu Asp Leu Gly Pro Gln
   115 120 125
   Phe Glu Phe Leu Val Ala Tyr Trp Arg Arg Glu Pro Gly Ala Glu Glu
   130 135 140
   His Val Lys Met Val Arg Ser Gly Gly Ile Pro Val His Leu Glu Thr
   145 150 155 160
   Met Glu Pro Gly Ala Ala Tyr Cys Val Lys Ala Gln Thr Phe Val Lys
   165 170 175
   Ala Ile Gly Arg Tyr Ser Ala Phe Ser Gln Thr Glu Cys Val Glu Val
   180 185 190
   Gln Gly Glu Ala
   195
<210> 68
   <211> 203
   <212> PRT
   <213> Homo sapiens
<400> 68
   Asp Glu Val Ala Ile Leu Pro Ala Pro Gln Asn Leu Ser Val Leu Ser
    1               5                  10                  15
   Thr Asn Met Lys His Leu Leu Met Trp Ser Pro Val Ile Ala Pro Gly
               20                  25                  30
   Glu Thr Val Tyr Tyr Ser Val Glu Tyr Gln Gly Glu Tyr Glu Ser Leu
           35                  40                  45
   Tyr Thr Ser His Ile Trp Ile Pro Ser Ser Trp Cys Ser Leu Thr Glu
       50                  55                  60
   Gly Pro Glu Cys Asp Val Thr Asp Asp Ile Thr Ala Thr Val Pro Tyr
   65                  70                  75                  80
   Asn Leu Arg Val Arg Ala Thr Leu Gly Ser Gln Thr Ser Ala Trp Ser
                   85                  90                  95
   Ile Leu Lys His Pro Phe Asn Arg Asn Ser Thr Ile Leu Thr Arg Pro
               100                 105                 110
   Gly Met Glu Ile Pro Lys His Gly Phe His Leu Val Ile Glu Leu Glu
           115                 120                 125
   Asp Leu Gly Pro Gln Phe Glu Phe Leu Val Ala Tyr Trp Thr Arg Glu
       130                 135                 140
   Pro Gly Ala Glu Glu His Val Lys Met Val Arg Ser Gly Gly Ile Pro
   145                 150                 155                 160
   Val His Leu Glu Thr Met Glu Pro Gly Ala Ala Tyr Cys Val Lys Ala
                   165                 170                 175
   Gln Thr Phe Val Lys Ala Ile Gly Arg Tyr Ser Ala Phe Ser Gln Thr
               180                 185                 190
   Glu Cys Val Glu Val Gln Gly Glu Ala Ile Pro
           195                 200
<210> 69
   <211> 196
   <212> PRT
   <213> Homo sapiens
<400> 69
   Leu Pro Ala Pro Gln Asn Leu Ser Val Leu Ser Thr Asn Met Lys His
   1               5                   10                  15
   Leu Leu Met Trp Ser Pro Val Ile Ala Pro Gly Glu Thr Val Tyr Tyr
               20                  25                  30
   Ser Val Glu Tyr Gln Gly Glu Tyr Glu Ser Leu Tyr Thr Ser His Ile
           35                  40                  45
   Trp Ile Pro Ser Ser Trp Cys Ser Leu Thr Glu Gly Pro Glu Cys Asp
       50                  55                  60
   Val Thr Asp Asp Ile Thr Ala Thr Val Pro Tyr Asn Leu Arg Val Arg
   65                  70                  75                  80
   Ala Thr Leu Gly Ser Gln Thr Ser Ala Trp Ser Ile Leu Lys His Pro
                   85                  90                  95
   Phe Asn Arg Asn Ser Thr Ile Leu Thr Arg Pro Gly Met Glu Ile Pro
               100                 105                 110
   Lys His Gly Phe His Leu Val Ile Glu Leu Glu Asp Leu Gly Pro Gln
           115                 120                 125
   Phe Glu Phe Leu Val Ala Tyr Trp Thr Arg Glu Pro Gly Ala Glu Glu
       130                 135                 140
   His Val Lys Met Val Arg Ser Gly Gly Ile Pro Val His Leu Glu Thr
   145                 150                 155                 160
   Met Glu Pro Gly Ala Ala Tyr Cys Val Lys Ala Gln Thr Phe Val Lys
                   165                 170                 175
   Ala Ile Gly Arg Tyr Ser Ala Phe Ser Gln Thr Glu Cys Val Glu Val
               180                 185                 190
   Gln Gly Glu Ala
           195
<210> 70
   <211> 135
   <212> PRT
   <213> Homo sapiens
<400> 70
   Cys Ser Leu Thr Glu Gly Pro Glu Cys Asp Val Thr Asp Asp Ile Thr
   1               5                   10                  15
   Ala Thr Val Pro Tyr Asn Leu Arg Val Arg Ala Thr Leu Gly Ser Gln
               20                  25                  30
   Thr Ser Ala Trp Ser Ile Leu Lys His Pro Phe Asn Arg Asn Ser Thr
           35                  40                  45
   Ile Leu Thr Arg Pro Gly Met Glu Ile Thr Lys Asp Gly Phe His Leu
       50                  55                  60
   Val Ile Glu Leu Glu Asp Leu Gly Pro Gln Phe Glu Phe Leu Val Ala
   65                  70                  75                  80
   Tyr Trp Arg Arg Glu Pro Gly Ala Glu Glu His Val Lys Met Val Arg
                   85                  90                  95
   Ser Gly Gly Ile Pro Val His Leu Glu Thr Met Glu Pro Gly Ala Ala
               100                 105                 110
   Tyr Cys Val Lys Ala Gln Thr Phe Val Lys Ala Ile Gly Arg Tyr Ser
           115                 120                 125
   Ala Phe Ser Gln Thr Glu Cys
       130                 135
<210> 71
   <211> 135
   <212> PRT
   <213> Homo sapiens
<400> 71
   Cys Ser Leu Thr Glu Gly Pro Glu Cys Asp Val Thr Asp Asp Ile Thr
   1               5                   10                  15
   Ala Thr Val Pro Tyr Asn Leu Arg Val Arg Ala Thr Leu Gly Ser Gln
               20                  25                  30
   Thr Ser Ala Trp Ser Ile Leu Lys His Pro Phe Asn Arg Asn Ser Thr
           35                  40                  45
   Ile Leu Thr Arg Pro Gly Met Glu Ile Pro Lys His Gly Phe His Leu
   50 55 60
   Val Ile Glu Leu Glu Asp Leu Gly Pro Gln Phe Glu Phe Leu Val Ala
   65 70 75 80
   Tyr Trp Thr Arg Glu Pro Gly Ala Glu Glu His Val Lys Met Val Arg
   85 90 95
   Ser Gly Gly Ile Pro Val His Leu Glu Thr Met Glu Pro Gly Ala Ala
   100 105 110
   Tyr Cys Val Lys Ala Gln Thr Phe Val Lys Ala Ile Gly Arg Tyr Ser
   115 120 125
   Ala Phe Ser Gln Thr Glu Cys
   130 135
<210> 72
   <211> 15
   <212> PRT
   <213> Homo sapiens
<400> 72
   Gly Gly Gly Gly Ser Gly Gly Gly Gly Ser Gly Gly Gly Gly Ser
   1 5 10 15